# EUROPEAN PATENT APPLICATION

(11) **EP 4 800 105 A1**
(43) Date of publication of application: **02.09.2026**
(21) Application number: 24881776.9
(22) Date of filing: 25.10.2024
(51) Int. Cl.: C12N 9/22, C07K 14/195, C07K 14/315, C12N 15/11, A61K 38/00

(54) **EFFICIENT, LOW OFF-TARGET GENE EDITING TOOL**

(30) Priority: 25.10.2023 CN 202311388744
(71) Applicant: CSPC Zhongqi Pharmaceutical Technology (Shijiazhuang) Co., Ltd., Shijiazhuang, Hebei 050035 (CN); Shanghai JMT-Bio Technology Co., Ltd., Shanghai 200032 (CN)
(72) Inventor: QI, Sixian, Shijiazhuang, Hebei 050035 (CN); ZHONG, Feiya, Shijiazhuang, Hebei 050035 (CN); WEI, Lifan, Shijiazhuang, Hebei 050035 (CN); SU, Xiaoye, Shijiazhuang, Hebei 050035 (CN); WANG, Yun, Shijiazhuang, Hebei 050035 (CN); WU, Leibin, Shijiazhuang, Hebei 050035 (CN); DING, Zhan, Shijiazhuang, Hebei 050035 (CN); YANG, Xuan, Shijiazhuang, Hebei 050035 (CN); ZHONG, Qiang, Shijiazhuang, Hebei 050035 (CN); SHAO, Yu, Shijiazhuang, Hebei 050035 (CN); YANG, Sicong, Shijiazhuang, Hebei 050035 (CN); WANG, Haoyan, Shijiazhuang, Hebei 050035 (CN)
(74) Representative: Lavoix
(86) International application number: PCT/CN2024/127525
(87) International publication number: WO 2025/087417

(57) **Abstract**

The invention relates to a gene editing tool having a reduced off-target rate, wherein a nuclease having significantly reduced off-target effects can be obtained by modifying a Cas9 nuclease. Said modification is applicable to all known gene editing systems that are based on DNA targeting functions of Cas9, greatly improving the specificity and safety of gene editing systems.

## Description

### TECHNICAL FIELD

The present application belongs to the field of biomedicine technology, and specifically relates to a gene editing tool with high efficiency and low off-target effects.

### BACKGROUND

CRISPR-Cas9 is the third generation of gene editing technology following the development of ZFN, TALENs and other gene editing technologies. Since Zhang Feng first reported the high editing efficiency of Cas9 in mammalian cells in 2013, CRISPR-Cas9 technology has developed rapidly and has become one of the most efficient, straightforward, cost-effective and most operable technologies for gene editing and gene modification available today, and is currently the most widely adopted gene editing system.

Clustered Regularly Interspersed Short Palindromic Repeats (CRISPR) is a natural immune system of prokaryotes, present in 40% of sequenced bacteria and 90% of sequenced archaea. Following viral invasion, certain bacteria can "store" a small fragment of the viral gene into their own DNA. Upon subsequent viral infection, the bacteria can recognize the virus based on the "stored" fragment and cleave the viral DNA to render it ineffective.

Currently, the classic CRISPR-Cas9 system consists of two parts, sgRNA and Cas9 protein. SgRNA (single guide RNA) consists of crRNA and tracrRNA. Currently, the 3' end of crRNA and the 5' end of tracrRNA are connected by linker to merge into one sgRNA. Cas9 protein binds to sgRNA to form an RNA-protein complex (RNP), which locates and recognizes PAM and the target sequence, and the DNA double helix will be unwound to form an R-Loop. The sgRNA hybridizes with the complementary chain, while the other chain remains free. Subsequently, the Cas9 protein cleaves precisely at a site located 3 nucleotides upstream of the PAM, generating blunt-end products. The HNH domain of the Cas9 protein is responsible for cleaving the DNA chain complementary to the crRNA, while the RuvC domain cleaves the other non-complementary DNA chain. Finally, Cas9 induces a DNA double stranded break (DSB), and gene knockout is achieved through frameshift mutations during genomic repair.

While the CRISPR-Cas9 system can efficiently edit target genes, the wild-type Cas9 protein exhibits a relatively high incidence of off-target editing, with an average of about 100 off-target sites. A high off-target frequency poses risks of severe genomic mutations and chromosomal translocations, hindering the development of gene editing.

Studies have shown that the specificity of the CRISPR/Cas9 system primarily depends on the recognition sequence of the sgRNA. Since the designed sgRNA may mismatch with non-target DNA sequences, unwanted genetic mutations can occur, which is referred to as off-target effects. Off-target mutations may cause genomic instability and impair the function of other normal genes. Therefore, researchers are committed to studying the factors affecting off-target effects in the CRISPR/Cas9 system. Specifically, the factors affecting off-target include: (1) the influence of sgRNA and DNA pairing sequences on off-target effects: base mismatches may occur between the sgRNA sequence and the DNA binding region; (2) the influence of PAM on off-target: usually, different PAM sequences result in varying off-target effects; generally, the more nucleotides required, the lower the off-target effect; (3) the influence of Cas9 and other factors: modifying wild-type Cas9 to enhance the specificity of the CRISPR/Cas9 system, and using mutant nucleases can induce efficient gene editing in human cells and greatly reduce off-target mutation levels.

Regarding the issue of off-target effects, the current CRISPR/Cas9 gene editing technology presents the following challenges: 1) the Cas9 protein exhibits a relatively high off-target rate, resulting in an increased risk of genomic damage and chromosome aberrations; 2) the predominant delivery vector for mainstream gene editing is AVV, and AVV-mediated delivery results in prolonged expression of the editing machinery, thereby increasing the risk of off-target events; 3) viral vector delivery possesses immunogenicity, rendering it unsuitable for repeated administration. Through engineering, the Cas9 nuclease can function as a precise DNA binding protein guided by gRNA, directing other functional proteins fused to it to the target site for nucleotide modification operations, thus forming a series of gene editing tools, including but not limited to base editors, epigenetic editors, etc. However, within these systems, off-target issues persist for analogous reasons.

### SUMMARY

The present application obtains a nuclease with significantly reduced off-target effects by modifying the Cas9 nuclease. The modification is applicable to all known gene editing systems based on the DNA targeting function of Cas9, including but not limited to Cas9 nucleic acid double stranded break nucleases, Cas9 nickase (nCas9), or Catalytically dead Cas9 (dCas9), or fusion proteins thereof, such as base editing systems such as ABE8e, epigenetic editors such as CRISPRoff-EE, etc.

Specifically, the present application involves the following technical solutions:
1. An isolated modified Cas9 nuclease or a DNA binding fragment thereof, comprising a mutation at one or more amino acid residue positions selected from the group consisting of: K526, N692, Q695, H698, N497, Y450, Q926, K377, E387, D397, R400, D406, A421, L423, R424, Q426, Y430, K442, P449, V452, A456, R457, W464, M465, K468, E470, T474, P475, W476, F478, K484, S487, A488, T496, F498, L502, N504, K506, P509, F518, N522, E523, L540, S541, I548, D550, F553, V561, K562, E573, A589, L598, D605, L607, N609, N612, E617, D618, D628, R629, R635, K637, L651, K652, R654, T657, G658, L666, K673, S675, I679, L680, L683, N690, R691, F693, S701, F704, Q712, G715, Q716, H723, I724, L727, I733, L738, Q739, N803, Q805, Q807, K810, Y812, D829, N831, R832, S834, D835, Q844, S845, K848, R859, K862, R864, K866, K890, T893, Q894, D898, N899, K902, K913, K918, Q920, T924, R925, T928, K929, H930, S960, K961, S964, K968, R976, H982, H983, Y1013, K1031, T1033, SI106, K1107, S1109, Y1237, Y1242, K1244 and K1246, wherein the amino acid residue positions correspond to or are defined with reference to the amino acid numbering in the amino acid sequence of Streptococcus pyogenes Cas9 (SpCas9) protein (SEQ ID NO.68).
2. The modified Cas9 nuclease or DNA binding fragment thereof according to item 1, comprising one or more mutations selected from the group consisting of: K526X, Q695X, H698X and R691X, wherein X is glycine (G), alanine (A), valine (V), isoleucine (I), leucine (L), aspartic acid (D), glutamic acid (E), asparagine (N), glutamine (Q), serine (S), threonine (T), lysine (K), arginine (R), phenylalanine (F) or tyrosine (Y).
3. The modified Cas9 nuclease or DNA binding fragment thereof according to item 1 or 2, wherein each X is independently any one selected from the group consisting of: glycine (G), alanine (A), aspartic acid (D) and glutamic acid (E).
4. The modified Cas9 nuclease or DNA binding fragment thereof according to item 3, comprising any mutation combination selected from the group consisting of K526A+R691A+Q695A+H698A, K526A+R691A+N692A+Q695A+H698A, K526G+R691G+Q695G+H698G, K526D+R691A+Q695A+H698A, K526A+R691D+Q695A+H698A, K526A+R691A+Q695A+H698D, K526E+R691A+ Q695A+H698A, K526A+R691E+Q695A+H698A, and K526A+R691A+Q695A+H698E.
5. The modified Cas9 nuclease or DNA binding fragment thereof according to any one of items 1 to 4, which is a Cas9 double stranded nucleic acid cleavage enzyme, nCas9 (Cas9 nickase), or dCas9 (catalytically dead Cas9).
6. The modified Cas9 nuclease or DNA binding fragment thereof according to any one of items 1 to 5, further comprising one or more mutations in the RuvC domain and/or the HNH domain, optionally comprising a mutation of aspartic acid to alanine (D10A) at position 10 in the RuvC domain and/or a mutation of histidine to alanine (H840A) at position 840 in the HNH domain.
7. The modified Cas9 nuclease or DNA binding fragment thereof according to any one of items 1 to 6, wherein the amino acid sequence of the Cas9 nuclease is shown in SEQ ID NO.69, SEQ ID NO.78 or SEQ ID NO.81, or comprises an amino acid sequence having at least 80%, 85%, 90%, 95%, 96%, 97%, 98%, 99% or 100% sequence identity to the amino acid sequence shown in SEQ ID NO.69, SEQ ID NO.78 or SEQ ID NO.81.
8. The Cas9 nuclease or DNA binding fragment thereof according to any one of items 1 to 7, wherein the DNA binding fragment does not comprise one or more amino acid segments selected from the group consisting of:
   amino acid segments at positions 494-501, 179-296, 503-708, 792-897 and 1010-1081, wherein the amino acid positions correspond to the amino acid numbering in the SpCas9 protein amino acid sequence of SEQ ID NO. 68,
   optionally, the DNA binding fragment is a DNA binding fragment comprised in SEQ ID NO.69, SEQ ID NO.78 or SEQ ID NO.81, wherein the DNA binding fragment does not comprise one or more amino acid segments selected from the following group in these sequences:
      amino acid segments at positions 494-501, 179-296, 503-708, 792-897 and 1010-1081.
9. A fusion protein comprising the Cas9 nuclease or DNA binding fragment thereof according to any one of items 1 to 8.
10. The fusion protein according to item 9, further comprising a nuclear localization signal peptide on the N-terminus and/or the C-terminus of the Cas9 nuclease or DNA binding fragment thereof.
11. The fusion protein according to item 9 or 10, further comprising a cytidine deaminase, an adenine deaminase, an oxidase, a glycosylase, an alkyltransferase, a DNA synthase, an RNA synthase, a uracil glycosylase inhibitor (UGI), a transcription activator, a transcription repressor, a methylase, a demethylase, a Gam protein from bacteriophage Mu, and/or a fluorescent protein, fused to the Cas9 nuclease or DNA binding fragment thereof; optionally, the transcription activator comprises VP64, and optionally, the transcription repressor comprises a KRAB protein.
12. The fusion protein according to item 11, wherein the fusion protein comprises or consists of, from the N-terminus to the C-terminus:
   1) a nuclear localization signal peptide-Cas9 double stranded nucleic acid cleavage enzyme, nCas9 or dCas9 or a DNA binding fragment thereof-a nuclear localization signal peptide;
   2) a nuclear localization signal peptide-TadA* enzyme-nCas9-a nuclear localization signal peptide;
   3) TadA* enzyme-nCas9;
   4) Dnmt3A-Dnmt3L-dCas9-KRAB; or
   5) Dnmt3A-Dnmt3L-dCas9-a nuclear localization signal peptide-KRAB;
   wherein "-" indicates a linkage via a peptide bond or a linker.
13. The fusion protein according to item 12, wherein:
   each linker independently comprises or is any one or more amino acid sequences selected from the group consisting of: SEQ ID NO.77, SEQ ID NO.85, SEQ ID NO.88, SEQ ID NO.89, SEQ ID NO.90 and SEQ ID NO.91, or an amino acid sequence having at least 80%, 85%, 90%, 95%, 96%, 97%, 98%, or 99% sequence identity thereto;
   the amino acid sequence of each nuclear localization signal peptide independently comprises or is any one selected from the group consisting of: SEQ ID NO.70, SEQ ID NO.71, SEQ ID NO.74, and SEQ ID NO.75, optionally, the amino acid sequence shown in SEQ ID NO.71 or 74 is located on the N-terminal side, and/or the amino acid sequence shown in SEQ ID NO.70, 75 or 85 is located on the C-terminal side;
   the amino acid sequence of the TadA*enzyme comprises or is the amino acid sequence shown in SEQ ID NO.76;
   the amino acid sequence of KRAB comprises or is the amino acid sequence shown in SEQ ID NO.87;
   the amino acid sequence of DNMT3A comprises or is the amino acid sequence shown in SEQ ID NO.87; and/or
   the amino acid sequence of DNMT3L comprises or is the amino acid sequence shown in SEQ ID NO.87.
14. The fusion protein according to any one of items 11 to 13, wherein the amino acid sequence of the fusion protein is shown in SEQ ID NO.66, SEQ ID NO.73 or SEQ ID NO.80, or comprises an amino acid sequence having at least 80%, 85%, 90%, 95%, 96%, 97%, 98%, 99% or 100% sequence identity to the amino acid sequence shown in SEQ ID NO.66, SEQ ID NO.73 or SEQ ID NO.80.
15. An engineered nucleic acid molecule, comprising a nucleotide sequence encoding the modified Cas9 nuclease or DNA binding fragment thereof according to any one of items 1 to 8, or the fusion protein according to any one of items 9 to 14.
16. The nucleic acid molecule according to item 15, comprising a 5' untranslated region sequence (5'UTR) and a 3' untranslated region (3'UTR) sequence; wherein the 5'UTR comprises or is a 5'UTR of tobacco etch virus gene, and/or the 3'UTR comprises or is a 3'UTR of human hemoglobin alpha 1 (hHBA1) gene.
17. The nucleic acid molecule according to item 15 or 16, wherein the nucleotide sequence of the 5'UTR is shown in SEQ ID NO.10 or 11, or comprises a nucleotide sequence having at least 80%, 85%, 90%, 95%, 96%, 97%, 98%, 99% or 100% nucleotide sequence identity to the nucleotide sequence shown in SEQ ID NO.10 or 11, and/or the nucleotide sequence of the 3'UTR is shown in SEQ ID NO.13, or comprises a nucleotide sequence having at least 80%, 85%, 90%, 95%, 96%, 97%, 98%, 99% or 100% sequence identity to the nucleotide sequence shown in SEQ ID NO.13.
18. The nucleic acid molecule according to any one of items 15 to 17, further comprising a poly (A) tail sequence or a polyadenylation signal sequence, preferably the poly (A) tail sequence comprises a nucleotide sequence shown in SEQ ID NO. 12 or comprises a nucleotide sequence having at least 80%, 85%, 90%, 95%, 96%, 97%, 98%, 99% or 100% sequence identity to SEQ ID NO.12.
19. The nucleic acid molecule according to any one of items 15 to 18, which is an mRNA and comprises a 5' cap structure, optionally, the 5' cap structure is m7G(5')ppp(5')(2'OMeA)pG.
20. The nucleic acid molecule according to any one of items 15 to 19, wherein the nucleotide sequence of the nucleic acid molecule is shown in SEQ ID NO.2, SEQ ID NO.6 or SEQ ID NO.8, or comprises a nucleotide sequence having at least 80%, 85%, 90%, 95%, 96%, 97%, 98%, 99% or 100% sequence identity to the nucleotide sequence shown in SEQ ID NO.2, SEQ ID NO.6 or SEQ ID NO.8.
21. The nucleic acid molecule according to any one of items 15 to 20, which is an mRNA molecule comprising one or more uridine (U) residues with modified bases, optionally, the U residues with modified bases are 1-methylpseudouridines; optionally, each U in the nucleic acid molecule encoding the Cas9 nuclease or an enzymatically active fragment thereof is 1-methylpseudouridine.
22. A composition, a ribonucleoprotein complex or a protein-lipid complex comprising the modified Cas9 nuclease or DNA binding fragment thereof according to any one of items 1 to 8, or the fusion protein according to any one of items 9 to 14, or the nucleic acid molecule according to any one of items 15 to 21.
23. The composition, ribonucleoprotein complex or protein-lipid complex according to item 22, further comprises a gRNA targeting a gene of interest, a nucleic acid molecule encoding the gRNA, or a construct comprising the gRNA.
24. The composition, ribonucleoprotein complex or protein-lipid complex according to item 23, wherein the target gene is any one or more selected from the group consisting of: hepatitis B virus (HBV) gene, PCSK9, EMX1 and VEGFA3.
25. A gene editing method, comprising introducing into a host cell the modified Cas9 nuclease or DNA binding fragment thereof according to any one of items 1 to 8, or the fusion protein according to any one of items 9 to 14, or the nucleic acid molecule according to any one of items 15 to 21, or the composition, ribonucleoprotein complex or protein-lipid complex according to any one of items 22 to 24.
26. Use of the modified Cas9 nuclease or DNA binding fragment thereof according to any one of items 1 to 8, or the fusion protein according to any one of items 9 to 14, or the nucleic acid molecule according to any one of items 15 to 21, or the composition, ribonucleoprotein complex or protein-lipid complex according to any one of items 22 to 24 in the preparation of a medicament for treating a disease or disorder in a subject in need thereof.

### BRIEF DESCRIPTION OF THE DRAWINGS

FIG. 1: Bioanalyzer analysis of the integrity of mRNA encoding Cas9 protein.
FIG. 2: Experimental results showing that Cas9 mRNA is normally translated in HEK293T cells.
FIG. 3: Results of the purity analysis of sgTTR-0 by HPLC-RP.
FIG. 4: The editing efficiency on the TTR gene and the knockdown effect on TTR protein of different gRNAs targeting TTR were detected by T7E1 enzymatic cleavage assay (A) and ELISA (B) respectively. Wherein panel A shows the cleavage results detected by T7E1 enzymatic cleavage assay; panel B shows the TTR protein content in the supernatant of HepG2 cells measured by ELISA.
FIG. 5: GUIDE-seq analysis of the off-target rates of sgTTR-0 and sgTTR-1. Wherein panel A shows the number of off-target sites detected for sgTTR-0 and sgTTR-1, respectively, when combined with SpCas9 mRNA. Panel B shows the distribution of cleavage sites on chromosomes for the combination of sgTTR-0 and Cas9 mRNA. Panel C shows the distribution of the cleavage sites on chromosomes for the combination of sgTTR-1 and Cas9 mRNA.
FIG. 6: The cleavage efficiency of various modified and mutant gRNAs on the TTR gene detected by T7E1 enzymatic cleavage assay.
FIG. 7: Off-target rate (safety) assay of Cas9-mut5 using GUIDE-seq. Wherein panel A shows the specific number of off-target sites detected by GUIDE-seq for different Cas9 mutants in combination with sgTTR-0; panel B shows the statistical analysis of off-target frequency (off-target reads/on-target reads) for different Cas9 mutants combined with sgTTR-0; panel C shows the potential off-target sites across the whole genome for Cas9-mut5 and Cas9-WT.
FIG. 8: Gene editing effects of different SpCas9 mutants in combination with sgTTR-0. Wherein panel A shows the reduction in serum TTR protein levels in mice one week after administration (1 mg/kg), as measured by ELISA; panel B shows the editing efficiency of TTR gene in mouse liver one month after administration (1 mg/kg), as measured by amplicon sequencing; panel C shows the knockdown effect of sgTTR-0+SpCas9-mut5 on serum TTR protein in humanized mice at different dosages.
FIG. 9: Gene editing effects of sgTTR-0 and SpCas9 mRNA delivered via different LNP formulations. Wherein panel A shows the changes in serum TTR protein levels in mice detected by ELISA one week after administration (0.3 mg/kg); panel B shows the editing efficiency results of TTR gene in liver measured one month after administration (0.3 mg/kg); panel C shows the changes in serum TTR protein content in mice detected by ELISA one week after administration (1 mg/kg) under different delivery formulations.
FIG. 10: Gene editing effects of different mass ratios of sgTTR-0 combined with SpCas9 mRNA. Wherein panel A shows the serum TTR protein content in mice one week after administration as detected by ELISA; panel B shows the editing efficiency of TTR gene in mouse liver one month after administration measured by amplicon sequencing.
FIG. 11: GUIDE-seq analysis for safety assessment under different gRNA to mRNA mass ratios. Wherein panel A shows the specific number of off-target sites measured by GUIDE-seq for different gRNA to mRNA mass ratios; panel B shows the statistically obtained off-target frequency (number of off-target reads/number of on-target reads) for different gRNA to mRNA mass ratios.
FIG. 12: Editing efficiency of different Cas9 mutants at TTR locus in HEK293T cells and HepG2 cells. Panel A shows the editing efficiency of different Cas9 mutants at TTR locus in HEK293T cells; panel B shows the editing efficiency of different Cas9 mutants at TTR locus in HepG2 cells; panel C shows the knockdown efficiency of TTR protein by different Cas9 mutants in HepG2 cells.
FIG. 13 shows the editing efficiency of SpCas9-WT and its mutants guided by sgTTR-0 at four different off-target sites (off-target site 1/2/3/4, shown in A to C, respectively).
FIG. 14 shows the editing efficiency of SpCas9-WT and its mutants at four target sites in the TRAC gene (guided by sgRNA-1/2/3/4, respectively, shown in A to D).
FIG. 15: editing efficiencies of SpCas9-WT and its mutants at two target sites (A: EMX1; B: VEGFA3), and off-target rates at corresponding high-frequency off-target sites.
FIG. 16: Schematic diagrams of the structures of ABE8e and ABE8e-Mut5. Panel A shows ABE8e reported in the literature (i.e., ABE8e-WT in the examples of this application), and panel B shows ABE8e-Mut5.
FIG. 17: The on-target editing efficiencies of ABE8e-mut5 and ABE8e-WT together with 7 gRNAs (A: EMX1; B: VEGFA3; C: TTR-ABE-1; D: HEK site1; E: HEK site2; F: HEK site3; G: HEK site4) and the editing efficiencies at their corresponding high-frequency off-target sites identified by next-generation sequencing.
FIG. 18: Editing efficiencies of ABE8e-mut5 and ABE8e-WT within and outside the editing windows at six genomic loci (A: EMX1; B: VEGFA3; C: HEK293-2; D: PCSK9; E: F-site2; F: ATGg2) identified by next-generation sequencing.
FIG. 19: Schematic diagram of the structures of EE-WT (A: CRISPRoff-EE-WT) and EE-mut5 (B: CRISPRoff-EE-Mut5).
FIG. 20: Inhibitory efficiency of EE-mut5 and EE-WT on PCSK9 protein in different hepatoma cell liness (A: HepG2, B: Huh7).
FIG. 21: Inhibitory effects of EE-mut5 and EE-WT on HBV DNA (A) and related proteins (B: HBsAg, C: HBeAg) in HepG2.2.15 cells, as well as their effects on cell viability (D).
FIG. 22: The number of differentially methylated sites detected in the genome of HepG2.2.15 cells treated with EE-mut5 and EE-WT.

### DETAIL DESCRIPTION

### Definition:

Unless otherwise defined, all technical and scientific terms used herein have the same meanings as understood by one of ordinary skill in the art. The abbreviations for amino acid residues are the standard 3-letter and/or 1-letter codes commonly used in the art to refer to one of the 20 common L-amino acids.

As used herein, the term "Cas9 nuclease", also known as CRISPR associated protein 9, is understood by those skilled in the art that, guided by a gRNA, binds to and cleaves double stranded DNA target sites. The Cas9 nucleases can be modified, such as through amino acid mutations, deletions or additions, to achieve higher editing efficiency, higher editing specificity, lower off-target rates, or loss of cleavage activity on one or both DNA strands. In some embodiments of the present application, "modified Cas9 nuclease" is a double stranded cleavage enzyme with higher editing specificity or lower off-target efficiency. In some embodiments, "modified Cas9 nuclease" is a nickase (Cas9 nickase or nCas9), which cleaves one strand of DNA. In some embodiments, "modified Cas9 nuclease" loses its DNA cleavage activity while retaining the ability to target and bind DNA under the guidance of gRNA, in this case, it is referred to as catalytically dead Cas9, dead Cas9 or dCas9.

As used herein, the term "coding sequence" may refer to a ribonucleotide sequence in a mature mRNA that can be translated into a protein, or it may refer to a complementary sequence of a deoxyribonucleotide (DNA) sequence that serves as a template for transcribing the ribonucleotide (RNA) sequence. In addition, the "coding sequence" of the present application can further include polynucleotide sequences encoding functional nucleic acids, such as miRNA, shRNA, dsRNA, etc.

In the present application, "N-terminal side" is used to describe the relative positional relationship between two sequence segments, between one amino acid and one sequence segment, or between two amino acids in the same amino acid sequence. Among them, "N-terminal" refers to the end of the amino acid sequence comprising a free amino group. For example, "the N-terminal side of the Cas9 nuclease or a DNA binding fragment thereof further comprises a nuclear localization signal peptide" means that the "nuclear localization signal peptide" is located closer to the N-terminus of the shared amino acid sequence than the "Cas9 nuclease or a DNA binding fragment thereof". Similarly, "C-terminal side" is also used to describe the relative positional relationship between two sequence segments, between an amino acid and a sequence segment, or between two amino acids within the same amino acid sequence. Among them, "C-terminal" refers to the end of the amino acid sequence that comprises a free carboxyl group. For example, "the C-terminal side of the Cas9 nuclease or a DNA binding fragment thereof further comprises a nuclear localization signal peptide" means that the "nuclear localization signal peptide" is positioned closer to the C-terminal of the shared amino acid sequence relative to the "Cas9 nuclease or a DNA binding fragment thereof". The sequence or amino acid located at the N-terminal side or C-terminal side of a given sequence or an amino acid can be directly linked to the sequence or the amino acid, or separated by one or more intervening amino acid residues.

Although the numerical ranges and approximate parameter values set forth in the broad scope of this application are approximations, the numerical values shown in the specific examples are recorded as accurately as possible. However, any numerical value inherently comprises certain errors resulting from the standard deviations present in their respective measurements. In addition, all ranges disclosed herein should be understood to cover any and all sub-ranges comprised therein. For example, the range of "1 to 10" recorded should be considered to include any and all sub-ranges between the minimum value 1 and the maximum value 10 (including the endpoints); that is, all sub-ranges starting with a minimum value of 1 or greater, such as 1 to 6.1, and sub-ranges ending with a maximum value of 10 or less, such as 5.5 to 10. In addition, any reference referred to as "incorporated herein" should be understood to be incorporated in its entirety.

It will be understood by those skilled in the art that, due to the degeneracy of the genetic code, many different polynucleotides can encode the same polypeptide. It should also be understood that a skilled artisan can use conventional techniques to introduce nucleotide substitutions that do not alter the polypeptide sequence encoded by the nucleic acid molecule, in response to the codon usage of any particular host organism in which the polypeptide is to be expressed. Therefore, unless otherwise specified, "polynucleotides encoding a protein or immunogenic fragment of the present application" include all polynucleotide sequences that are degenerate with respect to each other and encode the same amino acid sequence.

The term "ribonucleoprotein" (RNP) or "RNP complex" refers to a guide RNA together with an RNA-guided DNA binding agent, such as a Cas nuclease, e.g., a Cas cleavage enzyme, a Cas nickase, or a dCas DNA-binding agent (e.g., Cas9). In some embodiments, the guide RNA guides an RNA-guided DNA-binding agent such as Cas9 to a target sequence, and the guide RNA hybridizes to the target sequence and the agent binds to the target sequence; in the case where the agent is a cleavage enzyme or a nickase, the binding may be followed by cleavage or nicking.

The term "TTR", or transthyretin, refers to the protein product of the TTR gene, also known as vitamin A binding protein, which is an important component of plasma proteins and is widely distributed in a variety of cells, plasma and tissue fluids. As a carrier protein, TTR is mainly synthesized in the liver and the choroid plexus of the brain, and is secreted into the blood and cerebrospinal fluid, transporting thyroxine and retinol (i.e., vitamin A) to various tissues and cells throughout the body. As a carrier protein, the function of TTR can often be compensated for by thyroxine-binding globulin and albumin in plasma. Under physiological conditions, TTR is a stable protein. Dissociation of TTR into monomers can cause amyloidosis. In recent years, more and more TTR-targeting drugs have been progressively advancing into clinical use for the treatment of neurological diseases, endocrine and metabolic diseases, etc.

The term "nuclear localization signal", "NLS", "nuclear localization signal peptide" or "nuclear localization sequence" refers to an amino acid sequence or peptide segment that directs the transport of molecules comprising or linked to such a sequence into the nucleus of eukaryotic cells. A nuclear localization signal may constitute an integral part of the molecule to be transported. In some embodiments, the NLS may be attached to the molecule by covalent bonds, hydrogen bonds or ionic interactions.

### Nucleic Acid

The term "nucleic acid" or "nucleic acid molecule" are recognized and understood by those of ordinary skill in the art. As used herein, the term "nucleic acid" or "nucleic acid molecule" preferably refers to DNA (molecule) or RNA (molecule). They are preferably used synonymously with the term polynucleotide. Preferably, a nucleic acid or nucleic acid molecule is a polymer comprising or consisting of nucleotide monomers, which are covalently linked to each other via phosphodiester bonds of a sugar/phosphate backbone. The term "nucleic acid molecule" also includes modified nucleic acid molecules, such as base-modified, sugar-modified or backbone-modified DNA or RNA molecules as defined herein.

Unless otherwise specified, the term "nucleotide" in the present application, in addition to referring to naturally occurring ribonucleotides or deoxyribonucleotide monomers, should also be understood herein to refer to related structural variants thereof, including derivatives and analogs, which are functionally equivalent in the specific context of their use, unless the context clearly indicates otherwise. For example, "nucleotide" refers to deoxyribonucleotide or ribonucleotide. A nucleotide may be a standard nucleotide (i.e., adenosine (A), guanosine (G), cytidine (C), thymidine (T) and uridine (U)), a nucleotide isomer or a nucleotide analog, such as U or T used to represent a nucleotide in an mRNA sequence may represent natural uridine as well as pseudouridine, etc., such as 1-methylpseudouridine. Nucleotide analogs refer to nucleotides with modified purine or pyrimidine bases or modified ribose moieties. Nucleotide analogs may be either naturally occurring nucleotides (e.g., inosine, pseudouridine, etc.) or non-naturally occurring nucleotides. Non-limiting examples of modifications on the sugar or base moiety of a nucleotide include the addition (or removal) of acetyl, amino, carboxyl, carboxymethyl, hydroxyl, methyl, phosphoryl and thiol groups, as well as the substitution of carbon and nitrogen atoms in the base with other atoms (e.g., 7-deazapurine). Nucleotide analogs also include dideoxynucleotides, 2'-O-methyl nucleotide, locked nucleic acid (LNA), peptide nucleic acid (PNA) and morpholino oligonucleotides.

In some embodiments, the nucleic acid comprises at least one heterologous untranslated region (UTR). The term "untranslated region" or "UTR" or "UTR element" will be recognized and understood by those of ordinary skill in the art to refer to a portion of a nucleic acid molecule, usually located 5' or 3' to a coding sequence. It is referred to as the 5 'UTR at the 5' end and as the 3 'UTR at the 3' end. In general, UTRs are not translated into proteins; a UTR can be part of a nucleic acid, such as DNA or RNA. UTR can include elements for regulating gene expression, also referred to as regulatory elements. Such regulatory elements can be ribosome binding sites, miRNA binding sites, etc.; RNA (e.g., mRNA) can further comprise a 5'UTR, a 3'UTR, a 3'-poly A and/or a 5' cap analog.

In some embodiments, the 5'UTR is a heterologous UTR, i.e., a UTR found in nature that is associated with a different ORF; in another embodiment, the 5'UTR is a synthetic UTR; the 5'UTR is the region of mRNA located upstream (5') of the start codon (the first codon of the mRNA transcript to be translated by the ribosome). The 5'UTR does not encode a protein. Natural 5'UTR possess features that play a role in translation initiation, such as the Kozak sequence, which has a consensus CCR(A/G)CCAUGG; exemplary 5'UTR also include those from Tobacco etch virus, Xenopus or human α-globin or β-globin, human cytochrome b-245a polypeptide, hydroxysteroid (17b) dehydrogenase, and alpha-1-globin, etc.

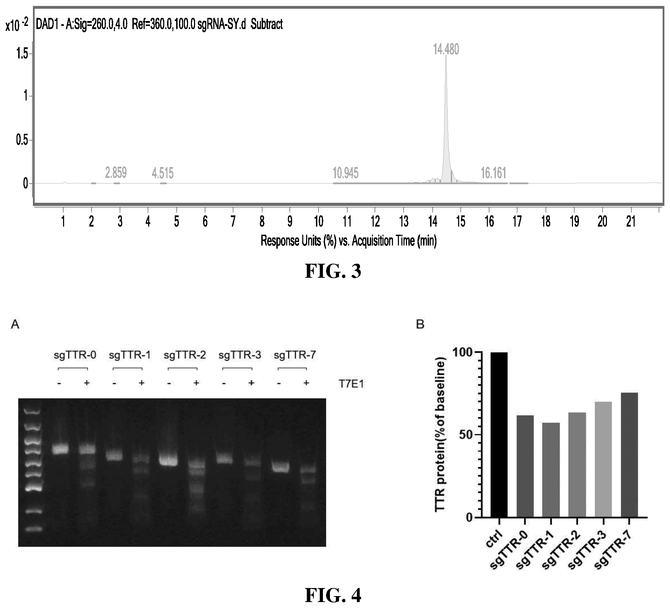

In some embodiments, the 3'UTR can be heterologous or synthetic; for example: the human Hemoglobin Subunit Alpha 1 (HBA1) UTR, globin UTRs including the Xenopus β-globin UTR and the human β-globin UTR; other 3'UTRs can also be 3'UTR sequences from cytochrome b-245alpha chain (CYBA), rabbit β-globin, hepatitis B virus (HBV), the α-globin 3'UTR and the Venezuelan equine encephalitis virus (VEEV) virus. In some embodiments, the 3'UTRs of Ribosomal Protein S9 (rps9), FIG4 Phosphoinositide 5-Phosphatase (FIG4), gp130, DH143 and human albumin human hemoglobin subunit beta (hHBB) can also be used.

In some embodiments, the 3'-polyadenylic acid, also known as the poly(A) tail, is a region of mRNA located downstream of the 3'UTR, for example, directly downstream (i.e., 3'), which comprises multiple consecutive adenosine monophosphates. The poly(A) tail may comprise 10 to 300 adenosine monophosphates, and may comprise 10, 20, 30, 40, 50, 60, 70, 80, 90, 100, 110, 120, 130, 140, 150, 160, 170, 180, 190, 210, 220, 230, 240, 250, 260, 270, 280, 290 or 300 adenosine monophosphates. In some preferred embodiments, the poly (A) tail comprises 50 to 250 adenosine monophosphates, more preferably 50-100 adenosine monophosphates; most preferably 100 adenosine monophosphates; in relevant biological contexts (e.g., in cells, in vivo), the function of the 3'-poly(A) tail is to protect mRNA from enzymatic degradation, such as in the cytoplasm, and to facilitate transcription termination and/or export of the mRNA from the nucleus and translation.

In some embodiments, RNA (e.g., mRNA) further comprises a 5' guanosine cap; the 5' guanosine cap is a hallmark of eukaryotic mRNA transcript, the 5' cap consists of an inverted 7-methylguanosine linked to the rest of the eukaryotic mRNA via a 5'-5' triphosphate bridge, commonly referred to as cap0, which mainly serves as a quality control for correct mRNA processing and helps to stabilize the eukaryotic mRNA; based on cap0, methylation of the 2'-OH group on the first nucleotide yields cap1; in addition to cap 0 and cap 1, further methylation modifications can be applied to the second nucleotide, referred to as cap 2; generally speaking, the synthesis method of the 5'-cap can be: different synthetic routes of 5' capped mRNA based on enzymatic, chemical or chemoenzymatic methods.

In some embodiments, during in vitro transcription, a cap analog is directly added to the in vitro transcription (IVT) system, and the 5' cap analog includes but is not limited to: m⁷Gppp(2'OMeA)pG, m⁷GpppApA, m⁷GpppApC, m⁷GpppApG, m⁷GpppApU, m⁷GpppCpA, m⁷GpppCpC, m⁷GpppCpG, m⁷GpppCpU, m⁷GpppGpA, m⁷GpppGpC, m⁷GpppGpG, m⁷GpppGpU, m⁷GpppUpA, m⁷GpppUpC, m⁷GpppUpG, m⁷GpppUpU, m⁷Gpppm⁶ApG, m⁷G_{3'Ome}pppApA, m⁷G_{3'Ome}pppApC, m⁷G_{3'Ome}pppApU, m⁷G_{3'Ome}pppApG, m⁷G_{3'Ome}pppCpA, m⁷G_{3'Ome}pppCpC, m⁷G_{3'Ome}pppCpG, m⁷G_{3'Ome}pppCpU, M⁷G_{3'Ome}pppUpA, m⁷G_{3'Ome}pppUpC, m⁷G_{3'Ome}pppUpG, m⁷G_{3'Ome}pppUpU, m⁷G_{3'Ome}pppA_{2'Ome}pG, m⁷G_{3'Ome}pppA_{2'Ome}pC, m⁷G_{3'Ome}pppA_{2'Ome}pU, m⁷G_{3'Ome}pppA_{2'Ome}pA, m⁷G_{3'Ome}pppC_{2'Ome}pA, m⁷G_{3'Ome}pppC_{2'Ome}pU, m⁷G_{3'Ome}pppC_{2'Ome}pG, m⁷G_{3'Ome}pppC_{2'Ome}pC, m⁷G_{3'Ome}pppG_{2'Ome}pA, m⁷G_{3'Ome}pppG_{2'Ome}pU, m⁷G_{3'Ome}pppG_{2'Ome}pG, m⁷G_{3'Ome}pppG_{2'Ome}pC, m⁷G_{3'Ome}pppU_{2'Ome}pA, m⁷G_{3'Ome}pppU_{2'Ome}pU, m⁷G_{3'Ome}pppU_{2'Ome}pG, m⁷G_{3'Ome}pppU_{2'Ome}pC, etc.

In some embodiments, the capping analogs may also adopt other structures, such as tetramers, pentamers, hexamers, heptamers, octamers, nonamers or decamers, etc. The specific sequence thereof may be determined based on the template conditions.

As used herein, "mRNA" (messenger RNA) refers to any RNA, whether naturally occurring, non-naturally occurring or modified, that encodes at least one protein, and which can be translated to produce the encoded protein ex vivo, in vivo, in situ or in vitro. It should be known to those skilled in the art that, unless otherwise indicated, the polynucleotide sequences described in this application may use "T" to denote thymine when representing DNA sequences, but when the polynucleotide sequence represents an RNA (e.g., mRNA), "T" will be replaced by "U" (uracil). Therefore, any DNA disclosed and identified by a specific sequence number (SEQ ID NO) herein also discloses the complementary or corresponding RNA (e.g., mRNA) sequence, wherein each "T" in the DNA sequence is replaced by "U".

### Open Reading Frame

An open reading frame (ORF) is a continuous segment of DNA or RNA that begins with a start codon (ATG or AUG, which will be translated into, for example, methionine) and ends with a stop codon (e.g., TAA, TAG, or TGA, or UAA, UAG, or UGA). In general, an ORF usually encodes a protein. It should be understood that the sequences disclosed herein may also include additional elements, such as 5' and 3' UTRs, but unlike ORF, these elements are not necessarily present in the RNA polynucleotides of the present application.

In some embodiments, the composition comprises an RNA (e.g., mRNA) comprising a nucleotide sequence having at least 80%, at least 85%, at least 90%, at least 95%, at least 98%, or 100% identity to SEQ ID NO.2.

In some embodiments, the open reading frame is preferably at least partially codon-optimized. Codon optimization is based on such discovery: translation efficiency can be determined by the varying frequencies of transfer RNA (tRNA) occurring in the cell. Therefore, if there is an increased degree of so-called "rare codons" in the coding region of the nucleic acid defined in this application, the translation efficiency of the corresponding modified nucleic acid sequence is lower than that in the presence of codons encoding relatively "common" tRNAs. Those skilled in the art can perform codon optimization for a sequence to be translated based on the characteristics of their ex vivo expression system.

### Chemically modified or unmodified nucleotides

In some embodiments, the RNA (e.g., mRNA) is not chemically modified, but comprises standard ribonucleotides consisting of adenosine, guanosine, cytidine, and uridine. In some embodiments, the nucleotides and nucleosides disclosed herein comprise standard nucleoside residues, such as those present in transcribed RNA (e.g., A, G, C, or U). In some embodiments, the nucleotides and nucleosides disclosed herein comprise standard deoxyribonucleosides, such as those present in DNA (e.g., dA, dG, dC, or dT).

In some embodiments, the nucleotides and nucleosides of the present application include modified nucleotides or nucleosides. Such modified nucleotides and nucleosides can be naturally occurring modified nucleotides and nucleosides, or non-naturally occurring modified nucleotides and nucleosides. Such modifications can include the sugar of nucleotides and/or nucleosides well known in the art, the modification of the backbone or the nucleobase moiety.

In some embodiments, the modified nucleobases in the nucleic acid (e.g., an RNA nucleic acid, e.g., an mRNA nucleic acid) include 1-methyl-pseudouridine, 1-ethyl-pseudouridine, 5-methoxy-uridine, 5-methyl-cytidine and/or pseudouridine.

### In vitro transcription system (IVT)

In vitro transcription is a process that mimics in vivo transcription, utilizing DNA as a template within a cell-free system containing components such as RNA polymerase and NTPs to generate mRNA. Generally speaking, the capped RNA synthesized in an in vitro transcription reaction can be used for subsequent experiments such as microinjection, in vitro translation, and transfection. A typical in vitro transcription system includes transcription buffer, nucleoside triphosphates (NTPs), RNase inhibitor, and polymerase. NTPs can be synthesized in-house or sourced from commercial suppliers and may be either natural or non-natural. Optional polymerases include, but are not limited to, phage RNA polymerases, such as T7 RNA polymerase, T3 RNA polymerase, SP6 RNA polymerase, and/or polymerase mutants thereof, for example include, but not limited to, polymerases capable of incorporating modified nucleic acids and/or modified nucleotides, including chemically modified nucleic acids and/or nucleotides. The use of DNase is excluded in some embodiments. In some embodiments, the RNA comprises a 5' guanosine cap.

In addition to synthesis via in vitro transcription systems, chemical synthesis methods can also be employed, including solid-phase chemical synthesis and liquid-phase chemical synthesis; with respect to solid-phase chemical synthesis, the nucleic acids disclosed in the present application can be prepared, in whole or in part, using solid-phase technology; solid-phase chemical synthesis of nucleic acids is an automated method in which molecules are immobilized on a solid support and synthesized stepwise in a solution of reactants. Solid-phase synthesis allows for the site-specific introduction of chemical modifications into the nucleic acid sequences; with respect to liquid-phase chemical synthesis, the nucleic acids of the present application can be synthesized in liquid phase through the sequential addition of monomer constructs. In addition, the above-mentioned synthesis methods can also be used in combination, because each of the synthesis methods discussed above each possesses its own advantages and limitations, combining these methods can be attempted to overcome the above-mentioned limitations. Such combinations of methods are within the scope of the present application.

The term "identity" refers to the relationship between the sequences of two or more polypeptides (e.g., antigens) or polynucleotides (nucleic acids) as determined by sequence comparison. Identity also denotes the degree of sequence correlation between or within sequences, as determined by the number of matches between strings of two or more amino acid residues or nucleic acid residues. Identity is measured as the percentage of identical matches between the smaller of two or more sequences, where any gaps in the alignment(if any) are resolved by a specific mathematical model or computer program (e.g., an "algorithm"). The identity of the related antigens or nucleic acids can be easily calculated by known methods. The term " identity percentage (%)" for polypeptide or polynucleotide sequences is defined as the percentage of residues (amino acid residues or nucleic acid residues) in a candidate amino acid or nucleic acid sequence that are identical to the residues in a reference amino acid or nucleic acid sequence, following alignment of the sequences and the introduction of gaps, if necessary, to achieve maximum percentage identity. The methods and computer programs used for alignment are well-known in the art. It is understood that identity depends on the calculation of the percent identity, but its value may vary due to the gaps and penalties introduced during the calculation. Typically, variants of a particular polynucleotide or polypeptide exhibit 40%, 45%, 50%, 55%, 60%, 65%, 70%, 75%, 80%, 85%, 90%, 91%, 92%, 93%, 94%, 95%, 96%, 97%, 98%, 99% or 100% sequence identity to a particular reference polynucleotide or polypeptide, as determined by the sequence alignment programs and parameters described herein and known to those of skilled in the art.

### Lipid Nanoparticles (LNP)

The RNA (e.g., mRNA, gRNA etc.) of the present application can be formulated in lipid nanoparticles (LNPs). Lipid nanoparticles generally comprise ionizable cationic lipids, auxiliary lipids, cholesterol and PEG lipid components, and nucleic acids of interest. The lipid nanoparticles of the present application can be generated using components, compositions and methods generally known in the art.

### Pharmaceutical Formulations

Provided herein are compositions (e.g., pharmaceutical compositions), methods, kits, and reagents for genetic modification or editing in human and other mammalian cells.

The term "pharmaceutical composition" refers to a combination of an active agent with an inert or active carrier, making the composition particularly suitable for in vivo or ex vivo diagnostic or therapeutic use. A "pharmaceutically acceptable carrier" does not cause undesirable physiological effects after administration to a subject. The carrier in a pharmaceutical composition must be "acceptable" in the sense that it is compatible with the active ingredient and capable of stabilizing it. One or more solubilizing agents can be used as pharmaceutical carriers for delivering the active agent. Examples of pharmaceutically acceptable carriers include, but are not limited to, biocompatible carriers, adjuvants, additives and diluents to obtain a composition that can be used as a dosage form. Examples of other carriers include colloidal silicon oxide, magnesium stearate, cellulose and sodium lauryl sulfate. Other suitable pharmaceutical carriers and diluents, as well as pharmaceutical necessities for them, are described in Remington's Pharmaceutical Sciences.

### sgRNA

In each embodiment of the compositions, uses and methods described herein, the guide RNA may comprise a single RNA molecule as a "single-guide RNA" or "sgRNA". The sgRNA may comprise a crRNA or a portion thereof comprising a guide sequence covalently linked to the tracrRNA. In some embodiments, the crRNA and the tracrRNA are covalently linked via a linker. In some embodiments, the sgRNA forms a stem-loop structure via base pairing between portions of the crRNA and the tracrRNA. In some embodiments, the crRNA and the tracrRNA are covalently linked via one or more bonds that are not phosphodiester bonds. In some embodiments, about 20 nucleotides at the 5' end of the sgRNA constitute a sequence complementary to the genomic target (referred to as the spacer), while nucleotides 21-100 constitute a sequence that interacts with Cas9.

### Modified Cas9 nuclease

In one aspect, the present application provides a technical solution for reducing the off-target efficiency of the CRISPR-Cas9 system, including introducing mutations at four sites -526, 691, 695 and 698- into the Cas9 nuclease to generate a modified Cas9 nuclease in which the amino acids at positions 526, 691, 695 and 698 are alanine (A) or its conservatively substituted amino acids, wherein the amino acid positions are numbered with reference to SEQ ID NO.68. It should be understood that some Cas9 nucleases known in the prior art, such as nCas9, dCas9 or Cas9 double stranded cleavage enzymes, may already possess A or its conservatively substituted amino acids at these four sites. Therefore, the solution of the present application should include scenarios where only 1, 2, or 3 amino acid mutations are introduced into the unmodified Cas9 nuclease to form the modified Cas9 nuclease. It should be understood that the conservative substitutions for amino acids A include G (glycine), D (aspartic acid), and E (glutamic acid). This application, through extensive examples, demonstrates that the four aforementioned mutations can reduce the off-target efficiency of Cas9 nuclease or other gene editing systems based on Cas9 nuclease (such as nCas9, dCas9, base editing systems, epigenetic editing systems) without compromising gene editing efficiency. This enables those skilled in the art to reasonably anticipate that introducing the above four mutations into known functional Cas9 nucleases, modified Cas9 nucleases or their fusion proteins will retain their functionality while simultaneously reducing off-target efficiency. Based on this, the present application provides at least a modified Cas9 nuclease or a DNA binding fragment thereof, and a fusion protein with DNA modification function, or at least capable of specifically recognizing a DNA target site under the guidance of a gRNA, comprising the modified Cas9 nuclease or DNA binding fragment thereof. In the modified Cas9 nuclease or DNA binding fragment thereof and the fusion protein, the amino acids at positions 526, 691, 695 and 698 relative to the reference sequence SEQ ID NO.68 are A or its conservatively substituted amino acids, while all other positions are identical to the amino acid sequence of the Cas9 nuclease (including dCas9) or a DNA binding fragment thereof known in the prior art before modification by the four mutations. In some embodiments, the modified Cas9 nuclease or DNA binding fragment thereof and the fusion protein are all A at positions 526, 691, 695 and 698 relative to the reference sequence SEQ ID NO. 68. In some embodiments, the modified Cas9 nuclease is SpCas9.

Examples of Cas9 nucleases before modification by the aforementioned four mutations include, for example, dCas9 and nCas9 disclosed in WO2019126709A1. Examples of DNA binding fragments of Cas9 nucleases before modification by the aforementioned four mutations include, for example, those disclosed in CN110241098A and WO2020005980A1. Examples of fusion proteins comprising Cas9 prior to modification by the aforementioned four mutations include base editors, such as those disclosed in WO2018165629A1, WO2018213708A1, WO2018213726A1, WO2020102659A1, WO2020181195A1, WO2020181193A1, WO2020181180A1, WO2020181178A1, WO2021030666A1, WO2017070632A3, WO2018027078A8 and WO2020181202A1; and epigenetic editors, such as those disclosed in articles with PMID: 34942274, 36864020, 35234927, 38418872, 38760566, 37116617 or 34580310, and WO2020014261A1; as well as other Cas9 nucleases in other developed gene editing systems, for example, those disclosed in WO2021183783A1, WO2020210751A1, WO2020191248A1, WO2014150624A1, WO2020041751A1, WO2021025750A1, WO2020182941A1, WO2020176389A1, WO2014093661A2, WO2016094874A1, WO2016094867A1, AU2015101792A4, WO2016205613A1, WO2016205759A1, WO2018035387A1, WO2018209320A8, US11155803B2, WO2021158921A, WO2015089427A1 and WO2021183807A1, and WO2018039438A1.

### Examples

The embodiments of the present application are described in detail below in conjunction with the examples, but it will be appreciated by those skilled in the art that the following examples are provided only to illustrate the present application and should not be construed as limiting the scope of the present application. Unless otherwise specified, all operations were performed under conventional conditions or according to the manufacturer's recommendations. Reagents or instruments for which the manufacturer is not indicated are conventional products commercially available.

The amino acid mutation sites of the SpCas9 mutants (except the nuclear localization signal and signal peptide portions) used in Examples 1-7 below, and the nCas9 and dCas9 mutants in Example 8, relative to the wild-type SpCas9 protein (SEQ ID NO.68), as well as the mRNA element structures encoding the mutants, are shown in Table 1 below. All U (uridine) in the mRNAs used in the examples of the present application are 1-methyl-pseudouridine. The mRNA sequences of SpCas9-WT (SEQ ID NO.2), SpCas9-Mut5 (SEQ ID NO.2), SpCas9-HF1 (SEQ ID NO.3) and HiFi-Cas9 (SEQ ID NO.4) used in the following examples are shown in the sequence listing. For the mRNA sequences of other SpCas9 nuclease variants (SpCas9-Mut1 to Mut7, and SpCas9-HF4), except for the corresponding codon changes at the amino acid mutation sites, the remaining sequence is identical to that of the SpCas9-HF1 mRNA sequence (SEQ ID NO.3).

**Table 1: mRNA structure of the Cas9 nuclease mutants**

| mRNA | mutation sites (relative to the reference sequence SEQ ID NO.68) | 5'UTR | 3'UTR | polyA sequence |
|---|---|---|---|---|
| SpCas9-WT | N/A | pVAX1, TEV 5'UTR (SEQ ID NO.10) | hHBA1 3'UTR (SEQ ID NO.13) | 30+70A( SEQ ID NO.12) |
| SpCas9-Mut1 | K526A-N692A-Q695A-H698A | | | |
| SpCas9-Mut2 | R895A-R919A | | | |
| SpCas9-Mut3 | R895A-R919A-R691A | | | |
| SpCas9-Mut4 | R895A-R919A-Q926A | | | |
| SpCas9-Mut5 | K526A-R691A-Q695A-H698A | | | |
| SpCas9-Mut6 | K526A-R691A-H698A | | | |
| SpCas9-Mut7 | K526A-R691A-N692A-Q695A-H6 98A | | | |
| SpCas9-HF1 | N497A-R661A-Q695A-Q926A | | | |
| SpCas9-HF4 | Y450A-N497A-R661A-Q695A-Q9 26A | | | |
| HiFiCas9 | R691A | | | |
| nCas9-WT | D10A | | | |
| nCas9-Mut5 | D10A-K526A-R691A-Q695A-H69 8A | | | |
| dCas9-WT | D10A-H840A | | | |
| dCas9-Mut5 | D10A-K526A-R691A-Q695A-H69 8A-H840A | | | |

It should be understood that the protein and nucleic acid sequences used in the following examples are shown in the sequence listing, or can be obtained by introducing the mutations described in Table 1 into the sequences shown in the Sequence Listing.

### Example 1: Quality Control of Cas9 mRNA

### 1.1 In vitro transcription (IVT) of Cas9 mRNA

1. According to the instructions of the IVT kit (E131, Novoprotein), the IVT reaction system was prepared by mixing 10 Transcription Buffer, ATP, GTP, CTP, 1-N-Me-Pseudo UTP (Cat. No.: WA0992, Hongene Biotech), 5' cap analog m7G(5')ppp(5')(2'OMeA)pG (Cat. No.: GAGNH23C2L1B, Hongene Biotech), water for injection, a linearized plasmid template containing the T7 promoter and the DNA sequence encoding Cas9 mRNA (GenScript Biotech Co., Ltd.), and the Enzyme Mix;
2. The reaction mixture was incubated at 37°C for 40 minutes;
3. DNase I was added at the corresponding ratio to terminate the reaction.

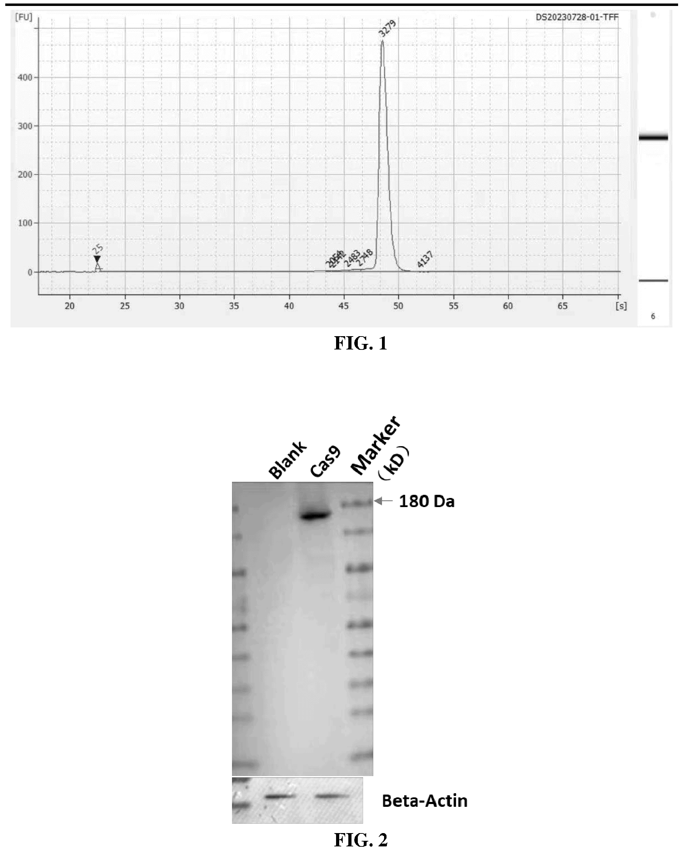

Following *in vitro* synthesis, the mRNA encoding the Cas9 protein was isolated and purified by hydrophobic chromatography and ultrafiltration concentration, and the purity of the Cas9-encoding mRNA was analyzed using a Bioanalyzer, confirming the acquisition of high-purity mRNA (>95%). Among them, the purity results of spCas9-WT mRNA are shown in FIG. 1.

### 1.2 Western blot analysis of Cas9 protein expression

HEK293T and HepG2 cells were transfected with mRNA encoding Cas9 as follows:
(1) Seeding: HEK293T or HepG2 cells were trypsinized, resuspended, counted, and seeded into 24-well plates at a density of 1.5 x 10⁵ cells per well.
(2) Transfection: 24 hours after seeding, two 1.5 ml EP tubes were prepared, and 250 µl Opti-MEM was added to each tube. Subsequently, 500 ng of Cas9 mRNA was added to EP tube No. "1" and mixed well, while 2.5 µl of Lipo2000 was added to EP tube No. "2" and mixed well. Then the liquid in EP tube No. "2" was transferred to EP tube No. "1", mixed well, centrifuged and incubated for 15 minutes before adding to the cell culture wells.
(3) Medium change: after 6 hours, the cell supernatant was removed and replaced with complete culture medium.
(4) Cell harvesting: 24 hours post-transfection of HEK29T cells with the mRNA, the cells were harvested, and the expression of Cas9 protein was analyzed by western blot.

The results, shown in FIG. 2, demonstrate that the Cas9 protein mRNA synthesized via the IVT procedure described in Example 1 was effectively expressed in HEK29T cells. It should be noted that all mRNA used in the subsequent examples underwent the procedures outlined in sections 1.1 and 1.2 prior to further testing.

### 1.3 Chemical Synthesis and Purity Analysis of gRNA

Step 1: solid-phase synthesis: the gRNA was synthesized using an automated synthesizer. Synthesis proceeded from the 3' end to the 5' end via sequential coupling of phosphoramidite nucleoside monomers on a UnyLinker support (Biocomma, 1000A CPG). Following the coupling of the last monomer, the 5' end dimethoxytrityl (DMT) protecting group was removed, and the phosphate backbone protecting groups (cyanoethyl) were deprotected to yield the solid-phase support intermediate.

Step 2: cleavage/deprotection via ammonolysis: the solid-phase support intermediate was cleaved from the solid-phase support and deprotected via ammonolysis, and the crude product was obtained after concentration and desilylation reaction.

Step 3: chromatographic purification and ultrafiltration desalting: the crude product was purified by hydrophobic chromatography, acid hydrolysis, and reverse phase chromatography to remove short-chain sequences and other impurities. The collected qualified fractions were subjected to ultrafiltration for desalting and pyrogen removal, yielding an aqueous gRNA solution without subsequent lyophilization.

Step 4: aseptic filling: the gRNA solution was sterile-filtered twice and aseptically filled in an isolator to obtain the final gRNA stock solution product.

The gRNA related sequence structures involved in this application are as follows:

**Table 2: gRNA used in the examples of this application**

| gRNA name | Spacer sequence | Origin | Complete nucleotide sequence | Chemical modification |
|---|---|---|---|---|
| sgTTR-0 | SEQ ID NO.24 | Human | SEQ ID NO.14 | The first to third nucleotides from the 5 'end and the last one to three nucleotides of each of the full nucleotide sequence are modified with 2'- O-methylation, and the linkages between the first to third nucleotides from the 5' end and between the last one to three nucleotides are modified with thiophosphate bond |
| sgTTR0-crRNA | | | SEQ ID NO.22 | |
| sgTTR0-tracerRNA | | | SEQ ID NO.23 | |
| sgNA-TTR-0-del (82 nt) | | | SEQ ID NO.21 | |
| sgTTR-0-v02 | | | SEQ ID NO.19 | |
| sgTTR-0-v04 | | | SEQ ID NO.20 | |
| sgTTR-1 | SEQ ID NO.25 | | SEQ ID NO.15 | |
| sgTTR-2 | SEQ ID NO.26 | | SEQ ID NO.16 | |
| sgTTR-3 | SEQ ID NO.27 | | SEQ ID NO.17 | |
| sgTTR-7 | SEQ ID NO.28 | | SEQ ID NO.18 | |
| sgRNA-1 | SEQ ID NO.29 | | Except for the Spacer sequence, other parts are the same as sgTTR-0 | |
| sgRNA-2 | SEQ ID NO.30 | | | |
| sgRNA-3 | SEQ ID NO.13 | | | |
| sgRNA-4 | SEQ ID NO.32 | | | |
| EMX1 | SEQ ID NO.33 | | | |
| VEGFA3 | SEQ ID NO.34 | | | |
| HEK293_2 | SEQ ID NO.35 | | | |
| ATGg2 | SEQ ID NO.36 | | | |
| PCSK9 | SEQ ID NO.37 | | | |
| F-site2 | SEQ ID NO.38 | | | |
| TTR-ABE-1 | SEQ ID NO.39 | | | |
| HEK site1 | SEQ ID NO.40 | | | |
| HEK site2 | SEQ ID NO.41 | | | |
| HEK site3 | SEQ ID NO.42 | | | |
| HEK site4 | SEQ ID NO.43 | | | |

The purity of the relevant gRNAs met the requirements for subsequent experiments. An exemplary result is shown in FIG. 3, which presents the purity analysis result of sgTTR-0.

### Example 2. Optimization of gRNA

### 2.1 Assessment of Editing Efficiency on the TTR gene and Protein by Different gRNAs

Different gRNAs (sgTTR-0, sgTTR-1, sgTTR-2, sgTTR-3, and sgTTR-7) were co-transfected with SpCas9-WT mRNA into HEK293T cells (the mass ratio of gRNA to mRNA was 1:1). After 24 hours, the cells were lysed, and a DNA fragment of about 1 kb flanking the respective target sites was amplified by PCR. The targeted cleavage efficiency was then assessed using the T7 Endonuclease I (T7E1) assay (the kit used was GeneArt^{™} Genomic Cleavage Detection Kit, A24372, ThermoFisher).

The specific steps for the T7E1 assay to determine targeted cleavage efficiency were as follows:
(1) the edited cells were lysed, and a DNA fragment of about 1000 bp upstream and downstream of the editing site (i.e., using an upstream primer designed about 1000 bp upstream of the editing site, and a downstream primer designed about 1000 bp downstream of the editing site) was amplified by PCR;
(2) the PCR products were purified by column and the concentrations were measured by Nanodrop;
(3) Using 100 ng of the purified DNA product, and enzymatic digestion reaction mixture was prepared according to the manufacturer's instructions. After annealing, 1 µl of T7E1 enzyme was added to react at 37°C for 1 h;
(4) the digestion products were analyzed by agarose gel electrophoresis to assess the editing efficiency.

Furthermore, different gRNAs were separately combined with Cas9 mRNA and transfected into HepG2 cells (the mass ratio of gRNA to mRNA was 1:1). After 72 hours, the cell culture supernatant was collected and the content of TTR protein in the cell supernatant was detected by ELISA. The ELISA detection steps were as follows:
(1) preparation of samples and standard solution: the samples to be tested (cell culture supernatant or animal serum) were diluted appropriately, and a series of standard solutions with gradient concentrations were prepared simultaneously according to the manufacturer's instructions.
(2) sample addition: the samples were added to a 96-well ELISA microplate at 50 µL/well, the plate was sealed with sealing film, and incubated at room temperature for 1 h.
(3) addition of detection antibody: after washing the plate three times with PBST, a biotinylated detection antibody was added at 50 µl/well, the plate was sealed with sealing film, and incubated at room temperature for 1 h.
(4) addition of secondary antibody: after washing the plate three times with PBST, the secondary antibody was added at 50 µl/well, the plate was sealed with sealing film, and incubated at room temperature for 30 min.
(5) color development: after washing the plate three times with PBST, TMB color development solution (50 µl/well) was added, and color development was performed at room temperature in the dark for 10-15 min.
(6) reaction termination: 50 µl of stop solution was added to each well to stop color development.
(7) plate reading: the OD values were measured at 450 nm using a microplate reader.

The results of the above experiment (FIG. 4) indicated that sgTTR-0, sgTTR-1, sgTTR-2, sgTTR-3, and sgTTR-7 all exhibited certain levels of cleavage activity, among which sgTTR-0 and sgTTR-1 demonstrated superior efficacy. Subsequent experiments were therefore focused on these two sgTTR candidates.

### 2.2 Dection of off-target rate of sgTTR-0 and sgTTR-1 by GUIDE-seq analysis

In the examples of the present application, the GUIDE-seq method was employed for off-target rate detection, as described in, for example, Tsai, Zheng et al. 2015, Malinin, Lee et al. 2021.

GUIDE-seq is a method for detecting off-target effects in gene editing. Its fundamental principle involves using a short double stranded oligodeoxynucleotide (dsODN) tag to label CRISPR-Cas9-induced double strand breaks (DSBs) (i.e., following DSB genereation by the Cas9 enzyme of the CRISPR system, there is a probability that the dsODN tag will be integrated into the genome during genome repair process). Subsequently, the genomic regions containing the integrated dsODN tags are amplified via PCR using primers designed upstream and downstream of the DSB sites. The amplified products are then used to construct a library for high-throughput sequencing. Finally, bioinformatics analysis is performed to identify the locations and frequencies of off-target mutations.

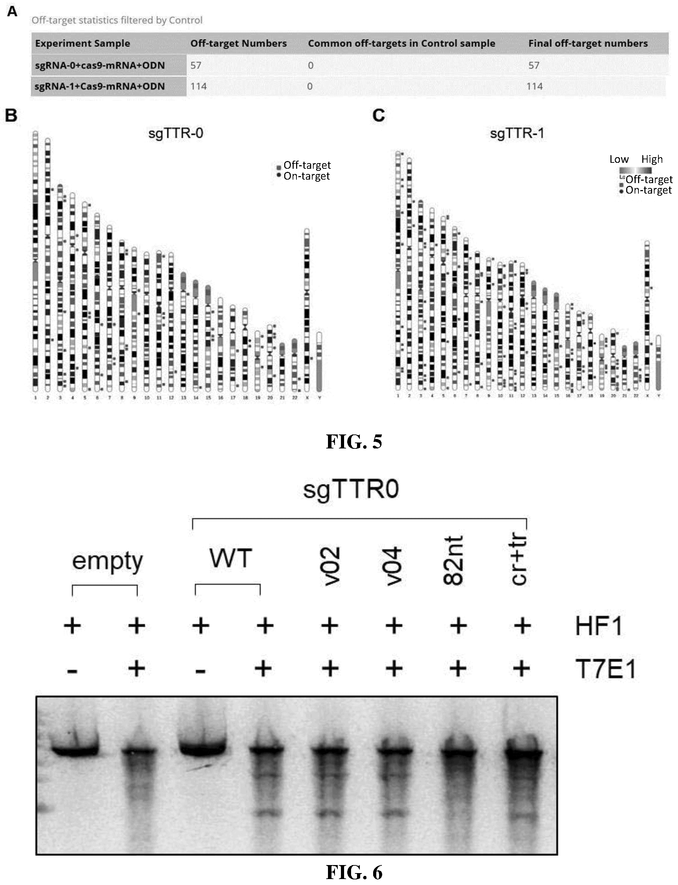

The procedures for off-target rate detection were as follows:
(1) Cell transfection. HepG2 cells were co-transfected via electroporation with gRNA (sg-TTR-0 and sgTTR-1), wild-type SpCas9 mRNA, and a double stranded oligodeoxynucleotide (dsODN) tag.
(2) Genomic DNA extraction: genomic DNA was extracted from the cells 72 hours after transfection.
(3) Detection of ODN integration efficiency: double stranded DNA cleavage efficiency was assessed using T7E1 enzyme digestion, while dsODN tag insertion efficiency was evaluated via Ndel digestion, and subsequent analysis was performed only when the combined Ndel/T7E1 integration efficiency reached 30%.
(4) An appropriate amount of qualified genomic DNA was used to construct sequencing libraries, which were then sequenced on the MGI-2000 system;
(5) the results of the bioinformatic analysis are presented in FIG. 5, where:
   Panel A shows the number of off-target sites detected for sgTTR-0 and sgTTR-1, respectively, when combined with SpCas9 mRNA;
   Panel B shows the distribution of cleavage sites on chromosomes induced by the combination of sgTTR-0 and Cas9 mRNA;
   Panel C shows the distribution of cleavage sites on chromosomes induced by the combination of sgTTR-1 and Cas9 mRNA.

It can be seen that different gRNAs exhibit distinct off-target efficiencies, with sgTTR-0 demonstrating a significantly lower off-target probability compared with sgTTR-1.

### 2.3 Further optimization exploration of sgTTR-0

### 2.3.1 Detection of TTR target protein by western blot

HEK293T cells were co-transfected with different sgTTR-0 variants (sgTTR-0-v02, sgTTR-0-v04, TTR-0-del (82nt); and cr+tr, a combination of the crRNA and tracrRNA components of sgTTR-0) and Cas9-HF1 mRNA. The cells were lysed after 24 hours, and a DNA fragment of about 1 kb surrounding the sgTTR-0 targeting site was amplified, and the cleavage efficiency was assessed using T7E1 enzyme digestion.

Among them, FIG. 6 shows the results of T7E1 enzyme digestion assay, indicating that both sgTTR-0-v02 and sgTTR-0-v04, as well as the separation of the sgRNA into crRNA and tracrRNA, maintained editing efficacy against the TTR gene, while truncation of the sgRNA to 82nt resulted in loss of editing activity against the TTR gene.

### Example 3 Screening of Cas9 mutants with low off-target rates

### 3.1 Assessment of off-target rate (safety) of wild-type Cas9 (SpCas9-WT) and mutants thereof via GUIDE-seq analysis

After co-transfection of HepG2 cells with mRNA encoding SpCas9-WT or its mutants (SpCas9-WT, SpCas9-HF1, SpCas9-Mut1, SpCas9-Mut2, SpCas9-Mut3, SpCas9-Mut4, SpCas9-Mut5, SpCas9-Mut6) and sgTTR-0, off-target efficiency was assessed. The specific steps were as follows:
(1) cell transfection. HepG2 cells were co-transfected with sg-TTR-0, wild-type or mutant SpCas9 mRNA, and a double stranded oligodeoxynucleotides (dsODN) tag via electroporation.
(2) genomic DNA extraction: 72 hours after transfection, genomic DNA was extracted.
(3) ODN integration rate detection: the efficiency of DNA double stranded cleavage was assessed using the T7E1 enzyme digestion. The insertion efficiency of the dsODN tag was evaluated via Ndel restriction enzyme digestion, and subsequent analysis was performed only when the Ndel/T7E1 integration efficiency reached 30%.
(4) appropriate genomic DNA of qualified quality was used to construct sequencing libraries, which were then sequenced using the MGI-2000 system;
(5) bioinformatics analysis.

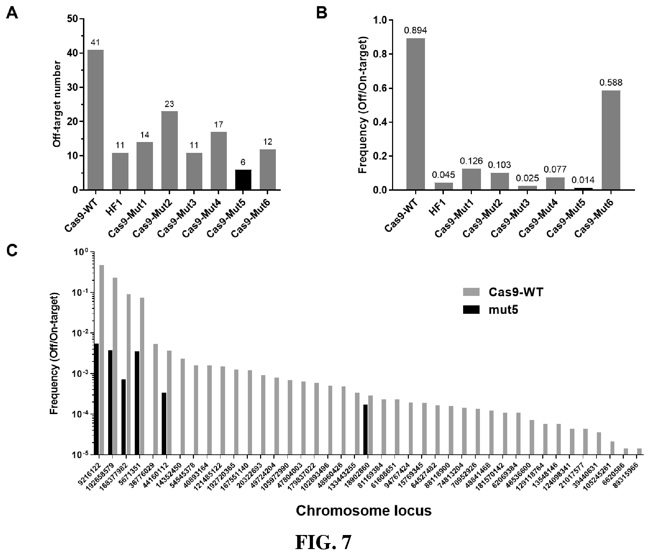

The analysis results are shown in FIG. 7, where:
FIG. 7: panel "A" shows the numbers of specific off-target sites detected by GUIDE-seq for different Cas9 mutants paired with sgTTR-0. The wild-type Cas9/sgTTR-0 combination yielded 41 off-target sites, the previously reported low off-target mutant Cas9-HF1/sgTTR-0 combination yielded 11 off-target sites, while Cas9-mut5/sgTTR-0 combination reduced the number of off-target sites to 6; panel "B" shows the off-target frequency (number of off-target reads/number of on-target reads) for different Cas9 mutants paired with sgTTR-0. The results from both panel A and panel B of FIG. 7 demonstrate that the safety profile of Cas9-mut5 is not only significantly superior to that of wild-type Cas9, but also outperforms the reported low off-target mutant and other mutants (such as Cas9-Mut1, Cas9-Mut2, Cas9-Mut3, Cas9-Mut4, and Cas9-Mut6).

Panel C of FIG. 7 shows the potential off-target sites of Cas9-mut5 and Cas9-WT across the whole genome.

### 3.2 Assessment of gene editing efficiency for different SpCas9 mutants paired with sgTTR-0

To determine whether the editing efficiency of the aforementioned mutants with reduced off-target efficiency (e.g., SpCas9-Mut5) was affected by the mutations, their gene editing efficiency at the TTR target site was evaluated.

The mice used for this assessment were a humanized TTR gene mouse model (Strain ID: T055186), purchased from GemPharmatech Co., Ltd. (Jiangsu, China). This model was generated using gene editing technology to replace the mouse TTR gene coding region and regulatory sequences with the corresponding human TTR gene fragment, which is often used in research on transthyretin amyloidosis and for drug screening.

The specific test steps were as follows:
(1) Preparation of test drugs. sgTTR-0 was encapsulated in LNP with mRNA of SpCas9-WT and various mutants (SpCas9-WT, SpCas9-HF1, SpCas9-HF4, SpCas9-Mut1, SpCas9-Mut2, Cas9-Mut3, Cas9-Mut4, SpCas9-Mut5, SpCas9-Mut6) at a mass ratio of 1:1. The encapsulation method is described in Example 4.
(2) Grouping of experimental animals. TTR-humanized mice aged 6-8 weeks, regardless of gender, underwent submandibular blood collection one week prior to dosing. Serum TTR protein expression levels were measured using an ELISA kit (ab231920, Abcam). Based on the baseline TTR expression levels measured before treatment, the mice were divided into groups of 4-6 animals each, ensuring that the overall baseline TTR expression levels were comparable across all groups.
(3) Drug administration. A single dose was administered via tail vein injection. The day of administration was designated as day 0. The administered doses were 1 mpk (1 mg/kg), 0.3 mg/kg, or 3 mg/kg, where "mg" refers to the total mass of the nucleic acid in the therapeutic agent.
(4) Periodic blood collection. Blood was collected from the submandibular area of mice on days 4, 7, 14, 21, and 28 after administration to obtain serum. Serum levels of TTR protein were measured using an ELISA kit (ab231920, Abcam) to determine the knockdown level of TTR protein.
(5) Tissue harvesting from mice. All mice were euthanized 28 days after administration, and the livers were harvested, quickly frozen in liquid nitrogen and stored at -80°C.
(6) Determination of liver editing efficiency. Genomic DNA was extracted from the liver tissues. Primers were designed flanking the target gene region for PCR amplification. The PCR products were subjected to high-throughput sequencing to obtain mutation frequency information for the target region, i.e., the editing efficiency was determined via amplicon sequencing.

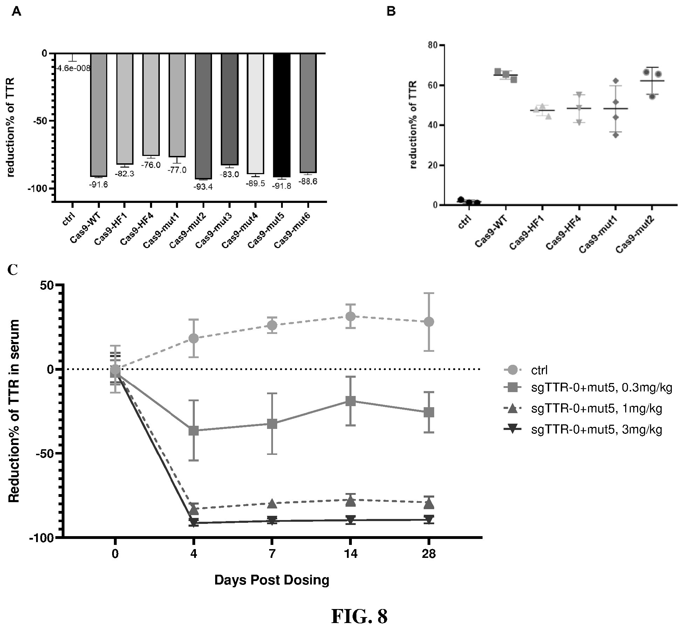

The experimental results are shown in FIG. 8. It can be seen that wild-type SpCas9 and various SpCas9 mutants, when combined with sgTTR-0, all exhibited a certain degree of editing efficacy on the TTR gene in humanized TTR mice. Using the reduction level of TTR protein in the serum of humanized TTR mice as an indicator, the previously reported low off-target mutant HF1 demonstrated improved safety at the cost of partially reduced efficacy, while Cas9-mut5 not only achieved further enhanced safety but also maintained editing efficacy comparable to that of wild-type Cas9.

### Example 4 Optimization of LNP delivery formulations

### 4.1 Gene editing experiments of sgTTR-0 and SpCas9 mRNA with different LNP delivery formulations

To obtain an optimal LNP delivery formulation for in vivo genome editing, various LNP formulations, as shown in Table 3, were tested in this example.

The main steps were as follows:
(1) Preparation of test drugs. sgTTR-0 and SpCas9 mRNA (SpCas9-HF1 mRNA or SpCas9-Mut5 mRNA) were encapsulated in LNPs formulated with different compositions.
(2) Grouping of experimental animals. One week prior to administration, serum TTR protein expression levels were measured using an ELISA kit (ab231920, Abcam). Based on the baseline TTR expression levels, 6-8-week-old TTR-humanized mice (both genders) were allocated into groups of 4-6 animals each, ensuring that the overall baseline TTR expression was comparable across all groups.

**Table 3**

| Mol% | LNP-01 | LNP-02 | LNP-03 | LNP-04 | LNP-05 | LNP-06 |
|---|---|---|---|---|---|---|
| SM102 | 50 | 0 | 25 | 25 | | |
| D-Lin-MC3-DMA | 0 | 50 | 25 | 25 | | |
| LP01 | | | | | 50 | |
| Lipid10 | | | | | | 50 |
| DSPC | 10 | 10 | 10 | 0 | 9 | 10 |
| DOPE | 0 | 0 | 0 | 10 | | |
| cholesterol | 38.5 | 38.5 | 38.5 | 38.5 | 38 | 37.5 |
| PEG2000-DMG | 1.5 | 1.5 | 1.5 | 1.5 | 3 | 2.5 |
| N/P | 6 | 6 | 6 | 6 | 6 | 6 |

(3) Administration. The drug was administered via tail vein injection at a dose of 1 mpk or 0.3 mpk, and the day of administration was recorded as day 0.
(4) One week after administration, serum TTR protein levels were measured by ELISA to determine the extent of TTR protein knockdown.
(5) Tissue Harvesting. 28 days after administration, mice were euthanized and their liver tissues were harvested, immediately snap-frozen in liquid nitrogen, and stored at -80°C.
(6) Determination of liver editing efficiency. Genomic DNA was extracted from the liver samples, the target gene region was amplified by PCR, and the PCR products were subjected to high-throughput sequencing to determine the editing efficiency.

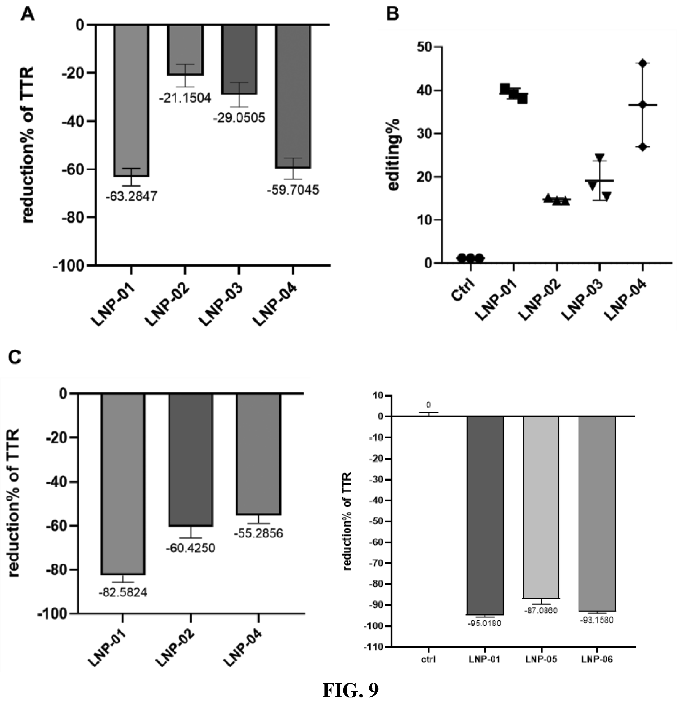

The results show (see FIG. 9) that LNP-01 is a more suitable LNP formulation for delivery, and the combination of its encapsulated gRNA and Cas9 mRNA (whether SpCas9-HF1 in panels A-C of FIG. 9 or SpCas9-MUT5 in panel D of FIG. 9) exhibited the best gene editing efficiency.

Therefore, the LNP formulation used in all subsequent examples of the present application was LNP-01. Therefore, taking LNP-01 as an example, the packaging method for LNP in the examples of the present application was as follows:
(1) Appropriate amounts of SM-102, DSPC, cholesterol, and DMG-PEG2000 lipid, with a molar mass ratio of 50%, 10%, 38.5%, and 1.5%, respectively, were precisely weighed and dissolved in a suitable volume of anhydrous ethanol to prepare a lipid working solution (final lipid working concentration: 20 mg/mL) for subsequent use.
(2) The following buffer solutions were prepared separately: a citric acid buffer solution (10 mM, pH 4.0) comprising 130 mM sodium chloride, a Tris-NaOAc buffer solution (20 mM, 10.7 mM, pH 7.5), and a Tris-NaOAc buffer solution (20 mM, 10.7 mM, pH 7.5) compriseing 60% sucrose.
(3) An appropriate amount of mRNA stock solution was diluted with the previously prepared sodium chloride-citrate buffer solution to adjust the final concentration of the mRNA working solution to 0.18 mg/mL.
(4) Using a microfluidic instrument and its compatible chip, the lipid working solution and the mRNA working solution were mixed at a volume ratio of 1:3 to prepare the mRNA-loaded LNP solution.
(5) The prepared LNP solution was diluted by adding 9-fold volume of Tris-NaOAc buffer solution, followed by concentration and purification via TFF to remove the ethanol from the system.
(6) The mRNA content in the LNP solution was determined using ultraviolet spectrometry. An appropriate amount of Tris-NaOAc buffer (20 mM, 10.7 mM, pH 7.5) compriseing 60% sucrose was added to adjust the final mRNA concentration in the finished LNP solution to 100 µg / mL, while the sucrose content in the external aqueous phase system was adjusted to 8.7%.

The experimental results indicated that the parameters of the LNP, such as encapsulation efficiency, particle size and other parameters met the requirements of subsequent experiments and could be used for lipid formulation screening.

### 4.2 Detection of gene editing efficiency with different mass ratios of sgTTR-0 to SpCas9 mRNA

To further optimize the editing efficiency of the gene editing system in this application, the ratio of gRNA and Cas9 mRNA in LNP liposomes was optimized based on the editing efficiency. The specific experimental steps were as follows:
(1) Preparation of test drugs. sgTTR-0 and SpCas9-HF1 mRNA were encapsulated into LNP at different mass ratios of 1:1, 1:4, 1:8, 1:16, and 1:32. The encapsulation method is shown in Section 4.1.
(2) Grouping of experimental animals. TTR-humanized mice aged 6-8 weeks, regardless of gender, were used. One week before administration, serum TTR protein expression levels were measured using an ELISA kit (ab231920, Abcam). The mice were then grouped according to the pre-dose baseline TTR expression levels, with 4-6 mice in each group, ensuring comparable initial TTR expression levels across all groups.
(3) Administration. Administration was performed via tail vein injection at a dose of 1 mpk, and the day of administration was designated as day 0.
(4) Blood was collected periodically, and serum TTR protein expression levels were measured to determine the extent of TTR protein knockdown.
(5) Mouse tissue harvesting. 28 days after administration, the mice were euthanized and their livers were harvested, quickly frozen in liquid nitrogen and stored at -80°C.
(6) Determination of liver editing efficiency. Genomic DNA was extracted from the liver tissues. The target gene region was amplified by PCR, and the PCR products were subjected to high-throughput sequencing to determine the editing efficiency.

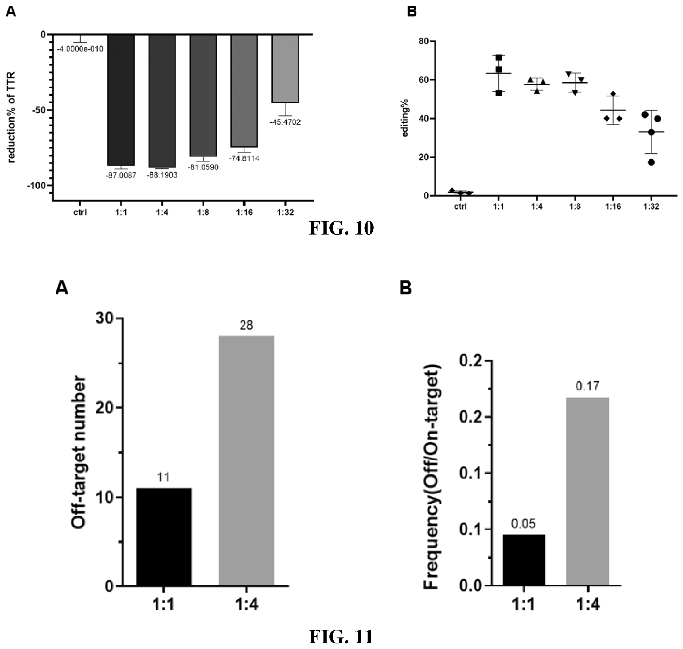

The results are shown in FIG. 10. It can be seen that all combinations of sgTTR-0 and SpCas9 mRNA at different mass ratios exhibited a certain degree of editing effect on the TTR gene. Among them, the editing effect was best when the mass ratio of gRNA to SpCas9 mRNA was 1:1 and 1:4. The editing efficiency began to decrease to some extent when the ratio reached 1:8, and the TTR editing efficiency was essentially reduced by half at a mass ratio of 1:32.

### 4.3 Off-target rate detection for combinations of sgTTR-0 and SpCas9 mRNA at different mass ratios

To further optimize the editing efficiency of the gene editing system in this application, the ratio of gRNA to Cas9 mRNA in the LNP liposomes was optimized based on the off-target efficiency. The specific experimental steps were as follows:
(1) Cell transfection. HepG2 cells were co-transfected via electroporation with sgTTR-0, SpCas9-WT mRNA and a double stranded oligodeoxynucleotides (dsODN) tag. The total transfection amount was maintained at 1µg, with HepG2 cells being transfected using different mass ratios of sgTTR-0 to SpCas9 mRNA.
(2) Genomic DNA extraction: genomic DNA was extracted from the cells 72 hours after transfection.
(3) Detection of ODN integration efficiency: double stranded DNA cleavage efficiency was assessed using T7E1 enzyme digestion, while dsODN tag insertion efficiency was evaluated via Ndel digestion, and subsequent analysis was performed only when the combined Ndel/T7E1 integration efficiency reached 30%.
(4) An appropriate amount of qualified genomic DNA was used to construct sequencing libraries, which were then sequenced on the MGI-2000 system;
(5) Bioinformatics analysis.

The results are shown in FIG. 11, indicating that the safety is better when the mass ratio of gRNA to mRNA is 1:1.

Using the transthyretin (TTR) gene as the target in Examples 1-4, a set of CRISPR-Cas9 nuclease system suitable for TTR gene editing with lower off-target efficiency was obtained by optimizing the gRNA, the Cas9 and LNP delivery systems, as well as the ratio of gRNA to Cas9 mRNA. Notable, through extensive screening in the aforementioned examples, the low off-target Cas9 enzyme mutant SpCas9-Mut5 was identified. This mutant achieved efficient TTR gene knockout effect while maximizing the reduction of off-target effects, thereby minimizing the side effects associated with the application of this system and yielding unexpected technical benefits.

In subsequent examples, the gene editing efficiency and low off-target rate of SpCas9-Mut5 will be validated across multiple cell lines and additional target genes. Concurrently, it was discovered in follow-up examples that after engineering SpCas9-Mut5 into dead Cas9 (dCas9) and Cas9 nickase (Cas9 nickase), it retained the characteristics of high editing efficiency and low off-target activity. Furthermore, these advantageous features can be incorporated into various engineered editing systems, such as Cas9nikase-based base editing systems and dCas9-based epigenetic editing systems.

### Example 5: validation of editing efficiency of SpCas9 mutants in different cell lines

To further assess whether the editing efficiency of SpCas9-Mut5 is superior to that of wild-type Cas9 and other mutants, validation experiments were conducted in HEK293T and HepG2 cells. The specific method was as follows:
(1) HEK293T and HepG2 cells were co-transfected with sgTTR-0 and different Cas9 mutants (SpCas9-Mut1 to Mut7);
(2) After 72 hours, cells were harvested and genomic DNA was extracted;
(3) A DNA fragment of about 200 bp surrounding the sgTTR-0 target site was amplified, and subjected to amplicon sequencing analysis;
(4) Concurrently, cell culture supernatant was collected and the TTR protein content in the supernatant was detected by ELISA.

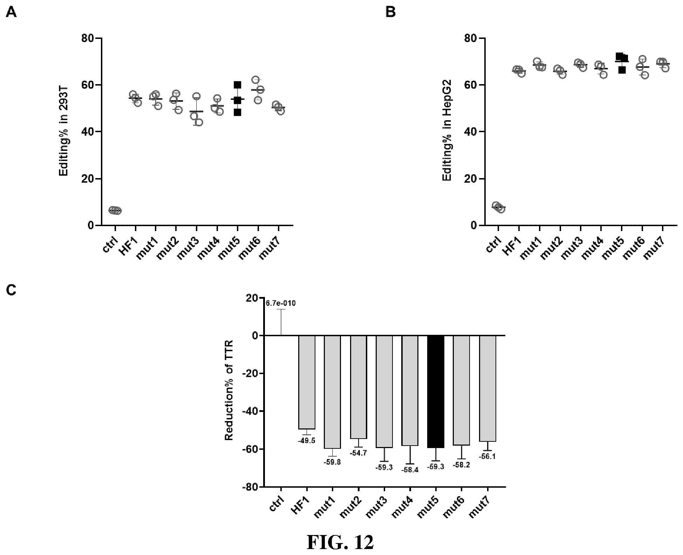

The results are shown in FIG. 12. Among them:
Panel A shows that in HEK293T cells, under the guidance of sgTTR-0, mut1-7 exhibited high editing efficiency (genomic level) on the TTR gene, ranging from 40% to 60%, which was comparable to the editing efficiency of the control group HF1 (about 55%).
Panel B shows that in HepG2 cells, under the guidance of sgTTR-0, the editing efficiency (genomic level) of mut1-7 on the TTR gene was comparable to that of the control group HF1 (about 66%).
Panel C shows that in HepG2 cells, under the guidance of sgTTR-0, mut1-7 strongly inhibited TTR protein expression (protein level). The level of TTR protein knockdown by mut1-7 (about 60% downregulation) was higher than that achieved by the control SpCas9-HF1 (about 49.5% downregulation).

### Example 6 Detection of off-target rates of SpCas9 mutants at high-frequency off-target sites

Following the prediction of high-frequency off-target sites for SpCas9-WT using the GUIDE-Seq technique, the off-target rates of SpCas9-Mut5 at four major high-frequency off-target sites were assessed to determine if they were lower. The specific method was as follows:
(1) HepG2 cells were co-transfected with sgTTR-0 and different Cas9 mutants;
(2) Cells were harvested 72 hours post-transfection, and genomic DNA was extracted;
(3) DNA fragments of about 200 flanking the high-frequency off-target site were amplified;
(4) Amplicon sequencing was performed on the amplified DNA fragments.

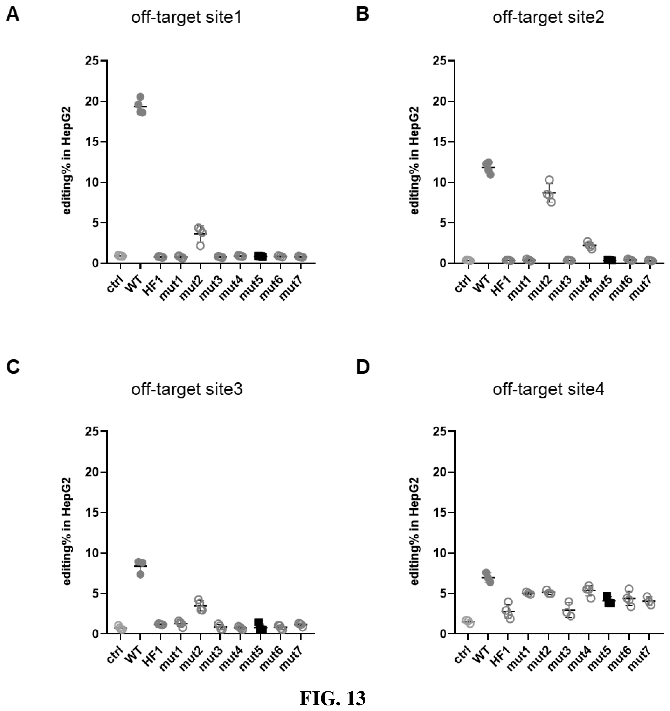

The editing efficiencies of wild-type SpCas9 and its mutants at four different off-target sites (off-target site 1/2/3/4) were analyzed, with the results shown in FIG. 13. Specifically:
Panel A shows that, guided by sgTTR-0, spCas9-WT exhibited an off-target editing efficiency of nearly 20% at off-target site1, while mut2 showed about 4%. All other Cas9 mutants (including mut5) reduced the off-target editing at this site to a level comparable to that of the control group (<1%). Regarding the editing efficiency at this off-target site, a statistically significant difference was observed between mut5 and WT (P<0.0001), as well as between mut5 and mut2 (P=0.0015). No statistically significant differences were found between mut5 and mut1/3/4/6/7.

The results presented in Panel B show that under the guidance of sgTTR-0, Cas9-WT exhibited an off-target editing efficiency exceeding 10% at off-target site 2, while mut2 showed nearly 10%, and mut4 about 2%. The remaining Cas9 mutants (including mut5) reduced off-target editing at this site to a level comparable to that of the control group (<0.5%). In terms of editing of this off-target site, mut5 showed a statistically significant difference compared with WT (P<0.0001), to mut2 (P<0.0001), and to mut4 (P<0.0001). No significant differences were observed between mut5 and mut1/3/6/7.

The results in Panel C show that under the guidance of sgTTR-0, Cas9-WT exhibited an off-target editing efficiency of nearly 10% at off-target site 3, while mut2 showed an efficiency of nearly 5%. All other Cas9 mutants (including mut5) reduced the off-target editing at this site to a level comparable to that of the control group (about 1%). In terms of the editing efficiency at this off-target site, a statistically significant difference was observed between mut5 and WT (P<0.0001), as well as between mut5 and mut2 (P=0.0015). No statistically significant differences were found between mut5 and mut1/3/4/6/7.

The results in Panel D show that under the guidance of sgTTR-0, Cas9-WT exhibited an off-target editing efficiency of approximately 7% at off-target site 4, while mut5 significantly reduced the editing at this off-target site (reduced to approximately 4%, p-value = 0.003).

Combined with the results from Example 3 (e.g., FIG.. 8 ), it can be concluded that compared with wild-type Cas9 and other mutants, SpCas9-Mut5 not only exhibits fewer off-target sites and a lower overall off-target rate, but also maintains a relatively lower off-target rate at various high-frequency off-target sites compared with the wild-type or other mutants.

### Example 7 Assessment of Cas9-mut5 editing efficiency at additional genomic loci

### 7.1 Evaluation of editing efficiency

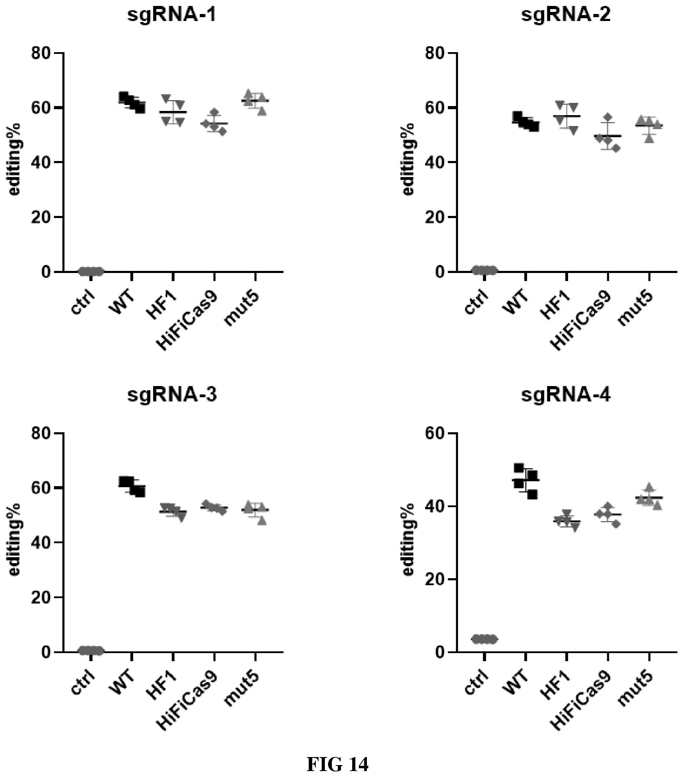

To investigate whether the editing efficiency of Cas9-mut5 at loci other than TTR is comparable to that of wild-type Cas9 and the reported high-efficiency and low-off-target editors, four distinct gRNAs (sgRNA-1/2/3/4) were selected. These gRNAs were individually co-transfected with mRNA encoding Cas9WT, Cas9-HF1, HiFi Cas9, or Cas9-Mut5 into HepG2 cells. 72 hours post-transfection, the cells were harvested, and DNA fragments of about 200 bp encompassing the respective target sites were amplified by PCR and subjected to amplicon sequencing analysis. The results are presented in FIG. 14. Specifically:
Panel A shows that under the guidance of sgRNA-1 (AGAGTCTCTCAGCTGGTACA, targeting TRAC), the editing efficiency of Cas9-mut5 is comparable to that of Cas9 WT/HF1/HiFiCas9, and their on-target editing efficiencies are all around 60%.
Panel B shows that under the guidance of sgRNA-2 (TCAGGGTTCTGGATATCTGT, target gene is TRAC), the editing efficiency of Cas9-mut5 is comparable to that of Cas9 WT/HF1/HiFiCas9, and their on-target editing efficiencies are all around 55%.
Panel C shows that under the guidance of sgRNA-3 (CTGGATATCTGTGGGACAAG, target gene is TRAC), the editing efficiency of Cas9-mut5 is comparable to that of Cas9 WT/HF1/HiFiCas9, and their on-target editing efficiencies are all around 60%.
Panel D shows that under the guidance of sgRNA-4 (ACGACGCGTGGGTGGCAAGC, target gene is REGNASE-1), the editing efficiency of Cas9-mut5 is comparable to that of Cas9 WT/HF1/HiFiCas9, and their on-target editing efficiencies are all around 40%.

The above results indicate that Mut5 also exhibits editing capabilities at sites other than TTR that are not inferior to those of the WT or previously reported high-efficiency and low-off-target editing tools.

### 7.2 Off-target rate assessment

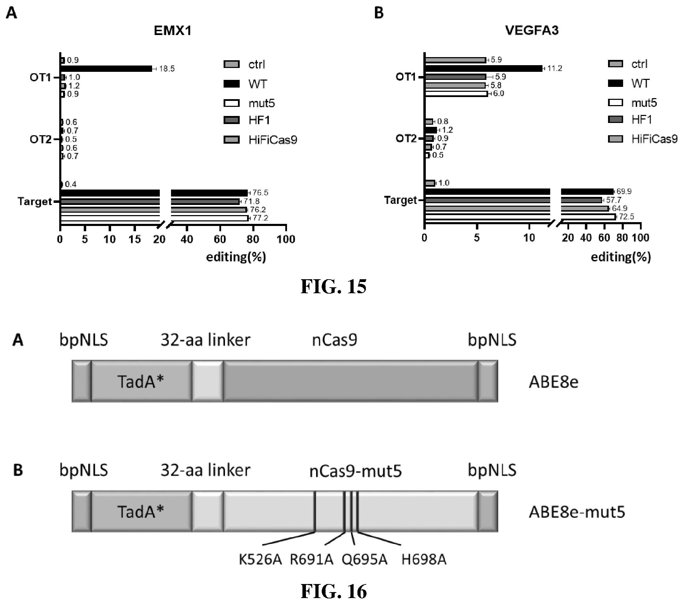

To investigate the off-target profiles of Cas9-mut5 when paired with a broader range of gRNAs, HEK293T cells were transfected via transfection reagent with Cas9 WT/HF1/HiFiCas9/mut5 (i.e., Cas9 WT, Cas9-HF1, HiFi-Cas9, or Cas9-mut5), each in combination with either EMX1 or VEGFA3 gRNA. The control group was only transfected with gRNAs, and the cells were harvested 72 hours post-transfection. DNA fragments of about 200 bp flanking the target sites were amplified and analyzed by next-generation sequencing. The results are shown in FIG. 15, where:
The results in Panel A show that under the guidance of the EMX1 gRNA, the on-target editing efficiencies of WT/HF1/HiFiCas9/mut5 for the EMX1 gene all exceeded 70%, with SpCas9-Mut5 exhibiting superior on-target editing efficiency compared with SpCas9-WT. Concurrently, mut5, like other high-efficiency and low-off-target editors, showed a significant reduction in off-target editing efficiency at the high-frequency off-target site OT1 of Sp-Cas9-WT (decreasing from 18.5% to 0.9%, P<0.0001), almost reaching the level of the control group. The reduction in off-target rate was most pronounced for SpCas9-Mut5.

The results in Panel B show that under the guidance of the VEGFA3 gRNA, the on-target editing efficiency of SpCas9-Mut5 for the VEGFA3 gene exceeded 70%, which was superior to that of SpCas9-WT. Meanwhile, the off-target rate of SpCas9-Mut5 at the high-frequency off-target site OT2 of SpCas9-WT was lower than that of SpCas9-WT and other mutants. Furthermore, SpCas9-Mut5 significantly reduced off-target editing at the high-frequency off-target site OT1 of SpCas9-WT (from 11.2% to 6.0%, P<0.0001).

Collectively, these findings demonstrate that, even in genes other than TTR, Mut5 exhibits relatively lower off-target rate and often achieves higher gene editing efficiency compared with SpCas9-WT and other low off-target Cas9 variants.

### Example 8 Editing effect assessment of SpCas9-Mut5 nikase (nCas9-Mut5) and Dead SpCas9-Mut5 (dCas9-Mut5)

Wild-type Cas9 can induce double stranded breaks in DNA due to its two nuclease domains: RuvC and HNH. The RuvC domain cleaves the non-target DNA strand, while the HNH domain cleaves the target DNA strand. If a mutation is introduced into the RuvC nuclease activity region to inactivate it, the resulting mutant nuclease can only cleave one strand of the dsDNA. This mutant form of Cas9 nickase is referred to as Cas9 nickase (nCas9). If mutations are simultaneously introduced into both the RuvC and HNH nuclease activity regions, the nuclease will lose its DNA cleavage ability but retain its ability to be guided into the genome by gRNA. This mutant form of Cas9 is termed dead Cas9 (dCas9).

As mentioned above, SpCas9-Mut5 generated by introducing four mutations (K526A/R691A/Q695A/H698A, referred to as the Mut5 quadruple mutation in this application) into the wild-type Cas9 nuclease, exhibited editing efficiency comparable to that of the wild-type at multiple targets, including TTR. This indicates that the mutation sites in SpCas9-Mut5 do not impair the ability of the nuclease to cleave on-target DNA. Meanwhile, the anti-off-target property of Mut5 is due to the fact that the Mut5 quadruple mutation reduces the electrostatic and hydrophobic interactions between the Cas9 protein and the phosphate backbone of genomic DNA. Since this interaction is independent of the DNA base sequence, the anti-off-target capability conferred by the Mut5 quadruple mutation is not sequence-specific.

To investigate whether the introduction of the mut5 quadruple mutation into nCas9 and dCas9 still results in high editing efficiency and low off-target effects, the examples of the present application respectively examined the on-target and off-target editing efficiencies of nCas9 fusion proteins (taking the single-base editor ABE8e as an example) and dCas9 fusion proteins (taking an epigenetic editor as an example) after incorporating the Mut5 quadruple mutation.

### 8.1 Application of the Mut5 quadruple mutation to nCas9 fusion proteins (single base editor) to reduce off-target levels while maintaining the original on-target editing efficiency

The fusion of the Cas9 nickase (nCas9) with cytidine deaminase from the APOBEC family or the adenine deaminase TadA yeilds cytosine base editors (CBEs), which mediate C-to-T base conversion, or adenine base editors (ABEs), which mediate A-to-G base conversion, respectively.

Taking the ABE (adenine base editor) as an example, the core components of the fusion protein are nCas9 (Cas9nickase, which harbors a D10A mutation compared with the wild-type Cas9 protein shown in SEQ ID NO.68) and the adenine deaminase TadA. When the fusion protein was guided by gRNA to target genomic DNA, the adenine deaminase binds to single-stranded DNA and deaminates adenine (A) within a specific window into inosine (I). Inosine will be recognized as a guanine (G) base during DNA replication, ultimately achieving the direct conversion of an A•T base pair to a G•C base pair.

The editing efficiency of the first-generation ABE editors (such as ABE7.10 and ABEmax) is low. To enhance editing efficiency, the David Liu group developed a novel ABE variant - ABE8e (structural schematic shown in FIG. 16A) - through molecular evolution of the eTadA monomer.

ABE8e showed high editing efficiency, with activity increased by 3 to 11 fold compared with ABE7.10. In this example, four mutations of mut5 were introduced into the nCas9 element of ABE8e, resulting in ABE8e-mut5 (structural schematic shown in FIG. 16B).

In this example, the bpNLS on the left side of the structures shown in FIG. 16A and FIG. 16B is referred to as the N-terminal signal peptide, and the bpNLS on the right side is referred to as the C-terminal signal peptide. The amino acid sequences of the aforementioned signal peptides, TadA*, 32-aa linker, nCas9-Mut5, ABE8e-WT (i.e., ABE8e in the FIG.) and ABE8e-Mut5 are all provided in the Sequence Listing.

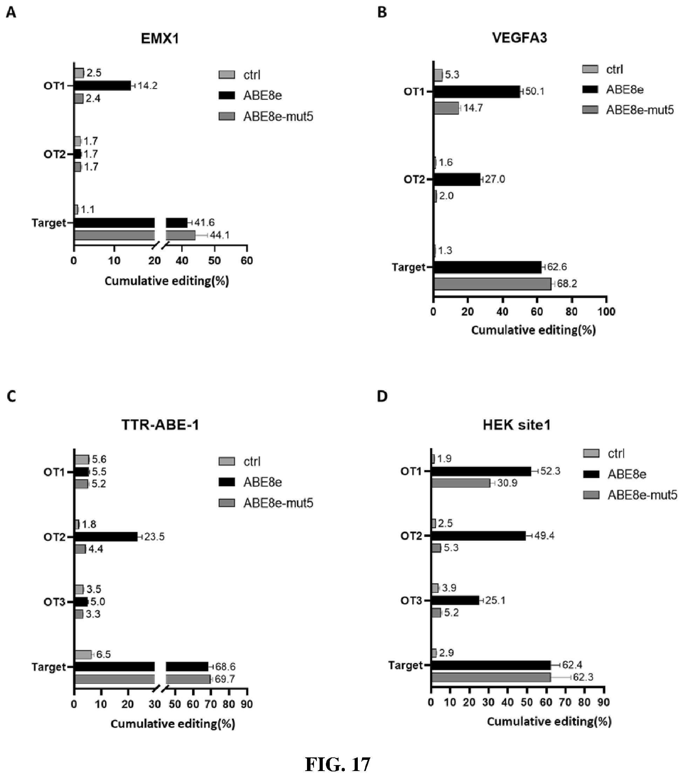

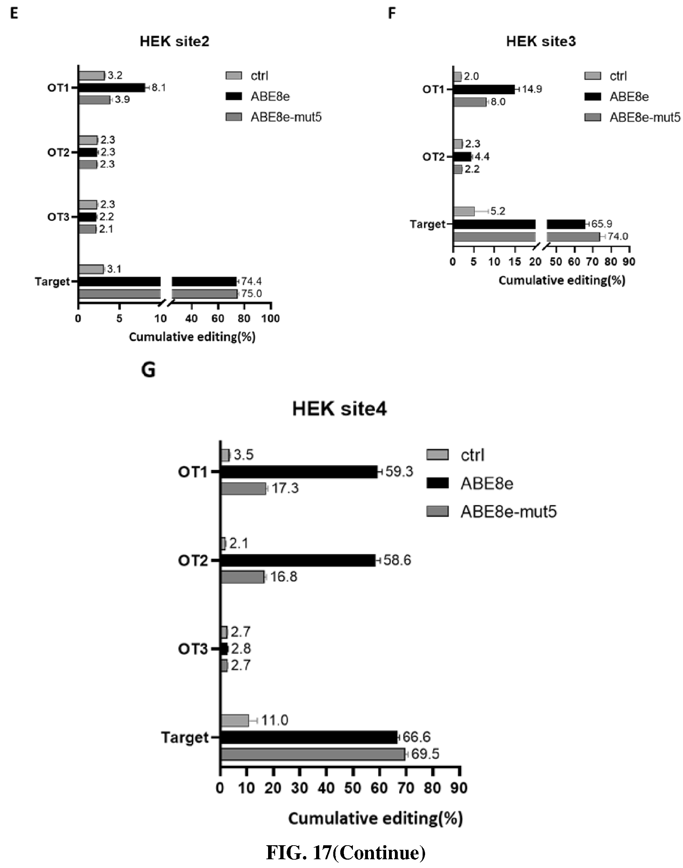

To investigate whether the introduction of the mut5 quadruple mutation enables nCas9 to reduce its off-target level while maintaining the original on-target editing efficiency, this example examined nCas9 with the introduced mut5 quadruple mutation in the ABE8e-mut5 system. In this experiment, HEK293T cells were transfected via transfection reagents with mRNA encoding either ABE8e-WT (amino acid sequence is SEQ ID NO.71, nucleotide sequence is SEQ ID NO.5) or ABE8e-mut5 (amino acid sequence is SEQ ID NO.72, nucleotide sequence is SEQ ID NO.6), each in combination with different gRNA. Cells were harvested 72h post-transfection. DNA fragments of about 200 bp surrounding the target site (Target) and relevant off-target sites (OT) were amplified and subjected to next-generation sequencing analysis. In this experiment, the control group was only transfected with gRNA, without mRNA. The results are shown in FIG. 17. The seven gRNAs selected for the experiment were all sgRNAs reported previously in literature or patents, and the related off-target sites were selected from high-frequency mutation sites identified in the GUIDE-seq data reported in literature or patents (Tsai, Zheng et al. 2015, Liang, Xie et al. 2019, Richter, Zhao et al. 2020 and WO2022246266A1), with the exception of HEK site2 OT2/OT3. Those two off-target sites were predicted using an online off-target prediction tool, accessible at http://www.rgenome.net/cas-offinder/. Wherein:
Panel A of FIG. 17 shows that when ABE8e and ABE8e-mut5 were respectively combined with the EMX1 gRNA (sgRNA targeting the EMX1 gene), their on-target editing efficiencies were comparable (both exceeding 40%), with no significant difference between the two groups. Regarding off-target effects, ABE8e-mut5 significantly reduced the off-target level at the OT1 site (from 14.2% to 2.4%, P<0.0001), while no significant effect was observed on the off-target level at the OT2 site.
Panel B of FIG. 17 shows that when ABE8e and ABE8e-mut5 were respectively combined with the VEGFA3 gRNA (sgRNA targeting the VEGFA3 gene), the on-target editing efficiency of ABE8e was 62.6%, while that of ABE8e-mut5 was 68.2%. This indicates that the on-target editing efficiency of ABE8e-mut5 at this site was significantly higher than that of ABE8e (P=0.0022). Regarding off-target effects, ABE8e-mut5 significantly reduced the off-target level at the OT1 site (from 50.1% to 14.7%, P<0.0001) and at the OT2 site (from 27% to 2%, P<0.0001).
Panel C of FIG. 17 shows that when ABE8e and ABE8e-mut5 were respectively combined with the TTR-ABE-1gRNA (sgRNA targeting the TTR gene), their on-target editing efficiencies are comparable (both exceeding 68%), with no statistically significant difference observed between the two groups. Regarding off-target effects, ABE8e-mut5 significantly reduced the off-target level at the OT2 site (from 23.5% to 4.4%, P<0.0001) and at the OT3 site (from 5.0% to 3.3%, P<0.0001), while no significant effect was observed on the off-target level at the OT1 site.
Panel D of FIG. 17 shows that when ABE8e and ABE8e-mut5 were respectively combined with the gRNA targeting HEK site1 (sgRNA targeting HEK site1), their on-target editing efficiencies are comparable (both about 62%). Regarding off-target effects, ABE8e-mut5 significantly reduced the off-target levels at the OT1 site (from 52.3% to 30.9%, P<0.0001), the OT2 site (from 49.4% to 5.3%, P<0.0001), and the OT3 site (from 25.1% to 5.2%, P<0.0001).
Panel E of FIG. 17 shows that when ABE8e and ABE8e-mut5 were respectively combined with the sgRNA targeting HEK site2, their on-target editing efficiencies were comparable (both exceeding 74%). Regarding off-target effects, ABE8e-mut5 significantly reduced the off-target level at the OT1 site (from 8.1% to 3.9%, P<0.0001), while no significant impact was observed on the off-target levels at OT2 and OT3.
Panel F of FIG. 17 shows that when ABE8e and ABE8e-mut5 were respectively combined with the sgRNA targeting HEK site3, ABE8e-mut5 exhibited a significant increase in on-target editing efficiency compared with ABE8e (from 65.9% to 74%, P = 0.001). Regarding off-target effects, ABE8e-mut5 significantly reduced the off-target level at the OT1 site (from 14.9% to 8.0%, P < 0.0001) and at the OT2 site (from 4.4% to 2.2%, P < 0.0001), while no significant effect was observed on the off-target level at the OT3 site.
Panel G of FIG. 17 shows that when ABE8e and ABE8e-mut5 were respectively combined with the sgRNA targeting HEK site4, ABE8e-mut5 exhibited a significant increase in on-target editing efficiency compared with ABE8e (from 66.6% to 69.5%, P = 0.0022). Regarding off-target effects, ABE8e-mut5 significantly reduced the off-target level at the OT1 site (from 59.3% to 17.3%, P < 0.0001) and at the OT2 site (from 58.6% to 16.8%, P < 0.0001), while no significant effect was observed on the off-target level at the OT3 site.

In summary, the results in FIG. 17 show that ABE8e-mut5 exhibits on-target editing efficiencies comparable to or even higher than those of ABE8e at multiple target sites, while simultaneously reducing the editing levels at the corresponding off-target sites. This proves that introducing the mut5 quadruple mutation into the nCas9 fusion protein enables maintenance of the original on-target editing efficiency while reducing off-target activity.

### 8.2 Application of Mut5 quadruple mutation to nCas9 fusion protein single-base editors reducing bystander editing effects

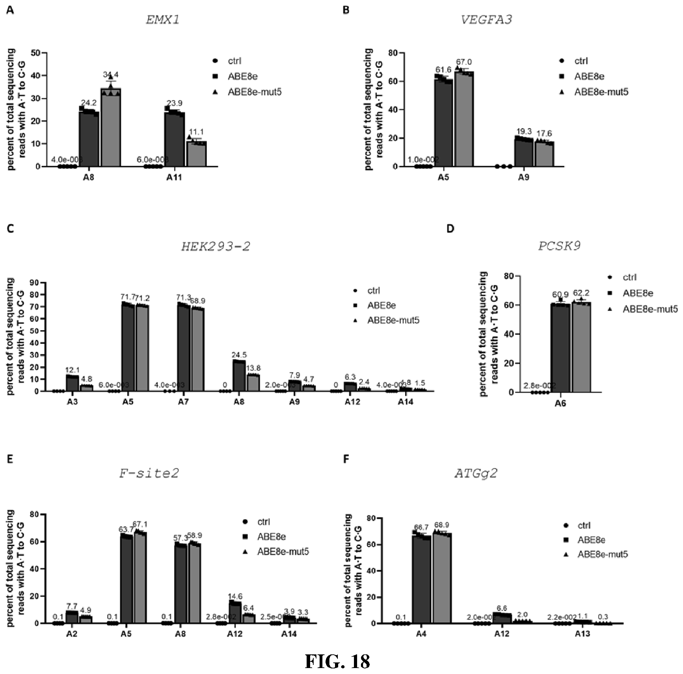

Compared with the first-generation ABE editors (such as ABE7.10, ABEmax), ABE8e exhibits improved editing activity. However, its editing window (the reagion susceptible to deamination) is further broadened (spanning positions A4 to A8, i.e., the A bases at positions 4 through 8). This broadening can lead to off-target base changes, resulting in a significant bystander editing effect. To investigate whether the introduction of the mut5 quadruple mutation could reduce the bystander editing effect of ABE8e, this example involved transfecting HEK293T cells with mRNA encoding ABE8e or ABE8e-mut5, respectively, in combination with different gRNAs using a transfection reagent. Cells were collected 72h post-transfection, and DNA fragments of about 200 bp surrounding the target sites were amplified. Following next-generation sequencing, the conversion rates from A to G at different positions within the 20-base region of the gRNA-paired region (as used herein, the "gRNA-paired region" refers to the double stranded DNA segment in the genome that is 100% complementary to the gRNA spacer sequence; when describing modifications within the gRNA-paired region, it refers to modifications in the portion identical to the gRNA spacer sequence) was analyzed. The results are shown in FIG. 18, among them:
Panel A shows that ABE8e-mut5 significantly improved the conversion efficiency of adenine (A) to guanine (G) at position 8 within the paired region of the EMX1 gRNA (sgRNA targeting the EMX1 gene) compared with ABE8e (from 24.2% to 34.4%, P value=0.0001), and significantly reducing the A-to-G conversion efficiency at position 11 (from 23.9% to 11.1%, P value<0.0001).
Panel B shows that compared with ABE8e, ABE8e-mut5 tends to improve the editing efficiency at the 5th base A within the paired region of the VEGFA3 gRNA (sgRNA targeting the VEGFA3 gene), with conversion to guanine (G) rising from 61.6% to 67% (P value = 0.024), and tends to reduce the editing efficiency at the 9th adenine (A) (decreasing from 19.3% to 17.6%, P value = 0.0174).
Panel C shows that the editing efficiencies of ABE8e-mut5 and ABE8e at the 5th base A within the gRNA paired region of HEK293_2sgRNA (see reference (Tsai, Zheng et al. 2015)) are comparable (both are about 70%, with no significant difference); the editing efficiency of ABE8e-mut5 at the 7th base A is slightly lower than that of ABE8e (from 71.3% to 68.9%, p value = 0.0066); and the A-to-G conversion rates at other positions within the gRNA pairing region, ABE8e-mut5 exhibited significantly lower values compared with ABE8e, specifically, the A to G conversion rate at the 3rd base (ABE8e: 12.1%; ABE8e-mut5: 4.8%; P value < 0.0001), the A to G conversion rate at the 8th base (ABE8e: 24.5%; ABE8e-mut5: 13.8%; P value < 0.0001), the A to G conversion rate at the 9th base (ABE8e:7.9%; ABE8e-mut5:4.7%; P value<0.0001), the A to G conversion rate at the 12th base (ABE8e:6.3%; ABE8e-mut5:2.4%; P value<0.0001), and the A to G conversion rate at the 14th base (ABE8e:1.8%; ABE8e-mut5:1.5%; P value=0.0187); at positions distal from the editing window, such as the 2nd and 16th A, both ABE8e-mut5 and ABE8e exhibited almost no editing activity.
Panel D shows that ABE8e-mut5 and ABE8e demonstrated comparable editing efficiencies (both greater than 60%, with no significant difference) for base A at the 6th position in the pairing region of PCSK9 gRNA (i.e., the sgRNA targeting the PCSK9 gene), and their editing efficiencies for the base A at the 16th position were both less than 1%.
Panel E showed that the editing efficiency of ABE8e-mut5 and ABE8e at the 5th base A and 8th base A in the pairing region of the F-site2 gRNA (targeting a non-coding region) is improved to a certain extent. Specifically, the A-to-G conversion rate were as follows: at the 5th base (ABE8e: 63.7%; ABE8e-mut5: 67.1%; P value = 0.0002), and at the 8th base (ABE8e: 57.3%; ABE8e-mut5: 58.9%; P value = 0.0126). In contrast, the A-to-G conversion rates for ABE8e-mut5 at other adenine positions within the same gRNA pairing region were significantly lower than those for ABE8e, including the 2nd base (ABE8e: 7.7%; ABE8e-mut5: 4.9%; P value <0.0001), the 12th base (ABE8e:14.6%; ABE8e-mut5:6.4%; P value<0.0001), and the 14th base (ABE8e:3.9%; ABE8e-mut5:3.3%; P value=0.0002). The editing efficiencies for both ABE8e-mut5 and ABE8e for base A at position 16 were less than 1%.
Panel F shows that ABE8e-mut5 and ABE8e exhibited comparable editing efficiencies (both greater than 66%, with no significant difference) at the A at position 4 within the pairing region of the ATGg2 gRNA (i.e., sgRNA targeting the ATGg2 gene). However, the A-to-G conversion rates at other A positions within the gRNA pairing region were significantly lower for ABE8e-mut5 than for ABE8e. Specifically, the conversion rate at position 12 was 6.6% for ABE8e versus 2.0% for ABE8e-mut5 (P value<0.0001), and at position 13 it was 1.1% for ABE8e versus 0.3% for ABE8e-mut5 (P value<0.0001). For A positions distal to the editing window, such as at positions 15, 16, and 19, the editing efficiencies of both ABE8e-mut5 and ABE8e were less than 1%.

In summary, the results in FIG. 18 show that within the core region of the editing window (A4-A8), the A-to-G conversion efficiencies of ABE8e-mut5 and ABE8e are comparable; while at the periphery of the editing window or outside the editing window, the A-to-G editing efficiency of ABE8e-mut5 is either lower than or similar to that of ABE8e. This means that by reducing its binding to off-target sites, ABE8e-mut5 can reduce unintended base modifications within the editing window, thereby effectively minimizing bystander editing while maintaining on-target editing efficiency.

### 8.3 The Mut5 quadruple mutation maintains the original on-target editing efficiency when applied to dCas9 fusion protein (epigenetic editor EE)

dCas9 is a catalytically inactive Cas9 protein that lacks DNA cleavage function but retains the ability to precisely target and bind DNA under the guidance of a gRNA. The combinations of CRISPR-dCas9 with epigenetic modifying enzymes (e.g., methyltransferases, or acetyltransferases, etc.) form dCas9-based epigenetic editing systems. Currently, common epigenetic modification enzymes used to activate target gene expression mainly include histone acetyltransferases and DNA demethyltransferases, such as p300 and Tet1; while the epigenetic modification enzymes that inhibit target gene expression mainly consist of DNA methyltransferases (e.g., DNMT3a), histone demethylases (e.g., LSD1), and histone deacetylases (e.g., HDAC3), etc.

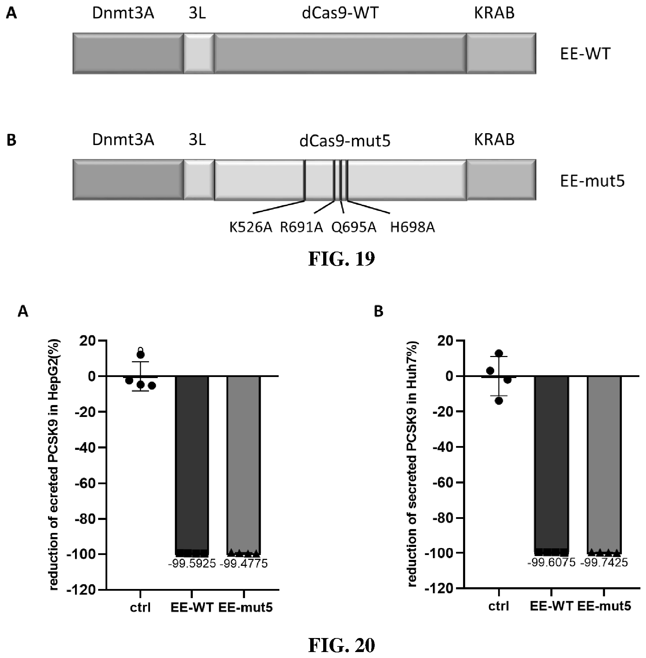

In this example, the epigenetic editor EE-WT, formed by the fusion of dCas9 and the DNA methyltransferase DNMT3a, is used as an example (the mutations in the dCas9 protein relative to the wild-type Cas9 protein are D10A and H840A, with a schematic structure shown in panel A of FIG. 19); four mutations of mut5 were introduced into its dCas9 element to obtain EE-mut5 (the schematic structure shown in panel B of FIG. 19).

Among them, the amino acid sequences of dCas9-WT, Dnmt3A+3L, KRAB, dCas9-mut5, EE-WT and EE-Mut5 used in this example are all shown in the sequence listing.

To investigate whether the introduction of mut5 quadruple mutation affects the editing efficiency of dCas9 and its fusion proteins, EE-WT mRNA/EE-mut5 mRNA and the gRNA targeting the PCSK9 gene (EE-PCSK9) were encapsulated into LNPs, respectively. The two types of LNPs were then used to treat hepatoma cell lines HepG2 and Huh7 at a dose of 500 ng / 2.5×10⁵ cell. The culture medium was changed or the cells were passaged every 2-4 days (cell counting was performed during passaging to ensure consistent cell numbers across different groups). 12 days post-transfection, the cell supernatant was collected and the concentration of PCSK9 protein in the supernatant was detected using ELISA kit. The results showed that in different hepatoma cell lines, both EE-mut5 and EE-WT downregulated the expression of PCSK9 protein by more than 99%. Specifically, both EE-mut5 and EE-WT effectively inhibited the expression of PCSK9 protein, and the extent of inhibition was comparable (the results are shown in FIG. 20).

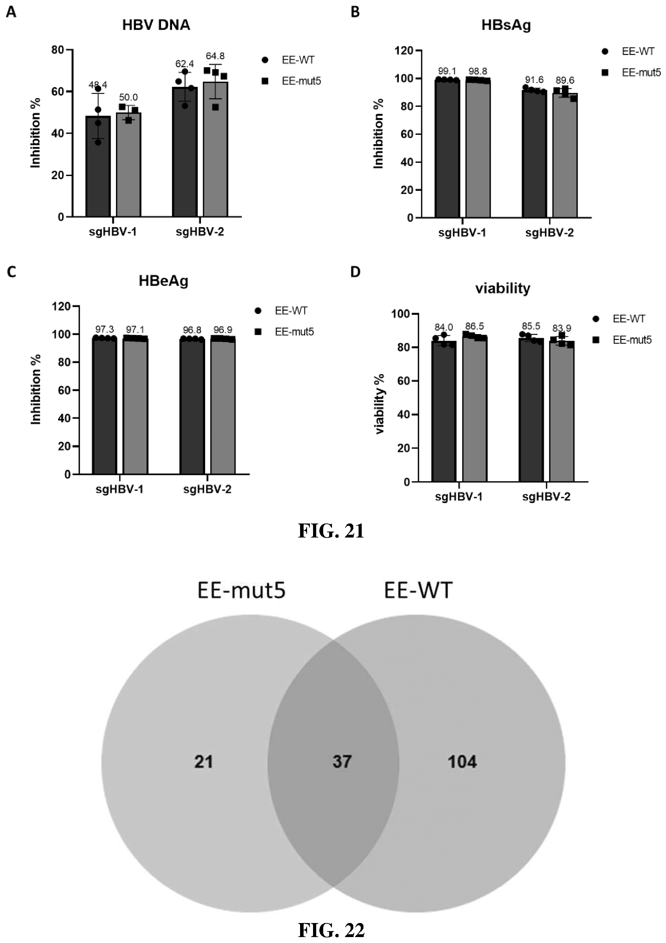

To further validate the efficacy of EE-mut5, gRNAs targeting HBV (sgHBV-1 and sgHBV-2) were separately encapsulated into LNPs with either EE-WT mRNA or EE-mut5 mRNA. These LNPs were then administered to HepG2.2.15 cells at a dose of 40 ng/2.25 × 10⁴ cells. The culture medium was replaced 2 days after transfection. 5 days post-transfection, the supernatant was collected for the detection of HBV DNA, HBsAg (HBV s antigen) and HBeAg (HBV e antigen), and cell viability was assessed using the cell-titer Glo assay. The results are shown in FIG. 21, where:
Panel A shows that under the guidance of sgHBV-1 (i.e., EE-sgHBV-1), the inhibitory rates of EE-mut5 and EE-WT on HBV DNA were comparable (both around 50%, with no significant difference). Under the guidance of sgHBV-2 (i.e., EE-sgHBV-2), the inhibitory rates of EE-mut5 and EE-WT on HBV DNA were also comparable (both over 60%, with no significant difference).
Panel B shows that under the guidance of sgHBV-1, the inhibitory rates of EE-mut5 and EE-WT on HBsAg were comparable (both exceeding 98%, with no significant difference); under the guidance of sgHBV-2, the inhibitory rates of EE-mut5 and EE-WT on HBsAg were also comparable (both around 90%, with no significant difference).
Panel C shows that under the guidance of sgHBV-1, the inhibition rates of HBeAg by EE-mut5 and EE-WT were comparable (both at 97%, with no significant difference); under the guidance of sgHBV-2, the inhibition rates of HBeAg by EE-mut5 and EE-WT were also comparable (both exceeding 96%, with no significant difference).
Panel D shows that there were no significant differences in the effects of treating cells with EE-mut5 and EE-WT in combination with different gRNAs on cell viability.

### 8.4 Application of the Mut5 quadruple mutation to dCas9 fusion proteins (epigenetic editor EE) results in lower off-target levels

To investigate whether the application of the mut5 quadruple mutation to an epigenetic editor would reduce its off-target levels, gRNA targeting HBV (sgHBV-1) was separately encapsulated into LNPs with either EE-WT mRNA or EE-mut5 mRNA. The control group LNPs comprised only the sgRNA. All three groups of LNPs were used to treat HepG2.2.15 cells at a dose of 1.5ug/8×10⁵ cells, with 3 replicates in each group. Cells were collected 7 days post-treatment, and whole-genome epigenetic sequencing was performed. The methylation level at CpG sites across the genome were analyzed for each group. Genes exhibiting differential methylation in the EE-WT and EE-mut5 groups relative to the control group were statistically analyzed. The results are shown in FIG. 22. The results indicated that compared with the control group, EE-WT exhibited 141 sites with significantly enhanced methylation levels, whereas EE-mut5 only showed 58 sites with significantly enhanced methylation (among which 37 differential sites overlapped between the EE-WT and EE-mut5 groups). This indicates that EE-mut5 can significantly reduce the methylation levels on the genome outside the target sites compared with EE-WT, that is, the application of Mut5 quadruple mutation to the dCas9 fusion protein epigenetic editor EE confers a lower off-target level.

The relevant sequences involved in this application are shown in the following sequence listing:

### Sequence Listing

| SEQ ID NO. | Name | Sequence (5 '-3' for nucleotide sequences; N-terminal to C-terminal for amino acid sequences) |
|---|---|---|
| Nucleotide sequences (mRNA sequences or DNA sequences encoding the mRNA sequences | | |
| 1 | SpCas9-WT | GGGAGACCCAAGCTGGCTAGCGTTTAAACTTAAGCTTGGTACCGAGCTCGGATCC ACTAGTCCAGTGTGGTGGAATTCGGGAAATAAGAGAGAAAAGAAGAGTAAGAAG |
| | mRNA | AAATATAAGAGCCACCATGGCCCCAAAGAAGAAGCGGAAGGTCGGTATCCACGG AGTCCCAGCAGCCGACAAGAAGTACAGCATCGGCCTGGACATCGGCACCAACTCT GTGGGCTGGGCCGTGATCACCGACGAGTACAAGGTGCCCAGCAAGAAATTCAAGG TGCTGGGCAACACCGACCGGCACAGCATCAAGAAGAACCTGATCGGAGCCCTGCT GTTCGACAGCGGCGAAACAGCCGAGGCCACCCGGCTGAAGAGAACCGCCAGAAG AAGATACACCAGACGGAAGAACCGGATCTGCTATCTGCAAGAGATCTTCAGCAAC GAGATGGCCAAGGTGGACGACAGCTTCTTCCACAGACTGGAAGAGTCCTTCCTGG TGGAAGAGGATAAGAAGCACGAGCGGCACCCCATCTTCGGCAACATCGTGGACG AGGTGGCCTACCACGAGAAGTACCCCACCATCTACCACCTGAGAAAGAAACTGGT GGACAGCACCGACAAGGCCGACCTGCGGCTGATCTATCTGGCCCTGGCCCACATG ATCAAGTTCCGGGGCCACTTCCTGATCGAGGGCGACCTGAACCCCGACAACAGCG ACGTGGACAAGCTGTTCATCCAGCTGGTGCAGACCTACAACCAGCTGTTCGAGGA AAACCCCATCAACGCCAGCGGCGTGGACGCCAAGGCCATCCTGTCTGCCAGACTG AGCAAGAGCAGACGGCTGGAAAATCTGATCGCCCAGCTGCCCGGCGAGAAGAAG AATGGCCTGTTCGGAAACCTGATTGCCCTGAGCCTGGGCCTGACCCCCAACTTCAA GAGCAACTTCGACCTGGCCGAGGATGCCAAACTGCAGCTGAGCAAGGACACCTAC GACGACGACCTGGACAACCTGCTGGCCCAGATCGGCGACCAGTACGCCGACCTGT TTCTGGCCGCCAAGAACCTGTCCGACGCCATCCTGCTGAGCGACATCCTGAGAGT GAACACCGAGATCACCAAGGCCCCCCTGAGCGCCTCTATGATCAAGAGATACGAC GAGCACCACCAGGACCTGACCCTGCTGAAAGCTCTCGTGCGGCAGCAGCTGCCTG AGAAGTACAAAGAGATTTTCTTCGACCAGAGCAAGAACGGCTACGCCGGCTACAT TGACGGCGGAGCCAGCCAGGAAGAGTTCTACAAGTTCATCAAGCCCATCCTGGAA AAGATGGACGGCACCGAGGAACTGCTCGTGAAGCTGAACAGAGAGGACCTGCTG CGGAAGCAGCGGACCTTCGACAACGGCAGCATCCCCCACCAGATCCACCTGGGAG AGCTGCACGCCATTCTGCGGCGGCAGGAAGATTTTTACCCATTCCTGAAGGACAA CCGGGAAAAGATCGAGAAGATCCTGACCTTCCGCATCCCCTACTACGTGGGCCCT CTGGCCAGGGGAAACAGCAGATTCGCCTGGATGACCAGAAAGAGCGAGGAAACC ATCACCCCCTGGAACTTCGAGGAAGTGGTGGACAAGGGCGCTTCCGCCCAGAGCT TCATCGAGCGGATGACCAACTTCGATAAGAACCTGCCCAACGAGAAGGTGCTGCC CAAGCACAGCCTGCTGTACGAGTACTTCACCGTGTATAACGAGCTGACCAAAGTG AAATACGTGACCGAGGGAATGAGAAAGCCCGCCTTCCTGAGCGGCGAGCAGAAA AAGGCCATCGTGGACCTGCTGTTCAAGACCAACCGGAAAGTGACCGTGAAGCAGC TGAAAGAGGACTACTTCAAGAAAATCGAGTGCTTCGACTCCGTGGAAATCTCCGG CGTGGAAGATCGGTTCAACGCCTCCCTGGGCACATACCACGATCTGCTGAAAATT ATCAAGGACAAGGACTTCCTGGACAATGAGGAAAACGAGGACATTCTGGAAGAT ATCGTGCTGACCCTGACACTGTTTGAGGACAGAGAGATGATCGAGGAACGGCTGA AAACCTATGCCCACCTGTTCGACGACAAAGTGATGAAGCAGCTGAAGCGGCGGAG ATACACCGGCTGGGGCAGGCTGAGCCGGAAGCTGATCAACGGCATCCGGGACAA GCAGTCCGGCAAGACAATCCTGGATTTCCTGAAGTCCGACGGCTTCGCCAACAGA AACTTCATGCAGCTGATCCACGACGACAGCCTGACCTTTAAAGAGGACATCCAGA AAGCCCAGGTGTCCGGCCAGGGCGATAGCCTGCACGAGCACATTGCCAATCTGGC CGGCAGCCCCGCCATTAAGAAGGGCATCCTGCAGACAGTGAAGGTGGTGGACGA GCTCGTGAAAGTGATGGGCCGGCACAAGCCCGAGAACATCGTGATCGAAATGGCC AGAGAGAACCAGACCACCCAGAAGGGACAGAAGAACAGCCGCGAGAGAATGAA |
| | | GCGGATCGAAGAGGGCATCAAAGAGCTGGGCAGCCAGATCCTGAAAGAACACCC CGTGGAAAACACCCAGCTGCAGAACGAGAAGCTGTACCTGTACTACCTGCAGAAT GGGCGGGATATGTACGTGGACCAGGAACTGGACATCAACCGGCTGTCCGACTACG ATGTGGACCATATCGTGCCTCAGAGCTTTCTGAAGGACGACTCCATCGACAACAA GGTGCTGACCAGAAGCGACAAGAACCGGGGCAAGAGCGACAACGTGCCCTCCGA AGAGGTCGTGAAGAAGATGAAGAACTACTGGCGGCAGCTGCTGAACGCCAAGCT GATTACCCAGAGAAAGTTCGACAATCTGACCAAGGCCGAGAGAGGCGGCCTGAG CGAACTGGATAAGGCCGGCTTCATCAAGAGACAGCTGGTGGAAACCCGGCAGATC ACAAAGCACGTGGCACAGATCCTGGACTCCCGGATGAACACTAAGTACGACGAGA ATGACAAGCTGATCCGGGAAGTGAAAGTGATCACCCTGAAGTCCAAGCTGGTGTC CGATTTCCGGAAGGATTTCCAGTTTTACAAAGTGCGCGAGATCAACAACTACCAC CACGCCCACGACGCCTACCTGAACGCCGTCGTGGGAACCGCCCTGATCAAAAAGT ACCCTAAGCTGGAAAGCGAGTTCGTGTACGGCGACTACAAGGTGTACGACGTGCG GAAGATGATCGCCAAGAGCGAGCAGGAAATCGGCAAGGCTACCGCCAAGTACTT CTTCTACAGCAACATCATGAACTTTTTCAAGACCGAGATTACCCTGGCCAACGGCG AGATCCGGAAGCGGCCTCTGATCGAGACAAACGGCGAAACCGGGGAGATCGTGT GGGATAAGGGCCGGGATTTTGCCACCGTGCGGAAAGTGCTGAGCATGCCCCAAGT GAATATCGTGAAAAAGACCGAGGTGCAGACAGGCGGCTTCAGCAAAGAGTCTATC CTGCCCAAGAGGAACAGCGATAAGCTGATCGCCAGAAAGAAGGACTGGGACCCT AAGAAGTACGGCGGCTTCGACAGCCCCACCGTGGCCTATTCTGTGCTGGTGGTGG CCAAAGTGGAAAAGGGCAAGTCCAAGAAACTGAAGAGTGTGAAAGAGCTGCTGG GGATCACCATCATGGAAAGAAGCAGCTTCGAGAAGAATCCCATCGACTTTCTGGA AGCCAAGGGCTACAAAGAAGTGAAAAAGGACCTGATCATCAAGCTGCCTAAGTA CTCCCTGTTCGAGCTGGAAAACGGCCGGAAGAGAATGCTGGCCTCTGCCGGCGAA CTGCAGAAGGGAAACGAACTGGCCCTGCCCTCCAAATATGTGAACTTCCTGTACC TGGCCAGCCACTATGAGAAGCTGAAGGGCTCCCCCGAGGATAATGAGCAGAAAC AGCTGTTTGTGGAACAGCACAAGCACTACCTGGACGAGATCATCGAGCAGATCAG CGAGTTCTCCAAGAGAGTGATCCTGGCCGACGCTAATCTGGACAAAGTGCTGTCC GCCTACAACAAGCACCGGGATAAGCCCATCAGAGAGCAGGCCGAGAATATCATCC ACCTGTTTACCCTGACCAATCTGGGAGCCCCTGCCGCCTTCAAGTACTTTGACACC ACCATCGACCGGAAGAGGTACACCAGCACCAAAGAGGTGCTGGACGCCACCCTG ATCCACCAGAGCATCACCGGCCTGTACGAGACACGGATCGACCTGTCTCAGCTGG GAGGCGACAAAAGGCCGGCGGCCACGAAAAAGGCCGGCCAGGCAAAAAAGAAA AAGTGATAATAGGCTGGAGCCTCGGTGGCCATGCTTCTTGCCCCTTGGGCCTCCCC CCAGCCCCTCCTCCCCTTCCTGCACCCGTACCCCCGTGGTCTTTGAATAAAGTCTG AGTGGGCGGCAAAAAAAAAAAAAAAAAAAAAAAAAAAAAAAGCATATGACTAA AAAAAAAAAAAAAAAAAAAAAAAAAAAAAAAAAAAAAAAAAAAAAAAAAAAAAA AAAAAAAAAAAAAAAAA |
| 2 | SpCas9-Mut5 mRNA | GGGAGACCCAAGCTGGCTAGCGTTTAAACTTAAGCTTGGTACCGAGCTCGGATCC ACTAGTCCAGTGTGGTGGAATTCGGGAAATAAGAGAGAAAAGAAGAGTAAGAAG AAATATAAGAGCCACCATGGCCCCTAAAAAAAAAAGAAAAGTTGGAATCCACGG CGTGCCCGCCGCCGACAAGAAGTACTCCATCGGCCTGGACATCGGCACCAACAGC GTGGGCTGGGCCGTGATCACCGACGAGTACAAGGTGCCCAGCAAGAAGTTCAAGG TGCTGGGCAACACCGACCGGCACAGCATCAAGAAGAACCTGATCGGCGCCCTGCT |
| | | GTTCGACAGCGGCGAGACCGCCGAGGCCACCCGGCTGAAGCGGACCGCCCGGCG GCGGTACACCCGGCGGAAGAACCGGATCTGCTACCTGCAGGAGATCTTCAGCAAC GAGATGGCCAAGGTGGACGACAGCTTCTTCCACCGGCTGGAGGAGAGCTTCCTGG TGGAGGAGGACAAGAAGCACGAGCGGCACCCCATCTTCGGCAACATCGTGGACG AGGTGGCCTACCACGAGAAGTACCCCACCATCTACCACCTGCGGAAGAAGCTGGT GGACAGCACCGACAAGGCCGACCTGCGGCTGATCTACCTGGCCCTGGCCCACATG ATCAAGTTCCGGGGCCACTTCCTGATCGAGGGCGACCTGAACCCCGACAACAGCG ACGTGGACAAGCTGTTCATCCAGCTGGTGCAGACCTACAACCAGCTGTTCGAGGA GAACCCCATCAACGCCAGCGGCGTGGACGCCAAGGCCATCCTGAGCGCCCGGCTG AGCAAGAGCCGGCGGCTGGAGAACCTGATCGCCCAGCTGCCCGGCGAGAAGAAG AACGGCCTGTTCGGCAACCTGATCGCCCTGAGCCTGGGCCTGACCCCCAACTTCAA GAGCAACTTCGACCTGGCCGAGGACGCCAAGCTGCAGCTGAGCAAGGACACCTAC GACGACGACCTGGACAACCTGCTGGCCCAGATCGGCGACCAGTACGCCGACCTGT TCCTGGCCGCCAAGAACCTGAGCGACGCCATCCTGCTGAGCGACATCCTGCGGGT GAACACCGAGATCACCAAGGCCCCCCTGAGCGCCAGCATGATCAAGCGGTACGAC GAGCACCACCAGGACCTGACCCTGCTGAAGGCCCTGGTGCGGCAGCAGCTGCCCG AGAAGTACAAGGAGATCTTCTTCGACCAGAGCAAGAACGGCTACGCCGGCTACAT CGACGGCGGCGCCAGCCAGGAGGAGTTCTACAAGTTCATCAAGCCCATCCTGGAG AAGATGGACGGCACCGAGGAGCTGCTGGTGAAGCTGAACCGGGAGGACCTGCTG CGGAAGCAGCGGACCTTCGACAACGGCAGCATCCCCCACCAGATCCACCTGGGCG AGCTGCACGCCATCCTGCGGCGGCAGGAGGACTTCTACCCCTTCCTGAAGGACAA CCGGGAGAAGATCGAGAAGATCCTGACCTTCCGGATCCCCTACTACGTGGGCCCC CTGGCCCGGGGCAACAGCCGGTTCGCCTGGATGACCCGGAAGAGCGAGGAGACC ATCACCCCCTGGAACTTCGAGGAGGTGGTGGACAAGGGCGCCAGCGCCCAGAGCT TCATCGAGCGGATGACCAACTTCGACAAGAACCTGCCCAACGAGAAGGTGCTGCC CAAGCACAGCCTGCTGTACGAGTACTTCACCGTGTACAACGAGCTGACCGCCGTG AAGTACGTGACCGAGGGCATGCGGAAGCCCGCCTTCCTGAGCGGCGAGCAGAAG AAGGCCATCGTGGACCTGCTGTTCAAGACCAACCGGAAGGTGACCGTGAAGCAGC TGAAGGAGGACTACTTCAAGAAGATCGAGTGCTTCGACAGCGTGGAGATCAGCGG CGTGGAGGACCGGTTCAACGCCAGCCTGGGCACCTACCACGACCTGCTGAAGATC ATCAAGGACAAGGACTTCCTGGACAACGAGGAGAACGAGGACATCCTGGAGGAC ATCGTGCTGACCCTGACCCTGTTCGAGGACCGGGAGATGATCGAGGAGCGGCTGA AGACCTACGCCCACCTGTTCGACGACAAGGTGATGAAGCAGCTGAAGCGGCGGCG GTACACCGGCTGGGGCCGCCTGAGCCGGAAGCTGATCAACGGCATCCGGGACAAG CAGAGCGGCAAGACCATCCTGGACTTCCTGAAGAGCGACGGCTTCGCCAACGCCA ACTTCATGGCCCTGATCGCCGACGACAGCCTGACCTTCAAGGAGGACATCCAGAA GGCCCAGGTGAGCGGCCAGGGCGACAGCCTGCACGAGCACATCGCCAACCTGGCC GGCAGCCCCGCCATCAAGAAGGGCATCCTGCAGACCGTGAAGGTGGTGGACGAG CTGGTGAAGGTGATGGGCCGGCACAAGCCCGAGAACATCGTGATCGAGATGGCCC GGGAGAACCAGACCACCCAGAAGGGCCAGAAGAACAGCCGGGAGCGGATGAAGC GGATCGAGGAGGGCATCAAGGAGCTGGGCAGCCAGATCCTGAAGGAGCACCCCG TGGAGAACACCCAGCTGCAGAACGAGAAGCTGTACCTGTACTACCTGCAGAACGG CCGGGACATGTACGTGGACCAGGAGCTGGACATCAACCGGCTGAGCGACTACGAC GTGGACCACATCGTGCCCCAGAGCTTCCTGAAGGACGACAGCATCGACAACAAGG |
| | | TGCTGACCCGGAGCGACAAGAACCGGGGCAAGAGCGACAACGTGCCCAGCGAGG AGGTGGTGAAGAAGATGAAGAACTACTGGCGGCAGCTGCTGAACGCCAAGCTGA TCACCCAGCGGAAGTTCGACAACCTGACCAAGGCCGAGCGGGGCGGCCTGAGCG AGCTGGACAAGGCCGGCTTCATCAAGCGGCAGCTGGTGGAGACCCGGCAGATCAC CAAGCACGTGGCCCAGATCCTGGACAGCCGGATGAACACCAAGTACGACGAGAA CGACAAGCTGATCCGGGAGGTGAAGGTGATCACCCTGAAGAGCAAGCTGGTGAG CGACTTCCGGAAGGACTTCCAGTTCTACAAGGTGCGGGAGATCAACAACTACCAC CACGCCCACGACGCCTACCTGAACGCCGTGGTGGGCACCGCCCTGATCAAGAAGT ACCCCAAGCTGGAGAGCGAGTTCGTGTACGGCGACTACAAGGTGTACGACGTGCG GAAGATGATCGCCAAGAGCGAGCAGGAGATCGGCAAGGCCACCGCCAAGTACTT CTTCTACAGCAACATCATGAACTTCTTCAAGACCGAGATCACCCTGGCCAACGGC GAGATCCGGAAGCGGCCCCTGATCGAGACCAACGGCGAGACCGGCGAGATCGTG TGGGACAAGGGCCGGGACTTCGCCACCGTGCGGAAGGTGCTGAGCATGCCCCAGG TGAACATCGTGAAGAAGACCGAGGTGCAGACCGGCGGCTTCAGCAAGGAGAGCA TCCTGCCCAAGCGGAACAGCGACAAGCTGATCGCCCGGAAGAAGGACTGGGACC CCAAGAAGTACGGCGGCTTCGACAGCCCCACCGTGGCCTACAGCGTGCTGGTGGT GGCCAAGGTGGAGAAGGGCAAGAGCAAGAAGCTGAAGAGCGTGAAGGAGCTGCT GGGCATCACCATCATGGAGCGGAGCAGCTTCGAGAAGAACCCCATCGACTTCCTG GAGGCCAAGGGCTACAAGGAGGTGAAGAAGGACCTGATCATCAAGCTGCCCAAG TACAGCCTGTTCGAGCTGGAGAACGGCCGGAAGCGGATGCTGGCCAGCGCCGGCG AGCTGCAGAAGGGCAACGAGCTGGCCCTGCCCAGCAAGTACGTGAACTTCCTGTA CCTGGCCAGCCACTACGAGAAGCTGAAGGGCAGCCCCGAGGACAACGAGCAGAA GCAGCTGTTCGTGGAGCAGCACAAGCACTACCTGGACGAGATCATCGAGCAGATC AGCGAGTTCAGCAAGCGGGTGATCCTGGCCGACGCCAACCTGGACAAGGTGCTGA GCGCCTACAACAAGCACCGGGACAAGCCCATCCGGGAGCAGGCCGAGAACATCA TCCACCTGTTCACCCTGACCAACCTGGGCGCCCCCGCCGCCTTCAAGTACTTCGAC ACCACCATCGACCGGAAGCGGTACACCAGCACCAAGGAGGTGCTGGACGCCACCC TGATCCACCAGAGCATCACCGGCCTGTACGAGACCCGGATCGACCTGAGCCAGCT GGGCGGCGACAAGCGGCCCGCCGCCACCAAGAAGGCCGGCCAGGCCAAGAAGAA GAAGTGATAATAGGCTGGAGCCTCGGTGGCCATGCTTCTTGCCCCTTGGGCCTCCC CCCAGCCCCTCCTCCCCTTCCTGCACCCGTACCCCCGTGGTCTTTGAATAAAGTCT GAGTGGGCGGCAAAAAAAAAAAAAAAAAAAAAAAAAAAAAAAGCATATGACTA AAAAAAAAAAAAAAAAAAAAAAAAAAAAAAAAAAAAAAAAAAAAAAAAAAAAAA AAAAAAAAAAAAAAAAAA |
| 3 | SpCas9-HF1 mRNA | GGGAGACCCAAGCTGGCTAGCGTTTAAACTTAAGCTTGGTACCGAGCTCGGATCC ACTAGTCCAGTGTGGTGGAATTCGGGAAATAAGAGAGAAAAGAAGAGTAAGAAG AAATATAAGAGCCACCATGGCCCCTAAAAAAAAAAGAAAAGTTGGAATCCACGG CGTGCCCGCCGCCGACAAGAAGTACAGCATCGGCCTGGACATCGGCACCAACAGC GTGGGCTGGGCCGTGATCACCGACGAGTACAAGGTGCCCAGCAAGAAGTTCAAGG TGCTGGGCAACACCGACCGGCACAGCATCAAGAAGAACCTGATCGGCGCCCTGCT GTTCGACAGCGGCGAGACCGCCGAGGCCACCCGGCTGAAGCGGACCGCCCGGCG GCGGTACACCCGGCGGAAGAACCGGATCTGCTACCTGCAGGAGATCTTCAGCAAC GAGATGGCCAAGGTGGACGACAGCTTCTTCCACCGGCTGGAGGAGAGCTTCCTGG TGGAGGAGGACAAGAAGCACGAGCGGCACCCCATCTTCGGCAACATCGTGGACG |
| | | AGGTGGCCTACCACGAGAAGTACCCCACCATCTACCACCTGCGGAAGAAGCTGGT GGACAGCACCGACAAGGCCGACCTGCGGCTGATCTACCTGGCCCTGGCCCACATG ATCAAGTTCCGGGGCCACTTCCTGATCGAGGGCGACCTGAACCCCGACAACAGCG ACGTGGACAAGCTGTTCATCCAGCTGGTGCAGACCTACAACCAGCTGTTCGAGGA GAACCCCATCAACGCCAGCGGCGTGGACGCCAAGGCCATCCTGAGCGCCCGGCTG AGCAAGAGCCGGCGGCTGGAGAACCTGATCGCCCAGCTGCCCGGCGAGAAGAAG AACGGCCTGTTCGGCAACCTGATCGCCCTGAGCCTGGGCCTGACCCCCAACTTCAA GAGCAACTTCGACCTGGCCGAGGACGCCAAGCTGCAGCTGAGCAAGGACACCTAC GACGACGACCTGGACAACCTGCTGGCCCAGATCGGCGACCAGTACGCCGACCTGT TCCTGGCCGCCAAGAACCTGAGCGACGCCATCCTGCTGAGCGACATCCTGCGGGT GAACACCGAGATCACCAAGGCCCCCCTGAGCGCCAGCATGATCAAGCGGTACGAC GAGCACCACCAGGACCTGACCCTGCTGAAGGCCCTGGTGCGGCAGCAGCTGCCCG AGAAGTACAAGGAGATCTTCTTCGACCAGAGCAAGAACGGCTACGCCGGCTACAT CGACGGCGGCGCCAGCCAGGAGGAGTTCTACAAGTTCATCAAGCCCATCCTGGAG AAGATGGACGGCACCGAGGAGCTGCTGGTGAAGCTGAACCGGGAGGACCTGCTG CGGAAGCAGCGGACCTTCGACAACGGCAGCATCCCCCACCAGATCCACCTGGGCG AGCTGCACGCCATCCTGCGGCGGCAGGAGGACTTCTACCCCTTCCTGAAGGACAA CCGGGAGAAGATCGAGAAGATCCTGACCTTCCGGATCCCCTACTACGTGGGCCCC CTGGCCCGGGGCAACAGCCGGTTCGCCTGGATGACCCGGAAGAGCGAGGAGACC ATCACCCCCTGGAACTTCGAGGAGGTGGTGGACAAGGGCGCCAGCGCCCAGAGCT TCATCGAGCGGATGACCGCCTTCGACAAGAACCTGCCCAACGAGAAGGTGCTGCC CAAGCACAGCCTGCTGTACGAGTACTTCACCGTGTACAACGAGCTGACCAAGGTG AAGTACGTGACCGAGGGCATGCGGAAGCCCGCCTTCCTGAGCGGCGAGCAGAAG AAGGCCATCGTGGACCTGCTGTTCAAGACCAACCGGAAGGTGACCGTGAAGCAGC TGAAGGAGGACTACTTCAAGAAGATCGAGTGCTTCGACAGCGTGGAGATCAGCGG CGTGGAGGACCGGTTCAACGCCAGCCTGGGCACCTACCACGACCTGCTGAAGATC ATCAAGGACAAGGACTTCCTGGACAACGAGGAGAACGAGGACATCCTGGAGGAC ATCGTGCTGACCCTGACCCTGTTCGAGGACCGGGAGATGATCGAGGAGCGGCTGA AGACCTACGCCCACCTGTTCGACGACAAGGTGATGAAGCAGCTGAAGCGGCGGCG GTACACCGGCTGGGGCGCCCTGAGCCGGAAGCTGATCAACGGCATCCGGGACAAG CAGAGCGGCAAGACCATCCTGGACTTCCTGAAGAGCGACGGCTTCGCCAACCGGA ACTTCATGGCCCTGATCCACGACGACAGCCTGACCTTCAAGGAGGACATCCAGAA GGCCCAGGTGAGCGGCCAGGGCGACAGCCTGCACGAGCACATCGCCAACCTGGCC GGCAGCCCCGCCATCAAGAAGGGCATCCTGCAGACCGTGAAGGTGGTGGACGAG CTGGTGAAGGTGATGGGCCGGCACAAGCCCGAGAACATCGTGATCGAGATGGCCC GGGAGAACCAGACCACCCAGAAGGGCCAGAAGAACAGCCGGGAGCGGATGAAGC GGATCGAGGAGGGCATCAAGGAGCTGGGCAGCCAGATCCTGAAGGAGCACCCCG TGGAGAACACCCAGCTGCAGAACGAGAAGCTGTACCTGTACTACCTGCAGAACGG CCGGGACATGTACGTGGACCAGGAGCTGGACATCAACCGGCTGAGCGACTACGAC GTGGACCACATCGTGCCCCAGAGCTTCCTGAAGGACGACAGCATCGACAACAAGG TGCTGACCCGGAGCGACAAGAACCGGGGCAAGAGCGACAACGTGCCCAGCGAGG AGGTGGTGAAGAAGATGAAGAACTACTGGCGGCAGCTGCTGAACGCCAAGCTGA TCACCCAGCGGAAGTTCGACAACCTGACCAAGGCCGAGCGGGGCGGCCTGAGCG AGCTGGACAAGGCCGGCTTCATCAAGCGGCAGCTGGTGGAGACCCGGGCCATCAC |
| | | CAAGCACGTGGCCCAGATCCTGGACAGCCGGATGAACACCAAGTACGACGAGAA CGACAAGCTGATCCGGGAGGTGAAGGTGATCACCCTGAAGAGCAAGCTGGTGAG CGACTTCCGGAAGGACTTCCAGTTCTACAAGGTGCGGGAGATCAACAACTACCAC CACGCCCACGACGCCTACCTGAACGCCGTGGTGGGCACCGCCCTGATCAAGAAGT ACCCCAAGCTGGAGAGCGAGTTCGTGTACGGCGACTACAAGGTGTACGACGTGCG CAAGATGATCGCCAAGAGCGAGCAGGAGATCGGCAAGGCCACCGCCAAGTACTT CTTCTACAGCAACATCATGAACTTCTTCAAGACCGAGATCACCCTGGCCAACGGC GAGATCCGGAAGCGGCCCCTGATCGAGACCAACGGCGAGACCGGCGAGATCGTG TGGGACAAGGGCCGGGACTTCGCCACCGTGCGGAAGGTGCTGAGCATGCCCCAGG TGAACATCGTGAAGAAGACCGAGGTGCAGACCGGCGGCTTCAGCAAGGAGTCCAT CCTGCCCAAGCGGAACAGCGACAAGCTGATCGCCCGGAAGAAGGACTGGGACCC CAAGAAGTACGGCGGCTTCGACAGCCCCACCGTGGCCTACAGCGTGCTGGTGGTG GCCAAGGTGGAGAAGGGCAAGAGCAAGAAGCTGAAGAGCGTGAAGGAGCTGCTG GGCATCACCATCATGGAGCGGAGCAGCTTCGAGAAGAACCCCATCGACTTCCTGG AGGCCAAGGGCTACAAGGAGGTGAAGAAGGACCTGATCATCAAGCTGCCCAAGT ACAGCCTGTTCGAGCTGGAGAACGGCCGGAAGCGGATGCTGGCCAGCGCCGGCG AGCTGCAGAAGGGCAACGAGCTGGCCCTGCCCAGCAAGTACGTGAACTTCCTGTA CCTGGCCAGCCACTACGAGAAGCTGAAGGGCAGCCCCGAGGACAACGAGCAGAA GCAGCTGTTCGTGGAGCAGCACAAGCACTACCTGGACGAGATCATCGAGCAGATC AGCGAGTTCAGCAAGCGGGTGATCCTGGCCGACGCCAACCTGGACAAGGTGCTGA GCGCCTACAACAAGCACCGGGACAAGCCCATCCGGGAGCAGGCCGAGAACATCA TCCACCTGTTCACCCTGACCAACCTGGGCGCCCCCGCCGCCTTCAAGTACTTCGAC ACCACCATCGACCGGAAGCGGTACACCAGCACCAAGGAGGTGCTGGACGCCACCC TGATCCACCAGAGCATCACCGGCCTGTACGAGACCCGGATCGACCTGAGCCAGCT GGGCGGCGACAAGCGGCCCGCCGCCACCAAGAAGGCCGGCCAGGCCAAGAAGAA GAAGTGATAATAGGCTGGAGCCTCGGTGGCCATGCTTCTTGCCCCTTGGGCCTCCC CCCAGCCCCTCCTCCCCTTCCTGCACCCGTACCCCCGTGGTCTTTGAATAAAGTCT GAGTGGGCGGCAAAAAAAAAAAAAAAAAAAAAAAAAAAAAAAGCATATGACTA AAAAAAAAAAAAAAAAAAAAAAAAAAAAAAAAAAAAAAAAAAAAAAAAAAAAAA AAAAAAAAAAAAAAAAAA |
| 4 | HiFi-Cas 9 mRNA | GGGAGACCCAAGCTGGCTAGCGTTTAAACTTAAGCTTGGTACCGAGCTCGGATCC ACTAGTCCAGTGTGGTGGAATTCGGGAAATAAGAGAGAAAAGAAGAGTAAGAAG AAATATAAGAGCCACCATGGCCCCAAAGAAGAAGCGGAAGGTCGGTATCCACGG AGTCCCAGCAGCCGACAAGAAGTACAGCATCGGCCTGGACATCGGCACCAACTCT GTGGGCTGGGCCGTGATCACCGACGAGTACAAGGTGCCCAGCAAGAAATTCAAGG TGCTGGGCAACACCGACCGGCACAGCATCAAGAAGAACCTGATCGGAGCCCTGCT GTTCGACAGCGGCGAAACAGCCGAGGCCACCCGGCTGAAGAGAACCGCCAGAAG AAGATACACCAGACGGAAGAACCGGATCTGCTATCTGCAAGAGATCTTCAGCAAC GAGATGGCCAAGGTGGACGACAGCTTCTTCCACAGACTGGAAGAGTCCTTCCTGG TGGAAGAGGATAAGAAGCACGAGCGGCACCCCATCTTCGGCAACATCGTGGACG AGGTGGCCTACCACGAGAAGTACCCCACCATCTACCACCTGAGAAAGAAACTGGT GGACAGCACCGACAAGGCCGACCTGCGGCTGATCTATCTGGCCCTGGCCCACATG ATCAAGTTCCGGGGCCACTTCCTGATCGAGGGCGACCTGAACCCCGACAACAGCG ACGTGGACAAGCTGTTCATCCAGCTGGTGCAGACCTACAACCAGCTGTTCGAGGA |
| | | AAACCCCATCAACGCCAGCGGCGTGGACGCCAAGGCCATCCTGTCTGCCAGACTG AGCAAGAGCAGACGGCTGGAAAATCTGATCGCCCAGCTGCCCGGCGAGAAGAAG AATGGCCTGTTCGGAAACCTGATTGCCCTGAGCCTGGGCCTGACCCCCAACTTCAA GAGCAACTTCGACCTGGCCGAGGATGCCAAACTGCAGCTGAGCAAGGACACCTAC GACGACGACCTGGACAACCTGCTGGCCCAGATCGGCGACCAGTACGCCGACCTGT TTCTGGCCGCCAAGAACCTGTCCGACGCCATCCTGCTGAGCGACATCCTGAGAGT GAACACCGAGATCACCAAGGCCCCCCTGAGCGCCTCTATGATCAAGAGATACGAC GAGCACCACCAGGACCTGACCCTGCTGAAAGCTCTCGTGCGGCAGCAGCTGCCTG AGAAGTACAAAGAGATTTTCTTCGACCAGAGCAAGAACGGCTACGCCGGCTACAT TGACGGCGGAGCCAGCCAGGAAGAGTTCTACAAGTTCATCAAGCCCATCCTGGAA AAGATGGACGGCACCGAGGAACTGCTCGTGAAGCTGAACAGAGAGGACCTGCTG CGGAAGCAGCGGACCTTCGACAACGGCAGCATCCCCCACCAGATCCACCTGGGAG AGCTGCACGCCATTCTGCGGCGGCAGGAAGATTTTTACCCATTCCTGAAGGACAA CCGGGAAAAGATCGAGAAGATCCTGACCTTCCGCATCCCCTACTACGTGGGCCCT CTGGCCAGGGGAAACAGCAGATTCGCCTGGATGACCAGAAAGAGCGAGGAAACC ATCACCCCCTGGAACTTCGAGGAAGTGGTGGACAAGGGCGCTTCCGCCCAGAGCT TCATCGAGCGGATGACCAACTTCGATAAGAACCTGCCCAACGAGAAGGTGCTGCC CAAGCACAGCCTGCTGTACGAGTACTTCACCGTGTATAACGAGCTGACCAAAGTG AAATACGTGACCGAGGGAATGAGAAAGCCCGCCTTCCTGAGCGGCGAGCAGAAA AAGGCCATCGTGGACCTGCTGTTCAAGACCAACCGGAAAGTGACCGTGAAGCAGC TGAAAGAGGACTACTTCAAGAAAATCGAGTGCTTCGACTCCGTGGAAATCTCCGG CGTGGAAGATCGGTTCAACGCCTCCCTGGGCACATACCACGATCTGCTGAAAATT ATCAAGGACAAGGACTTCCTGGACAATGAGGAAAACGAGGACATTCTGGAAGAT ATCGTGCTGACCCTGACACTGTTTGAGGACAGAGAGATGATCGAGGAACGGCTGA AAACCTATGCCCACCTGTTCGACGACAAAGTGATGAAGCAGCTGAAGCGGCGGAG ATACACCGGCTGGGGCAGGCTGAGCCGGAAGCTGATCAACGGCATCCGGGACAA GCAGTCCGGCAAGACAATCCTGGATTTCCTGAAGTCCGACGGCTTCGCCAACGCC AACTTCATGCAGCTGATCCACGACGACAGCCTGACCTTTAAAGAGGACATCCAGA AAGCCCAGGTGTCCGGCCAGGGCGATAGCCTGCACGAGCACATTGCCAATCTGGC CGGCAGCCCCGCCATTAAGAAGGGCATCCTGCAGACAGTGAAGGTGGTGGACGA GCTCGTGAAAGTGATGGGCCGGCACAAGCCCGAGAACATCGTGATCGAAATGGCC AGAGAGAACCAGACCACCCAGAAGGGACAGAAGAACAGCCGCGAGAGAATGAA GCGGATCGAAGAGGGCATCAAAGAGCTGGGCAGCCAGATCCTGAAAGAACACCC CGTGGAAAACACCCAGCTGCAGAACGAGAAGCTGTACCTGTACTACCTGCAGAAT GGGCGGGATATGTACGTGGACCAGGAACTGGACATCAACCGGCTGTCCGACTACG ATGTGGACCATATCGTGCCTCAGAGCTTTCTGAAGGACGACTCCATCGACAACAA GGTGCTGACCAGAAGCGACAAGAACCGGGGCAAGAGCGACAACGTGCCCTCCGA AGAGGTCGTGAAGAAGATGAAGAACTACTGGCGGCAGCTGCTGAACGCCAAGCT GATTACCCAGAGAAAGTTCGACAATCTGACCAAGGCCGAGAGAGGCGGCCTGAG CGAACTGGATAAGGCCGGCTTCATCAAGAGACAGCTGGTGGAAACCCGGCAGATC ACAAAGCACGTGGCACAGATCCTGGACTCCCGGATGAACACTAAGTACGACGAGA ATGACAAGCTGATCCGGGAAGTGAAAGTGATCACCCTGAAGTCCAAGCTGGTGTC CGATTTCCGGAAGGATTTCCAGTTTTACAAAGTGCGCGAGATCAACAACTACCAC CACGCCCACGACGCCTACCTGAACGCCGTCGTGGGAACCGCCCTGATCAAAAAGT |
| | | ACCCTAAGCTGGAAAGCGAGTTCGTGTACGGCGACTACAAGGTGTACGACGTGCG GAAGATGATCGCCAAGAGCGAGCAGGAAATCGGCAAGGCTACCGCCAAGTACTT CTTCTACAGCAACATCATGAACTTTTTCAAGACCGAGATTACCCTGGCCAACGGCG AGATCCGGAAGCGGCCTCTGATCGAGACAAACGGCGAAACCGGGGAGATCGTGT GGGATAAGGGCCGGGATTTTGCCACCGTGCGGAAAGTGCTGAGCATGCCCCAAGT GAATATCGTGAAAAAGACCGAGGTGCAGACAGGCGGCTTCAGCAAAGAGTCTATC CTGCCCAAGAGGAACAGCGATAAGCTGATCGCCAGAAAGAAGGACTGGGACCCT AAGAAGTACGGCGGCTTCGACAGCCCCACCGTGGCCTATTCTGTGCTGGTGGTGG CCAAAGTGGAAAAGGGCAAGTCCAAGAAACTGAAGAGTGTGAAAGAGCTGCTGG GGATCACCATCATGGAAAGAAGCAGCTTCGAGAAGAATCCCATCGACTTTCTGGA AGCCAAGGGCTACAAAGAAGTGAAAAAGGACCTGATCATCAAGCTGCCTAAGTA CTCCCTGTTCGAGCTGGAAAACGGCCGGAAGAGAATGCTGGCCTCTGCCGGCGAA CTGCAGAAGGGAAACGAACTGGCCCTGCCCTCCAAATATGTGAACTTCCTGTACC TGGCCAGCCACTATGAGAAGCTGAAGGGCTCCCCCGAGGATAATGAGCAGAAAC AGCTGTTTGTGGAACAGCACAAGCACTACCTGGACGAGATCATCGAGCAGATCAG CGAGTTCTCCAAGAGAGTGATCCTGGCCGACGCTAATCTGGACAAAGTGCTGTCC GCCTACAACAAGCACCGGGATAAGCCCATCAGAGAGCAGGCCGAGAATATCATCC ACCTGTTTACCCTGACCAATCTGGGAGCCCCTGCCGCCTTCAAGTACTTTGACACC ACCATCGACCGGAAGAGGTACACCAGCACCAAAGAGGTGCTGGACGCCACCCTG ATCCACCAGAGCATCACCGGCCTGTACGAGACACGGATCGACCTGTCTCAGCTGG GAGGCGACAAAAGGCCGGCGGCCACGAAAAAGGCCGGCCAGGCAAAAAAGAAA AAGTGATAATAGGCTGGAGCCTCGGTGGCCATGCTTCTTGCCCCTTGGGCCTCCCC CCAGCCCCTCCTCCCCTTCCTGCACCCGTACCCCCGTGGTCTTTGAATAAAGTCTG AGTGGGCGGCAAAAAAAAAAAAAAAAAAAAAAAAAAAAAAAAAAGCATATGACTAA AAAAAAAAAAAAAAAAAAAAAAAAAAAAAAAAAAAAAAAAAAAAAAAAAAAAAAA AAAAAAAAAAAAAAAAA |
| 5 | ABE8e-WT mRNA | GGGAGACCCAAGCTGGCTAGCGTTTAAACTTAAGCTTGGTACCGAGCTCGGATCC ACTAGTCCAGTGTGGTGGAATTCGGGAAATAAGAGAGAAAAGAAGAGTAAGAAG AAATATAAGAGCCACCATGAAGAGAACAGCTGATGGAAGTGAATTTGAGTCCCCC AAGAAGAAGCGGAAGGTGAGCGAGGTGGAGTTCAGCCACGAGTACTGGATGCGG CACGCCCTGACCCTGGCCAAGCGGGCCCGGGACGAGCGGGAGGTGCCCGTGGGC GCCGTGCTGGTGCTGAACAACCGGGTGATCGGCGAGGGCTGGAACCGGGCCATCG GCCTGCACGACCCCACCGCCCACGCCGAGATCATGGCCCTGCGGCAGGGCGGCCT GGTGATGCAGAACTACCGGCTGATCGACGCCACCCTGTACGTGACCTTCGAGCCC TGCGTGATGTGCGCCGGCGCCATGATCCACAGCCGGATCGGCCGGGTGGTGTTCG GCGTGCGGAACAGCAAGCGGGGCGCCGCCGGCAGCCTGATGAACGTGCTGAACT ACCCCGGCATGAACCACCGGGTGGAGATCACCGAGGGCATCCTGGCCGACGAGTG CGCCGCCCTGCTGTGCGACTTCTACCGGATGCCCCGGCAGGTGTTCAACGCCCAGA AGAAGGCCCAGAGCAGCATCAACAGCGGCGGCAGCAGCGGCGGCAGCAGCGGCA GCGAGACCCCCGGCACCTCCGAGAGCGCCACCCCCGAGAGCTCCGGCGGCAGCAG CGGCGGCAGCGACAAGAAGTACAGCATCGGCCTGGCCATCGGCACCAACAGCGT GGGCTGGGCCGTGATCACCGACGAGTACAAGGTGCCCAGCAAGAAGTTCAAGGTG CTGGGCAACACCGACCGGCACAGCATCAAGAAGAACCTGATCGGCGCCCTGCTGT TCGACAGCGGCGAGACCGCCGAGGCCACCCGGCTGAAGCGGACCGCCCGGCGGC |
| | | GGTACACCCGGCGGAAGAACCGGATCTGCTACCTGCAGGAGATCTTCAGCAACGA GATGGCCAAGGTGGACGACAGCTTCTTCCACCGGCTGGAGGAGAGCTTCCTGGTG GAGGAGGACAAGAAGCACGAGCGGCACCCCATCTTCGGCAACATCGTGGACGAG GTGGCCTACCACGAGAAGTACCCCACCATCTACCACCTGCGGAAGAAGCTGGTGG ACAGCACCGACAAGGCCGACCTGCGGCTGATCTACCTGGCCCTGGCCCACATGAT CAAGTTCCGGGGCCACTTCCTGATCGAGGGCGACCTGAACCCCGACAACAGCGAC GTGGACAAGCTGTTCATCCAGCTGGTGCAGACCTACAACCAGCTGTTCGAGGAGA ACCCCATCAACGCCAGCGGCGTGGACGCCAAGGCCATCCTGAGCGCCCGGCTGAG CAAGAGCCGGCGGCTGGAGAACCTGATCGCCCAGCTGCCCGGCGAGAAGAAGAA CGGCCTGTTCGGCAACCTGATCGCCCTGAGCCTGGGCCTGACCCCCAACTTCAAGA GCAACTTCGACCTGGCCGAGGACGCCAAGCTGCAGCTGAGCAAGGACACCTACGA CGACGACCTGGACAACCTGCTGGCCCAGATCGGCGACCAGTACGCCGACCTGTTC CTGGCCGCCAAGAACCTGAGCGACGCCATCCTGCTGAGCGACATCCTGCGGGTGA ACACCGAGATCACCAAGGCCCCCCTGAGCGCCAGCATGATCAAGCGGTACGACGA GCACCACCAGGACCTGACCCTGCTGAAGGCCCTGGTGCGGCAGCAGCTGCCCGAG AAGTACAAGGAGATCTTCTTCGACCAGAGCAAGAACGGCTACGCCGGCTACATCG ACGGCGGCGCCAGCCAGGAGGAGTTCTACAAGTTCATCAAGCCCATCCTGGAGAA GATGGACGGCACCGAGGAGCTGCTGGTGAAGCTGAACCGGGAGGACCTGCTGCG GAAGCAGCGGACCTTCGACAACGGCAGCATCCCCCACCAGATCCACCTGGGCGAG CTGCACGCCATCCTGCGGCGGCAGGAGGACTTCTACCCCTTCCTGAAGGACAACC GGGAGAAGATCGAGAAGATCCTGACCTTCCGGATCCCCTACTACGTGGGCCCCCT GGCCCGGGGCAACAGCCGGTTCGCCTGGATGACCCGGAAGAGCGAGGAGACCAT CACCCCCTGGAACTTCGAGGAGGTGGTGGACAAGGGCGCCAGCGCCCAGAGCTTC ATCGAGCGGATGACCAACTTCGACAAGAACCTGCCCAACGAGAAGGTGCTGCCCA AGCACAGCCTGCTGTACGAGTACTTCACCGTGTACAACGAGCTGACCAAGGTGAA GTACGTGACCGAGGGCATGCGGAAGCCCGCCTTCCTGAGCGGCGAGCAGAAGAA GGCCATCGTGGACCTGCTGTTCAAGACCAACCGGAAGGTGACCGTGAAGCAGCTG AAGGAGGACTACTTCAAGAAGATCGAGTGCTTCGACAGCGTGGAGATCAGCGGCG TGGAGGACCGGTTCAACGCCAGCCTGGGCACCTACCACGACCTGCTGAAGATCAT CAAGGACAAGGACTTCCTGGACAACGAGGAGAACGAGGACATCCTGGAGGACAT CGTGCTGACCCTGACCCTGTTCGAGGACCGGGAGATGATCGAGGAGCGGCTGAAG ACCTACGCCCACCTGTTCGACGACAAGGTGATGAAGCAGCTGAAGCGGCGGCGGT ACACCGGCTGGGGCCGGCTGAGCCGGAAGCTGATCAACGGCATCCGGGACAAGC AGAGCGGCAAGACCATCCTGGACTTCCTGAAGAGCGACGGCTTCGCCAACCGGAA CTTCATGCAGCTGATCCACGACGACAGCCTGACCTTCAAGGAGGACATCCAGAAG GCCCAGGTGAGCGGCCAGGGCGACAGCCTGCACGAGCACATCGCCAACCTGGCCG GCAGCCCCGCCATCAAGAAGGGCATCCTGCAGACCGTGAAGGTGGTGGACGAGCT GGTGAAGGTGATGGGCCGGCACAAGCCCGAGAACATCGTGATCGAGATGGCCCG GGAGAACCAGACCACCCAGAAGGGCCAGAAGAACAGCCGGGAGCGGATGAAGCG GATCGAGGAGGGCATCAAGGAGCTGGGCAGCCAGATCCTGAAGGAGCACCCCGT GGAGAACACCCAGCTGCAGAACGAGAAGCTGTACCTGTACTACCTGCAGAACGGC CGGGACATGTACGTGGACCAGGAGCTGGACATCAACCGGCTGAGCGACTACGACG TGGACCACATCGTGCCCCAGAGCTTCCTGAAGGACGACAGCATCGACAACAAGGT GCTGACCCGGAGCGACAAGAACCGGGGCAAGAGCGACAACGTGCCCAGCGAGGA |
| | | GGTGGTGAAGAAGATGAAGAACTACTGGCGGCAGCTGCTGAACGCCAAGCTGATC ACCCAGCGGAAGTTCGACAACCTGACCAAGGCCGAGCGGGGCGGCCTGAGCGAG CTGGACAAGGCCGGCTTCATCAAGCGGCAGCTGGTGGAGACCCGGCAGATCACCA AGCACGTGGCCCAGATCCTGGACAGCCGGATGAACACCAAGTACGACGAGAACG ACAAGCTGATCCGGGAGGTGAAGGTGATCACCCTGAAGAGCAAGCTGGTGAGCG ACTTCCGGAAGGACTTCCAGTTCTACAAGGTGCGGGAGATCAACAACTACCACCA CGCCCACGACGCCTACCTGAACGCCGTGGTGGGCACCGCCCTGATCAAGAAGTAC CCCAAGCTGGAGAGCGAGTTCGTGTACGGCGACTACAAGGTGTACGACGTGCGGA AGATGATCGCCAAGAGCGAGCAGGAGATCGGCAAGGCCACCGCCAAGTACTTCTT CTACAGCAACATCATGAACTTCTTCAAGACCGAGATCACCCTGGCCAACGGCGAG ATCCGGAAGCGGCCCCTGATCGAGACCAACGGCGAGACCGGCGAGATCGTGTGG GACAAGGGCCGGGACTTCGCCACCGTGCGGAAGGTGCTGAGCATGCCCCAGGTGA ACATCGTGAAGAAGACCGAGGTGCAGACCGGCGGCTTCAGCAAGGAGAGCATCC TGCCCAAGCGGAACAGCGACAAGCTGATCGCCCGGAAGAAGGACTGGGACCCCA AGAAGTACGGCGGCTTCGACAGCCCCACCGTGGCCTACAGCGTGCTGGTGGTGGC CAAGGTGGAGAAGGGCAAGAGCAAGAAGCTGAAGAGCGTGAAGGAGCTGCTGGG CATCACCATCATGGAGCGGAGCAGCTTCGAGAAGAACCCCATCGACTTCCTGGAG GCCAAGGGCTACAAGGAGGTGAAGAAGGACCTGATCATCAAGCTGCCCAAGTAC AGCCTGTTCGAGCTGGAGAACGGCCGGAAGCGGATGCTGGCCAGCGCCGGCGAG CTGCAGAAGGGCAACGAGCTGGCCCTGCCCAGCAAGTACGTGAACTTCCTGTACC TGGCCAGCCACTACGAGAAGCTGAAGGGCAGCCCCGAGGACAACGAGCAGAAGC AGCTGTTCGTGGAGCAGCACAAGCACTACCTGGACGAGATCATCGAGCAGATCAG CGAGTTCAGCAAGCGGGTGATCCTGGCCGACGCCAACCTGGACAAGGTGCTGAGC GCCTACAACAAGCACCGGGACAAGCCCATCCGGGAGCAGGCCGAGAACATCATC CACCTGTTCACCCTGACCAACCTGGGCGCCCCCGCCGCCTTCAAGTACTTCGACAC CACCATCGACCGGAAGCGGTACACCAGCACCAAGGAGGTGCTGGACGCCACCCTG ATCCACCAGAGCATCACCGGCCTGTACGAGACCCGGATCGACCTGAGCCAGCTGG GCGGCGACAGCGGCGGCAGCAAGCGGACCGCCGACGGCAGCGAGTTCGAGCCCA AGAAGAAGCGGAAGGTGTGATAATAGGCTGGAGCCTCGGTGGCCATGCTTCTTGC CCCTTGGGCCTCCCCCCAGCCCCTCCTCCCCTTCCTGCACCCGTACCCCCGTGGTCT TTGAATAAAGTCTGAGTGGGCGGCAAAAAAAAAAAAAAAAAAAAAAAAAAAAAA AGCATATGACTAAAAAAAAAAAAAAAAAAAAAAAAAAAAAAAAAAAAAAAAAA AAAAAAAAAAAAAAAAAAAAAAAAAAAAAA |
| 6 | ABE8e-Mut5 mRNA | GGGAGACCCAAGCTGGCTAGCGTTTAAACTTAAGCTTGGTACCGAGCTCGGATCC ACTAGTCCAGTGTGGTGGAATTCGGGAAATAAGAGAGAAAAGAAGAGTAAGAAG AAATATAAGAGCCACCATGAAGAGAACAGCTGATGGAAGTGAATTTGAGTCCCCC AAGAAGAAGCGGAAGGTGAGCGAGGTGGAGTTCAGCCACGAGTACTGGATGCGG CACGCCCTGACCCTGGCCAAGCGGGCCCGGGACGAGCGGGAGGTGCCCGTGGGC GCCGTGCTGGTGCTGAACAACCGGGTGATCGGCGAGGGCTGGAACCGGGCCATCG GCCTGCACGACCCCACCGCCCACGCCGAGATCATGGCCCTGCGGCAGGGCGGCCT GGTGATGCAGAACTACCGGCTGATCGACGCCACCCTGTACGTGACCTTCGAGCCC TGCGTGATGTGCGCCGGCGCCATGATCCACAGCCGGATCGGCCGGGTGGTGTTCG GCGTGCGGAACAGCAAGCGGGGCGCCGCCGGCAGCCTGATGAACGTGCTGAACT ACCCCGGCATGAACCACCGGGTGGAGATCACCGAGGGCATCCTGGCCGACGAGTG |
| | | CGCCGCCCTGCTGTGCGACTTCTACCGGATGCCCCGGCAGGTGTTCAACGCCCAGA AGAAGGCCCAGAGCAGCATCAACAGCGGCGGCAGCAGCGGCGGCAGCAGCGGCA GCGAGACCCCCGGCACCTCCGAGAGCGCCACCCCCGAGAGCTCCGGCGGCAGCAG CGGCGGCAGCGACAAGAAGTACAGCATCGGCCTGGCCATCGGCACCAACAGCGT GGGCTGGGCCGTGATCACCGACGAGTACAAGGTGCCCAGCAAGAAGTTCAAGGTG CTGGGCAACACCGACCGGCACAGCATCAAGAAGAACCTGATCGGCGCCCTGCTGT TCGACAGCGGCGAGACCGCCGAGGCCACCCGGCTGAAGCGGACCGCCCGGCGGC GGTACACCCGGCGGAAGAACCGGATCTGCTACCTGCAGGAGATCTTCAGCAACGA GATGGCCAAGGTGGACGACAGCTTCTTCCACCGGCTGGAGGAGAGCTTCCTGGTG GAGGAGGACAAGAAGCACGAGCGGCACCCCATCTTCGGCAACATCGTGGACGAG GTGGCCTACCACGAGAAGTACCCCACCATCTACCACCTGCGGAAGAAGCTGGTGG ACAGCACCGACAAGGCCGACCTGCGGCTGATCTACCTGGCCCTGGCCCACATGAT CAAGTTCCGGGGCCACTTCCTGATCGAGGGCGACCTGAACCCCGACAACAGCGAC GTGGACAAGCTGTTCATCCAGCTGGTGCAGACCTACAACCAGCTGTTCGAGGAGA ACCCCATCAACGCCAGCGGCGTGGACGCCAAGGCCATCCTGAGCGCCCGGCTGAG CAAGAGCCGGCGGCTGGAGAACCTGATCGCCCAGCTGCCCGGCGAGAAGAAGAA CGGCCTGTTCGGCAACCTGATCGCCCTGAGCCTGGGCCTGACCCCCAACTTCAAGA GCAACTTCGACCTGGCCGAGGACGCCAAGCTGCAGCTGAGCAAGGACACCTACGA CGACGACCTGGACAACCTGCTGGCCCAGATCGGCGACCAGTACGCCGACCTGTTC CTGGCCGCCAAGAACCTGAGCGACGCCATCCTGCTGAGCGACATCCTGCGGGTGA ACACCGAGATCACCAAGGCCCCCCTGAGCGCCAGCATGATCAAGCGGTACGACGA GCACCACCAGGACCTGACCCTGCTGAAGGCCCTGGTGCGGCAGCAGCTGCCCGAG AAGTACAAGGAGATCTTCTTCGACCAGAGCAAGAACGGCTACGCCGGCTACATCG ACGGCGGCGCCAGCCAGGAGGAGTTCTACAAGTTCATCAAGCCCATCCTGGAGAA GATGGACGGCACCGAGGAGCTGCTGGTGAAGCTGAACCGGGAGGACCTGCTGCG GAAGCAGCGGACCTTCGACAACGGCAGCATCCCCCACCAGATCCACCTGGGCGAG CTGCACGCCATCCTGCGGCGGCAGGAGGACTTCTACCCCTTCCTGAAGGACAACC GGGAGAAGATCGAGAAGATCCTGACCTTCCGGATCCCCTACTACGTGGGCCCCCT GGCCCGGGGCAACAGCCGGTTCGCCTGGATGACCCGGAAGAGCGAGGAGACCAT CACCCCCTGGAACTTCGAGGAGGTGGTGGACAAGGGCGCCAGCGCCCAGAGCTTC ATCGAGCGGATGACCAACTTCGACAAGAACCTGCCCAACGAGAAGGTGCTGCCCA AGCACAGCCTGCTGTACGAGTACTTCACCGTGTACAACGAGCTGACCGCCGTGAA GTACGTGACCGAGGGCATGCGGAAGCCCGCCTTCCTGAGCGGCGAGCAGAAGAA GGCCATCGTGGACCTGCTGTTCAAGACCAACCGGAAGGTGACCGTGAAGCAGCTG AAGGAGGACTACTTCAAGAAGATCGAGTGCTTCGACAGCGTGGAGATCAGCGGCG TGGAGGACCGGTTCAACGCCAGCCTGGGCACCTACCACGACCTGCTGAAGATCAT CAAGGACAAGGACTTCCTGGACAACGAGGAGAACGAGGACATCCTGGAGGACAT CGTGCTGACCCTGACCCTGTTCGAGGACCGGGAGATGATCGAGGAGCGGCTGAAG ACCTACGCCCACCTGTTCGACGACAAGGTGATGAAGCAGCTGAAGCGGCGGCGGT ACACCGGCTGGGGCCGGCTGAGCCGGAAGCTGATCAACGGCATCCGGGACAAGC AGAGCGGCAAGACCATCCTGGACTTCCTGAAGAGCGACGGCTTCGCCAACGCCAA CTTCATGGCCCTGATCGCCGACGACAGCCTGACCTTCAAGGAGGACATCCAGAAG GCCCAGGTGAGCGGCCAGGGCGACAGCCTGCACGAGCACATCGCCAACCTGGCCG GCAGCCCCGCCATCAAGAAGGGCATCCTGCAGACCGTGAAGGTGGTGGACGAGCT |
| | | GGTGAAGGTGATGGGCCGGCACAAGCCCGAGAACATCGTGATCGAGATGGCCCG GGAGAACCAGACCACCCAGAAGGGCCAGAAGAACAGCCGGGAGCGGATGAAGCG GATCGAGGAGGGCATCAAGGAGCTGGGCAGCCAGATCCTGAAGGAGCACCCCGT GGAGAACACCCAGCTGCAGAACGAGAAGCTGTACCTGTACTACCTGCAGAACGGC CGGGACATGTACGTGGACCAGGAGCTGGACATCAACCGGCTGAGCGACTACGACG TGGACCACATCGTGCCCCAGAGCTTCCTGAAGGACGACAGCATCGACAACAAGGT GCTGACCCGGAGCGACAAGAACCGGGGCAAGAGCGACAACGTGCCCAGCGAGGA GGTGGTGAAGAAGATGAAGAACTACTGGCGGCAGCTGCTGAACGCCAAGCTGATC ACCCAGCGGAAGTTCGACAACCTGACCAAGGCCGAGCGGGGCGGCCTGAGCGAG CTGGACAAGGCCGGCTTCATCAAGCGGCAGCTGGTGGAGACCCGGCAGATCACCA AGCACGTGGCCCAGATCCTGGACAGCCGGATGAACACCAAGTACGACGAGAACG ACAAGCTGATCCGGGAGGTGAAGGTGATCACCCTGAAGAGCAAGCTGGTGAGCG ACTTCCGGAAGGACTTCCAGTTCTACAAGGTGCGGGAGATCAACAACTACCACCA CGCCCACGACGCCTACCTGAACGCCGTGGTGGGCACCGCCCTGATCAAGAAGTAC CCCAAGCTGGAGAGCGAGTTCGTGTACGGCGACTACAAGGTGTACGACGTGCGGA AGATGATCGCCAAGAGCGAGCAGGAGATCGGCAAGGCCACCGCCAAGTACTTCTT CTACAGCAACATCATGAACTTCTTCAAGACCGAGATCACCCTGGCCAACGGCGAG ATCCGGAAGCGGCCCCTGATCGAGACCAACGGCGAGACCGGCGAGATCGTGTGG GACAAGGGCCGGGACTTCGCCACCGTGCGGAAGGTGCTGAGCATGCCCCAGGTGA ACATCGTGAAGAAGACCGAGGTGCAGACCGGCGGCTTCAGCAAGGAGAGCATCC TGCCCAAGCGGAACAGCGACAAGCTGATCGCCCGGAAGAAGGACTGGGACCCCA AGAAGTACGGCGGCTTCGACAGCCCCACCGTGGCCTACAGCGTGCTGGTGGTGGC CAAGGTGGAGAAGGGCAAGAGCAAGAAGCTGAAGAGCGTGAAGGAGCTGCTGGG CATCACCATCATGGAGCGGAGCAGCTTCGAGAAGAACCCCATCGACTTCCTGGAG GCCAAGGGCTACAAGGAGGTGAAGAAGGACCTGATCATCAAGCTGCCCAAGTAC AGCCTGTTCGAGCTGGAGAACGGCCGGAAGCGGATGCTGGCCAGCGCCGGCGAG CTGCAGAAGGGCAACGAGCTGGCCCTGCCCAGCAAGTACGTGAACTTCCTGTACC TGGCCAGCCACTACGAGAAGCTGAAGGGCAGCCCCGAGGACAACGAGCAGAAGC AGCTGTTCGTGGAGCAGCACAAGCACTACCTGGACGAGATCATCGAGCAGATCAG CGAGTTCAGCAAGCGGGTGATCCTGGCCGACGCCAACCTGGACAAGGTGCTGAGC GCCTACAACAAGCACCGGGACAAGCCCATCCGGGAGCAGGCCGAGAACATCATC CACCTGTTCACCCTGACCAACCTGGGCGCCCCCGCCGCCTTCAAGTACTTCGACAC CACCATCGACCGGAAGCGGTACACCAGCACCAAGGAGGTGCTGGACGCCACCCTG ATCCACCAGAGCATCACCGGCCTGTACGAGACCCGGATCGACCTGAGCCAGCTGG GCGGCGACAGCGGCGGCAGCAAGCGGACCGCCGACGGCAGCGAGTTCGAGCCCA AGAAGAAGCGGAAGGTGTGATAATAGGCTGGAGCCTCGGTGGCCATGCTTCTTGC CCCTTGGGCCTCCCCCCAGCCCCTCCTCCCCTTCCTGCACCCGTACCCCCGTGGTCT TTGAATAAAGTCTGAGTGGGCGGCAAAAAAAAAAAAAAAAAAAAAAAAAAAAAA AGCATATGACTAAAAAAAAAAAAAAAAAAAAAAAAAAAAAAAAAAAAAAAAAA AAAAAAAAAAAAAAAAAAAAAAAAAAAAAAA |
| 7 | CRISPRo ff-EE-W T mRNA | GGGAGACCCAAGCTGGCTAGCGTTTAAACTTAAGCTTGGTACCGAGCTCGGATCC ACTAGTCCAGTGTGGTGGAATTCGGGAAATAAGAGAGAAAAGAAGAGTAAGAAG AAATATAAGAGCCACCATGAATCATGATCAAGAATTCGATCCTCCAAAGGTTTAT CCTCCTGTTCCAGCTGAAAAGAGAAAGCCTATTAGAGTTCTTTCTTTGTTCGACGG |
| | | CATCGCCACCGGCCTGCTGGTGCTGAAGGACCTGGGCATCCAGGTGGACCGGTAC ATCGCCAGCGAGGTGTGCGAGGACAGCATCACCGTGGGCATGGTGCGGCACCAGG GCAAGATCATGTACGTGGGCGACGTGCGGAGCGTGACCCAGAAGCACATCCAGG AGTGGGGCCCCTTCGACCTGGTGATCGGCGGCAGCCCCTGCAACGACCTGAGCAT CGTGAACCCCGCCCGGAAGGGCCTGTACGAGGGCACCGGCCGGCTGTTCTTCGAG TTCTACCGGCTGCTGCACGACGCCCGGCCCAAGGAGGGCGACGACCGGCCCTTCT TCTGGCTGTTCGAGAACGTGGTGGCCATGGGCGTGAGCGACAAGCGGGACATCAG CCGCTTCCTGGAGAGCAACCCCGTGATGATCGACGCCAAGGAGGTGAGCGCCGCC CACCGGGCCCGCTACTTCTGGGGCAACCTGCCCGGCATGAACCGGCCCCTGGCCA GCACCGTGAACGACAAGCTGGAGCTGCAGGAGTGCCTGGAGCACGGCCGGATCG CCAAGTTCTCCAAGGTGCGGACCATCACCACCCGGAGCAACAGCATCAAGCAGGG CAAGGACCAGCACTTCCCCGTGTTCATGAACGAGAAGGAGGACATCCTGTGGTGC ACCGAGATGGAGCGGGTGTTCGGCTTCCCCGTGCACTACACCGACGTGAGCAACA TGAGCCGGCTGGCCCGGCAGCGGCTGCTGGGCCGGAGCTGGAGCGTGCCCGTGAT CCGGCACCTGTTCGCCCCCCTGAAGGAGTACTTCGCCTGCGTGAGCAGCGGCAAC AGCAACGCCAACAGCCGGGGCCCCAGCTTCAGCAGCGGCCTGGTGCCCCTGAGCC TGCGGGGCAGCCACATGGGCCCCATGGAGATCTACAAGACCGTGAGCGCCTGGAA GCGGCAGCCCGTGCGGGTGCTGAGCCTGTTCCGGAACATCGACAAGGTGCTGAAG AGCCTGGGCTTCCTGGAGAGCGGCAGCGGCAGCGGCGGCGGCACCCTGAAGTACG TGGAGGACGTGACCAACGTGGTGCGGCGGGACGTGGAGAAGTGGGGCCCCTTCG ACCTGGTGTACGGCAGCACCCAGCCCCTGGGCAGCAGCTGCGACCGGTGCCCCGG CTGGTACATGTTCCAGTTCCACCGGATCCTGCAGTACGCCCTGCCCCGGCAGGAGA GCCAGCGGCCCTTCTTCTGGATCTTCATGGACAACCTGCTGCTGACCGAGGACGAC CAGGAGACCACCACCCGGTTCCTGCAGACCGAGGCCGTGACCCTGCAGGACGTGC GGGGCCGGGACTACCAGAACGCCATGCGGGTGTGGAGCAACATCCCCGGCCTGAA GAGCAAGCACGCCCCCCTGACCCCCAAGGAGGAGGAGTACCTGCAGGCCCAGGT GCGGAGCCGGAGCAAGCTGGACGCCCCCAAGGTGGACCTGCTGGTGAAGAACTG CCTGCTGCCCCTGCGGGAGTACTTCAAGTACTTCAGCCAGAACAGCCTGCCCCTGG GCGGCCCCAGCAGCGGCGCCCCCCCCCCCAGCGGCGGCAGCCCCGCCGGCAGCCC CACCAGCACCGAGGAGGGCACCAGCGAGAGCGCCACCCCCGAGAGCGGCCCCGG CACCAGCACCGAGCCCAGCGAGGGCAGCGCCCCCGGCAGCCCCGCCGGCAGCCC CACCAGCACCGAGGAGGGCACCAGCACCGAGCCCAGCGAGGGCAGCGCCCCCGG CACCAGCACCGAGCCCAGCGAGATGGACAAGAAGTACAGCATCGGCCTGGCCATC GGCACCAACAGCGTGGGCTGGGCCGTGATCACCGACGAGTACAAGGTGCCCAGCA AGAAGTTCAAGGTGCTGGGCAACACCGACCGGCACAGCATCAAGAAGAACCTGA TCGGCGCCCTGCTGTTCGACAGCGGCGAGACCGCCGAGGCCACCCGGCTGAAGCG GACCGCCCGGCGGCGGTACACCCGGCGGAAGAACCGGATCTGCTACCTGCAGGAG ATCTTCAGCAACGAGATGGCCAAGGTGGACGACAGCTTCTTCCACCGGCTGGAGG AGAGCTTCCTGGTGGAGGAGGACAAGAAGCACGAGCGGCACCCCATCTTCGGCAA CATCGTGGACGAGGTGGCCTACCACGAGAAGTACCCCACCATCTACCACCTGCGG AAGAAGCTGGTGGACAGCACCGACAAGGCCGACCTGCGGCTGATCTACCTGGCCC TGGCCCACATGATCAAGTTCCGGGGCCACTTCCTGATCGAGGGCGACCTGAACCC CGACAACAGCGACGTGGACAAGCTGTTCATCCAGCTGGTGCAGACCTACAACCAG CTGTTCGAGGAGAACCCCATCAACGCCAGCGGCGTGGACGCCAAGGCCATCCTGA |
| | | GCGCCCGGCTGAGCAAGAGCCGGCGGCTGGAGAACCTGATCGCCCAGCTGCCCGG CGAGAAGAAGAACGGCCTGTTCGGCAACCTGATCGCCCTGAGCCTGGGCCTGACC CCCAACTTCAAGAGCAACTTCGACCTGGCCGAGGACGCCAAGCTGCAGCTGAGCA AGGACACCTACGACGACGACCTGGACAACCTGCTGGCCCAGATCGGCGACCAGTA CGCCGACCTGTTCCTGGCCGCCAAGAACCTGAGCGACGCCATCCTGCTGAGCGAC ATCCTGCGGGTGAACACCGAGATCACCAAGGCCCCCCTGAGCGCCAGCATGATCA AGCGGTACGACGAGCACCACCAGGACCTGACCCTGCTGAAGGCCCTGGTGCGGCA GCAGCTGCCCGAGAAGTACAAGGAGATCTTCTTCGACCAGAGCAAGAACGGCTAC GCCGGCTACATCGACGGCGGCGCCAGCCAGGAGGAGTTCTACAAGTTCATCAAGC CCATCCTGGAGAAGATGGACGGCACCGAGGAGCTGCTGGTGAAGCTGAACCGGG AGGACCTGCTGCGGAAGCAGCGGACCTTCGACAACGGCAGCATCCCCCACCAGAT CCACCTGGGCGAGCTGCACGCCATCCTGCGGCGGCAGGAGGACTTCTACCCCTTC CTGAAGGACAACCGGGAGAAGATCGAGAAGATCCTGACCTTCCGGATCCCCTACT ACGTGGGCCCCCTGGCCCGGGGCAACAGCCGGTTCGCCTGGATGACCCGGAAGAG CGAGGAGACCATCACCCCCTGGAACTTCGAGGAGGTGGTGGACAAGGGCGCCAG CGCCCAGAGCTTCATCGAGCGGATGACCAACTTCGACAAGAACCTGCCCAACGAG AAGGTGCTGCCCAAGCACAGCCTGCTGTACGAGTACTTCACCGTGTACAACGAGC TGACCAAGGTGAAGTACGTGACCGAGGGCATGCGGAAGCCCGCCTTCCTGAGCGG CGAGCAGAAGAAGGCCATCGTGGACCTGCTGTTCAAGACCAACCGGAAGGTGACC GTGAAGCAGCTGAAGGAGGACTACTTCAAGAAGATCGAGTGCTTCGACAGCGTGG AGATCAGCGGCGTGGAGGACCGGTTCAACGCCAGCCTGGGCACCTACCACGACCT GCTGAAGATCATCAAGGACAAGGACTTCCTGGACAACGAGGAGAACGAGGACAT CCTGGAGGACATCGTGCTGACCCTGACCCTGTTCGAGGACCGGGAGATGATCGAG GAGCGGCTGAAGACCTACGCCCACCTGTTCGACGACAAGGTGATGAAGCAGCTGA AGCGGCGGCGGTACACCGGCTGGGGCCGGCTGAGCCGGAAGCTGATCAACGGCA TCCGGGACAAGCAGAGCGGCAAGACCATCCTGGACTTCCTGAAGAGCGACGGCTT CGCCAACCGGAACTTCATGCAGCTGATCCACGACGACAGCCTGACCTTCAAGGAG GACATCCAGAAGGCCCAGGTGAGCGGCCAGGGCGACAGCCTGCACGAGCACATC GCCAACCTGGCCGGCAGCCCCGCCATCAAGAAGGGCATCCTGCAGACCGTGAAGG TGGTGGACGAGCTGGTGAAGGTGATGGGCCGGCACAAGCCCGAGAACATCGTGAT CGAGATGGCCCGGGAGAACCAGACCACCCAGAAGGGCCAGAAGAACAGCCGGGA GCGGATGAAGCGGATCGAGGAGGGCATCAAGGAGCTGGGCAGCCAGATCCTGAA GGAGCACCCCGTGGAGAACACCCAGCTGCAGAACGAGAAGCTGTACCTGTACTAC CTGCAGAACGGCCGGGACATGTACGTGGACCAGGAGCTGGACATCAACCGGCTGA GCGACTACGACGTGGACGCCATCGTGCCCCAGAGCTTCCTGAAGGACGACAGCAT CGACAACAAGGTGCTGACCCGGAGCGACAAGAACCGGGGCAAGAGCGACAACGT GCCCAGCGAGGAGGTGGTGAAGAAGATGAAGAACTACTGGCGGCAGCTGCTGAA CGCCAAGCTGATCACCCAGCGGAAGTTCGACAACCTGACCAAGGCCGAGCGGGGC GGCCTGAGCGAGCTGGACAAGGCCGGCTTCATCAAGCGGCAGCTGGTGGAGACCC GGCAGATCACCAAGCACGTGGCCCAGATCCTGGACAGCCGGATGAACACCAAGTA CGACGAGAACGACAAGCTGATCCGGGAGGTGAAGGTGATCACCCTGAAGAGCAA GCTGGTGAGCGACTTCCGGAAGGACTTCCAGTTCTACAAGGTGCGGGAGATCAAC AACTACCACCACGCCCACGACGCCTACCTGAACGCCGTGGTGGGCACCGCCCTGA TCAAGAAGTACCCCAAGCTGGAGAGCGAGTTCGTGTACGGCGACTACAAGGTGTA |
| | | CGACGTGCGGAAGATGATCGCCAAGAGCGAGCAGGAGATCGGCAAGGCCACCGC CAAGTACTTCTTCTACAGCAACATCATGAACTTCTTCAAGACCGAGATCACCCTGG CCAACGGCGAGATCCGGAAGCGGCCCCTGATCGAGACCAACGGCGAGACCGGCG AGATCGTGTGGGACAAGGGCCGGGACTTCGCCACCGTGCGGAAGGTGCTGAGCAT GCCCCAGGTGAACATCGTGAAGAAGACCGAGGTGCAGACCGGCGGCTTCAGCAA GGAGAGCATCCTGCCCAAGCGGAACAGCGACAAGCTGATCGCCCGGAAGAAGGA CTGGGACCCCAAGAAGTACGGCGGCTTCGACAGCCCCACCGTGGCCTACAGCGTG CTGGTGGTGGCCAAGGTGGAGAAGGGCAAGAGCAAGAAGCTGAAGAGCGTGAAG GAGCTGCTGGGCATCACCATCATGGAGCGGAGCAGCTTCGAGAAGAACCCCATCG ACTTCCTGGAGGCCAAGGGCTACAAGGAGGTGAAGAAGGACCTGATCATCAAGCT GCCCAAGTACAGCCTGTTCGAGCTGGAGAACGGCCGGAAGCGGATGCTGGCCAGC GCCGGCGAGCTGCAGAAGGGCAACGAGCTGGCCCTGCCCAGCAAGTACGTGAACT TCCTGTACCTGGCCAGCCACTACGAGAAGCTGAAGGGCAGCCCCGAGGACAACGA GCAGAAGCAGCTGTTCGTGGAGCAGCACAAGCACTACCTGGACGAGATCATCGAG CAGATCAGCGAGTTCAGCAAGCGGGTGATCCTGGCCGACGCCAACCTGGACAAGG TGCTGAGCGCCTACAACAAGCACCGGGACAAGCCCATCCGGGAGCAGGCCGAGA ACATCATCCACCTGTTCACCCTGACCAACCTGGGCGCCCCCGCCGCCTTCAAGTAC TTCGACACCACCATCGACCGGAAGCGGTACACCAGCACCAAGGAGGTGCTGGACG CCACCCTGATCCACCAGAGCATCACCGGCCTGTACGAGACCCGGATCGACCTGAG CCAGCTGGGCGGCGACCCCAAGAAGAAGCGGAAGGTGGAGGACCCCAAGAAGAA GCGGAAGGTGGGCGGCGGCGGCATGGACGCCAAGAGCCTGACCGCCTGGAG CCGGACCCTGGTGACCTTCAAGGACGTGTTCGTGGACTTCACCCGGGAGGAGTGG AAGCTGCTGGACACCGCCCAGCAGATCGTGTACCGGAACGTGATGCTGGAGAACT ACAAGAACCTGGTGAGCCTGGGCTACCAGCTGACCAAGCCCGACGTGATCCTGCG GCTGGAGAAGGGCGAGGAGCCCTGATAATAGGCTGGAGCCTCGGTGGCCATGCTT CTTGCCCCTTGGGCCTCCCCCCAGCCCCTCCTCCCCTTCCTGCACCCGTACCCCCGT GGTCTTTGAATAAAGTCTGAGTGGGCGGCAAAAAAAAAAAAAAAAAAAAAAAAA AAAAAAGCATATGACTAAAAAAAAAAAAAAAAAAAAAAAAAAAAAAAAAAAAAA AAAAAAAAAAAAAAAAAAAAAAAAAAAAAAAA |
| 8 | CRISPRo ff-EE-mut5 mRNA | GGGAGACCCAAGCTGGCTAGCGTTTAAACTTAAGCTTGGTACCGAGCTCGGATCC ACTAGTCCAGTGTGGTGGAATTCGGGAAATAAGAGAGAAAAGAAGAGTAAGAAG AAATATAAGAGCCACCATGAATCATGATCAAGAATTCGATCCTCCAAAGGTTTAT CCTCCTGTTCCAGCTGAAAAGAGAAAGCCTATTAGAGTTCTTTCTTTGTTCGACGG CATCGCCACCGGCCTGCTGGTGCTGAAGGACCTGGGCATCCAGGTGGACCGGTAC ATCGCCAGCGAGGTGTGCGAGGACAGCATCACCGTGGGCATGGTGCGGCACCAGG GCAAGATCATGTACGTGGGCGACGTGCGGAGCGTGACCCAGAAGCACATCCAGG AGTGGGGCCCCTTCGACCTGGTGATCGGCGGCAGCCCCTGCAACGACCTGAGCAT CGTGAACCCCGCCCGGAAGGGCCTGTACGAGGGCACCGGCCGGCTGTTCTTCGAG TTCTACCGGCTGCTGCACGACGCCCGGCCCAAGGAGGGCGACGACCGGCCCTTCT TCTGGCTGTTCGAGAACGTGGTGGCCATGGGCGTGAGCGACAAGCGGGACATCAG CCGCTTCCTGGAGAGCAACCCCGTGATGATCGACGCCAAGGAGGTGAGCGCCGCC CACCGGGCCCGCTACTTCTGGGGCAACCTGCCCGGCATGAACCGGCCCCTGGCCA GCACCGTGAACGACAAGCTGGAGCTGCAGGAGTGCCTGGAGCACGGCCGGATCG CCAAGTTCTCCAAGGTGCGGACCATCACCACCCGGAGCAACAGCATCAAGCAGGG |
| | | CAAGGACCAGCACTTCCCCGTGTTCATGAACGAGAAGGAGGACATCCTGTGGTGC ACCGAGATGGAGCGGGTGTTCGGCTTCCCCGTGCACTACACCGACGTGAGCAACA TGAGCCGGCTGGCCCGGCAGCGGCTGCTGGGCCGGAGCTGGAGCGTGCCCGTGAT CCGGCACCTGTTCGCCCCCCTGAAGGAGTACTTCGCCTGCGTGAGCAGCGGCAAC AGCAACGCCAACAGCCGGGGCCCCAGCTTCAGCAGCGGCCTGGTGCCCCTGAGCC TGCGGGGCAGCCACATGGGCCCCATGGAGATCTACAAGACCGTGAGCGCCTGGAA GCGGCAGCCCGTGCGGGTGCTGAGCCTGTTCCGGAACATCGACAAGGTGCTGAAG AGCCTGGGCTTCCTGGAGAGCGGCAGCGGCAGCGGCGGCGGCACCCTGAAGTACG TGGAGGACGTGACCAACGTGGTGCGGCGGGACGTGGAGAAGTGGGGCCCCTTCG ACCTGGTGTACGGCAGCACCCAGCCCCTGGGCAGCAGCTGCGACCGGTGCCCCGG CTGGTACATGTTCCAGTTCCACCGGATCCTGCAGTACGCCCTGCCCCGGCAGGAGA GCCAGCGGCCCTTCTTCTGGATCTTCATGGACAACCTGCTGCTGACCGAGGACGAC CAGGAGACCACCACCCGGTTCCTGCAGACCGAGGCCGTGACCCTGCAGGACGTGC GGGGCCGGGACTACCAGAACGCCATGCGGGTGTGGAGCAACATCCCCGGCCTGAA GAGCAAGCACGCCCCCCTGACCCCCAAGGAGGAGGAGTACCTGCAGGCCCAGGT GCGGAGCCGGAGCAAGCTGGACGCCCCCAAGGTGGACCTGCTGGTGAAGAACTG CCTGCTGCCCCTGCGGGAGTACTTCAAGTACTTCAGCCAGAACAGCCTGCCCCTGG GCGGCCCCAGCAGCGGCGCCCCCCCCCCCAGCGGCGGCAGCCCCGCCGGCAGCCC CACCAGCACCGAGGAGGGCACCAGCGAGAGCGCCACCCCCGAGAGCGGCCCCGG CACCAGCACCGAGCCCAGCGAGGGCAGCGCCCCCGGCAGCCCCGCCGGCAGCCC CACCAGCACCGAGGAGGGCACCAGCACCGAGCCCAGCGAGGGCAGCGCCCCCGG CACCAGCACCGAGCCCAGCGAGATGGACAAGAAGTACAGCATCGGCCTGGCCATC GGCACCAACAGCGTGGGCTGGGCCGTGATCACCGACGAGTACAAGGTGCCCAGCA AGAAGTTCAAGGTGCTGGGCAACACCGACCGGCACAGCATCAAGAAGAACCTGA TCGGCGCCCTGCTGTTCGACAGCGGCGAGACCGCCGAGGCCACCCGGCTGAAGCG GACCGCCCGGCGGCGGTACACCCGGCGGAAGAACCGGATCTGCTACCTGCAGGAG ATCTTCAGCAACGAGATGGCCAAGGTGGACGACAGCTTCTTCCACCGGCTGGAGG AGAGCTTCCTGGTGGAGGAGGACAAGAAGCACGAGCGGCACCCCATCTTCGGCAA CATCGTGGACGAGGTGGCCTACCACGAGAAGTACCCCACCATCTACCACCTGCGG AAGAAGCTGGTGGACAGCACCGACAAGGCCGACCTGCGGCTGATCTACCTGGCCC TGGCCCACATGATCAAGTTCCGGGGCCACTTCCTGATCGAGGGCGACCTGAACCC CGACAACAGCGACGTGGACAAGCTGTTCATCCAGCTGGTGCAGACCTACAACCAG CTGTTCGAGGAGAACCCCATCAACGCCAGCGGCGTGGACGCCAAGGCCATCCTGA GCGCCCGGCTGAGCAAGAGCCGGCGGCTGGAGAACCTGATCGCCCAGCTGCCCGG CGAGAAGAAGAACGGCCTGTTCGGCAACCTGATCGCCCTGAGCCTGGGCCTGACC CCCAACTTCAAGAGCAACTTCGACCTGGCCGAGGACGCCAAGCTGCAGCTGAGCA AGGACACCTACGACGACGACCTGGACAACCTGCTGGCCCAGATCGGCGACCAGTA CGCCGACCTGTTCCTGGCCGCCAAGAACCTGAGCGACGCCATCCTGCTGAGCGAC ATCCTGCGGGTGAACACCGAGATCACCAAGGCCCCCCTGAGCGCCAGCATGATCA AGCGGTACGACGAGCACCACCAGGACCTGACCCTGCTGAAGGCCCTGGTGCGGCA GCAGCTGCCCGAGAAGTACAAGGAGATCTTCTTCGACCAGAGCAAGAACGGCTAC GCCGGCTACATCGACGGCGGCGCCAGCCAGGAGGAGTTCTACAAGTTCATCAAGC CCATCCTGGAGAAGATGGACGGCACCGAGGAGCTGCTGGTGAAGCTGAACCGGG AGGACCTGCTGCGGAAGCAGCGGACCTTCGACAACGGCAGCATCCCCCACCAGAT |
| | | CCACCTGGGCGAGCTGCACGCCATCCTGCGGCGGCAGGAGGACTTCTACCCCTTC CTGAAGGACAACCGGGAGAAGATCGAGAAGATCCTGACCTTCCGGATCCCCTACT ACGTGGGCCCCCTGGCCCGGGGCAACAGCCGGTTCGCCTGGATGACCCGGAAGAG CGAGGAGACCATCACCCCCTGGAACTTCGAGGAGGTGGTGGACAAGGGCGCCAG CGCCCAGAGCTTCATCGAGCGGATGACCAACTTCGACAAGAACCTGCCCAACGAG AAGGTGCTGCCCAAGCACAGCCTGCTGTACGAGTACTTCACCGTGTACAACGAGC TGACCGCCGTGAAGTACGTGACCGAGGGCATGCGGAAGCCCGCCTTCCTGAGCGG CGAGCAGAAGAAGGCCATCGTGGACCTGCTGTTCAAGACCAACCGGAAGGTGACC GTGAAGCAGCTGAAGGAGGACTACTTCAAGAAGATCGAGTGCTTCGACAGCGTGG AGATCAGCGGCGTGGAGGACCGGTTCAACGCCAGCCTGGGCACCTACCACGACCT GCTGAAGATCATCAAGGACAAGGACTTCCTGGACAACGAGGAGAACGAGGACAT CCTGGAGGACATCGTGCTGACCCTGACCCTGTTCGAGGACCGGGAGATGATCGAG GAGCGGCTGAAGACCTACGCCCACCTGTTCGACGACAAGGTGATGAAGCAGCTGA AGCGGCGGCGGTACACCGGCTGGGGCCGGCTGAGCCGGAAGCTGATCAACGGCA TCCGGGACAAGCAGAGCGGCAAGACCATCCTGGACTTCCTGAAGAGCGACGGCTT CGCCAACGCCAACTTCATGGCCCTGATCCGCGACGACAGCCTGACCTTCAAGGAG GACATCCAGAAGGCCCAGGTGAGCGGCCAGGGCGACAGCCTGCACGAGCACATC GCCAACCTGGCCGGCAGCCCCGCCATCAAGAAGGGCATCCTGCAGACCGTGAAGG TGGTGGACGAGCTGGTGAAGGTGATGGGCCGGCACAAGCCCGAGAACATCGTGAT CGAGATGGCCCGGGAGAACCAGACCACCCAGAAGGGCCAGAAGAACAGCCGGGA GCGGATGAAGCGGATCGAGGAGGGCATCAAGGAGCTGGGCAGCCAGATCCTGAA GGAGCACCCCGTGGAGAACACCCAGCTGCAGAACGAGAAGCTGTACCTGTACTAC CTGCAGAACGGCCGGGACATGTACGTGGACCAGGAGCTGGACATCAACCGGCTGA GCGACTACGACGTGGACGCCATCGTGCCCCAGAGCTTCCTGAAGGACGACAGCAT CGACAACAAGGTGCTGACCCGGAGCGACAAGAACCGGGGCAAGAGCGACAACGT GCCCAGCGAGGAGGTGGTGAAGAAGATGAAGAACTACTGGCGGCAGCTGCTGAA CGCCAAGCTGATCACCCAGCGGAAGTTCGACAACCTGACCAAGGCCGAGCGGGGC GGCCTGAGCGAGCTGGACAAGGCCGGCTTCATCAAGCGGCAGCTGGTGGAGACCC GGCAGATCACCAAGCACGTGGCCCAGATCCTGGACAGCCGGATGAACACCAAGTA CGACGAGAACGACAAGCTGATCCGGGAGGTGAAGGTGATCACCCTGAAGAGCAA GCTGGTGAGCGACTTCCGGAAGGACTTCCAGTTCTACAAGGTGCGGGAGATCAAC AACTACCACCACGCCCACGACGCCTACCTGAACGCCGTGGTGGGCACCGCCCTGA TCAAGAAGTACCCCAAGCTGGAGAGCGAGTTCGTGTACGGCGACTACAAGGTGTA CGACGTGCGGAAGATGATCGCCAAGAGCGAGCAGGAGATCGGCAAGGCCACCGC CAAGTACTTCTTCTACAGCAACATCATGAACTTCTTCAAGACCGAGATCACCCTGG CCAACGGCGAGATCCGGAAGCGGCCCCTGATCGAGACCAACGGCGAGACCGGCG AGATCGTGTGGGACAAGGGCCGGGACTTCGCCACCGTGCGGAAGGTGCTGAGCAT GCCCCAGGTGAACATCGTGAAGAAGACCGAGGTGCAGACCGGCGGCTTCAGCAA GGAGAGCATCCTGCCCAAGCGGAACAGCGACAAGCTGATCGCCCGGAAGAAGGA CTGGGACCCCAAGAAGTACGGCGGCTTCGACAGCCCCACCGTGGCCTACAGCGTG CTGGTGGTGGCCAAGGTGGAGAAGGGCAAGAGCAAGAAGCTGAAGAGCGTGAAG GAGCTGCTGGGCATCACCATCATGGAGCGGAGCAGCTTCGAGAAGAACCCCATCG ACTTCCTGGAGGCCAAGGGCTACAAGGAGGTGAAGAAGGACCTGATCATCAAGCT GCCCAAGTACAGCCTGTTCGAGCTGGAGAACGGCCGGAAGCGGATGCTGGCCAGC |
| | | GCCGGCGAGCTGCAGAAGGGCAACGAGCTGGCCCTGCCCAGCAAGTACGTGAACT TCCTGTACCTGGCCAGCCACTACGAGAAGCTGAAGGGCAGCCCCGAGGACAACGA GCAGAAGCAGCTGTTCGTGGAGCAGCACAAGCACTACCTGGACGAGATCATCGAG CAGATCAGCGAGTTCAGCAAGCGGGTGATCCTGGCCGACGCCAACCTGGACAAGG TGCTGAGCGCCTACAACAAGCACCGGGACAAGCCCATCCGGGAGCAGGCCGAGA ACATCATCCACCTGTTCACCCTGACCAACCTGGGCGCCCCCGCCGCCTTCAAGTAC TTCGACACCACCATCGACCGGAAGCGGTACACCAGCACCAAGGAGGTGCTGGACG CCACCCTGATCCACCAGAGCATCACCGGCCTGTACGAGACCCGGATCGACCTGAG CCAGCTGGGCGGCGACCCCAAGAAGAAGCGGAAGGTGGAGGACCCCAAGAAGAA GCGGAAGGTGGGCGGCGGCGGCATGGACGCCAAGAGCCTGACCGCCTGGAG CCGGACCCTGGTGACCTTCAAGGACGTGTTCGTGGACTTCACCCGGGAGGAGTGG AAGCTGCTGGACACCGCCCAGCAGATCGTGTACCGGAACGTGATGCTGGAGAACT ACAAGAACCTGGTGAGCCTGGGCTACCAGCTGACCAAGCCCGACGTGATCCTGCG GCTGGAGAAGGGCGAGGAGCCCTGATAATAGGCTGGAGCCTCGGTGGCCATGCTT CTTGCCCCTTGGGCCTCCCCCCAGCCCCTCCTCCCCTTCCTGCACCCGTACCCCCGT GGTCTTTGAATAAAGTCTGAGTGGGCGGCAAAAAAAAAAAAAAAAAAAAAAAAA AAAAAAGCATATGACTAAAAAAAAAAAAAAAAAAAAAAAAAAAAAAAAAAAAAA AAAAAAAAAAAAAAAAAAAAAAAAAAAAAAAAAA |
| 9 | KOZAK | gccaccatgg |
| 10 | pVAX1 vector, 5'UTR+T EV 5'UTR | GGGAGACCCAAGCTGGCTAGCGTTTAAACTTAAGCTTGGTACCGAGCTCGGATCC ACTAGTCCAGTGTGGTGGAATTCGGGAAATAAGAGAGAAAAGAAGAGTAAGAAG AAATATAAGAGCCACC |
| 11 | TEV 5'UTR | GGGAAATAAGAGAGAAAAGAAGAGTAAGAAGAAATATAAGAGCCACC |
| 12 | polyA | aaaaaaaaaaaaaaaaaaaaaaaaaaaaaagcatatgactaaaaaaaaaaaaaaaaaaaaaaaaaaaaaaaaaaaaaaaaaaaaa aaaaaaaaaaaaaaaaaaaaaaaa |
| 13 | hHBA1 3'UTR | TGATAATAGGCTGGAGCCTCGGTGGCCATGCTTCTTGCCCCTTGGGCCTCCCCCCA GCCCCTCCTCCCCTTCCTGCACCCGTACCCCCGTGGTCTTTGAATAAAGTCTGAGT GGGCGGC |

| **gRNA sequence or DNA sequence encoding the gRNA sequence; wherein the spacer sequence of the gRNA is the same as the sequence of its editing window** | | |
|---|---|---|
| 14 | sgTTR-0 | AAAGGCTGCTGATGACACCTGTTTTAGAGCTAGAAATAGCAAGTTAAAATAAGGC TAGTCCGTTATCAACTTGAAAAAGTGGCACCGAGTCGGTGCTTTT |
| 15 | sgTTR-1 | ATACCAGTCCAGCAAGGCAGGTTTTAGAGCTAGAAATAGCAAGTTAAAATAAGGC TAGTCCGTTATCAACTTGAAAAAGTGGCACCGAGTCGGTGCTTTT |
| 16 | sgTTR-2 | AGTGAGTCTGGAGAGCTGCAGTTTTAGAGCTAGAAATAGCAAGTTAAAATAAGGC TAGTCCGTTATCAACTTGAAAAAGTGGCACCGAGTCGGTGCTTTT |
| 17 | sgTTR-3 | TCACAGAAACACTCACCGTAGTTTTAGAGCTAGAAATAGCAAGTTAAAATAAGGC TAGTCCGTTATCAACTTGAAAAAGTGGCACCGAGTCGGTGCTTTT |
| 18 | sgTTR-7 | TTTGACCATCAGAGGACACTGTTTTAGAGCTAGAAATAGCAAGTTAAAATAAGGC TAGTCCGTTATCAACTTGAAAAAGTGGCACCGAGTCGGTGCTTTT |
| 19 | sgTTR-0- | AAAGGCTGCTGATGACACCTGTTTTAGAGCTAGAAATAGCAAGTTAAAATAAGGC |
| | v02 | TAGTCCGTTATGAACTTGAAAAAGTGGCACCGAGTCGGTGCTTTT |
| 20 | sgTTR-0-v04 | AAAGGCTGCTGATGACACCTGTTTTAGAGCTAGAAATAGCAAGTTAAAATAAGGC TAGTCCGTTTACAACTTGAAAAAGTGGCACCGAGTCGGTGCTTTT |
| 21 | sgNA-TT R-0-del (82 nt) | AAAGGCTGCTGATGACACCTGTTTTAGAGCTAGAAATAGCAAGTTAAAATAAGGC TAGTCCGTTATCAACTTGAAAAAGTGG |
| 22 | sgTTR0-c rRNA | AAAGGCTGCTGATGACACCTGTTTTAGAGCTATGCT |
| 23 | sgTTR0-t racrRNA | AGCATAGCAAGTTAAAATAAGGCTAGTCCGTTATCAACTTGAAAAAGTGGCACCG AGTCGGTGCTTTT |
| 24 | sgTTR-0 spacer | AAAGGCTGCTGATGACACCT |
| 25 | sgTTR-1 spacer | ATACCAGTCCAGCAAGGCAG |
| 26 | sgTTR-2 spacer | AGTGAGTCTGGAGAGCTGCA |
| 27 | sgTTR-3 spacer | TCACAGAAACACTCACCGTA |
| 28 | sgTTR-7 spacer | TTTGACCATCAGAGGACACT |
| 29 | sgRNA-1 spacer | AGAGTCTCTCAGCTGGTACA |
| 30 | sgRNA-2 spacer | TCAGGGTTCTGGATATCTGT |
| 31 | sgRNA-3 spacer | CTGGATATCTGTGGGACAAG |
| 32 | sgRNA-4 spacer | ACGACGCGTGGGTGGCAAGC |
| 33 | EMX1 spacer | GAGTCCGAGCAGAAGAAGAA |
| 34 | VEGFA3 spacer | GGTGAGTGAGTGTGTGCGTG |
| 35 | HEK293 _ 2 spacer | GAACACAAAGCATAGACTGC |
| 36 | ATGg2 spacer | GCCATCCTGCCAAGAATGAG |
| 37 | PCSK9 spacer | cccgcacCTTGGCGCAGCGG |
| 38 | F-site2 spacer | GAGTATGAGGCATAGACTGC |
| 39 | TTR-AB E-1 spacer | TATAGGAAAACCAGTGAGTC |
| 40 | HEK site 1 spacer | GGGAAAGACCCAGCATCCGT |
| 41 | HEK site2 spacer | GAACACAAAGCATAGACTGC |
| 42 | HEK site3 spacer | GGCCCAGACTGAGCACGTGA |
| 43 | HEK site4 spacer | GGCACTGCGGCTGGAGGTGG |
| 44 | EE-PCSK 9 spacer | GCGGAAACCTTCTAGGGTGT |
| 45 | EE-sgHB V-1 spacer | AGGAGTTCCGCAGTATGGAT |
| 46 | EE-sgHB V-2 spacer | CAGATGAGAAGGCACAGACG |

| sequences of gRNA high-frequency off-target sites | | |
|---|---|---|
| 47 | EMX1 OT1 | GAGTCTAAGCAGAAGAAGAA |
| 48 | EMX1 OT2 | GAGGCCGAGCAGAAGAAAGA |
| 49 | VEGFA3 OT1 | GTGTGAGTAAGTGTGTGTGTG |
| 50 | VEGFA3 OT2 | GGTGAGTGTGTGTGTGCATG |
| 51 | TTR-AB E-1 OT1 | TAGAGGAAAACCAGTCAGTC |
| 52 | TTR-AB E-1 OT2 | CATAGGAAAACCAGTGAGTT |
| 53 | TTR-AB E-1 OT3 | TAAAGGAAAACCAGTGGGTC |
| 54 | HEK site1 OT1 | GGGAAAGTCCCAGCATCCTT |
| 55 | HEK site1 OT2 | GGGAAAAGCCCAGCATCCCT |
| 56 | HEK site1 OT3 | GGGAAGGACCCAGCATCCTG |
| 57 | HEK site2 OT1 | GAACACAATGCATAGATTGC |
| 58 | HEK site2 OT2 | GAAACAAAAGCATAGACTGC |
| 59 | HEK site2 OT3 | GAACACAAGGCAGAGACTGT |
| 60 | HEK site3 OT1 | CACCCAGACTGAGCACGTGC |
| 61 | HEK site3 OT2 | GACACAGACTGGGCACGTGA |
| 62 | HEK site4 OT1 | TGCACTGCGGCCGGAGGAGG |
| 63 | HEK site4 OT2 | GGCATCACGGCTGGAGGTGG |
| 64 | HEK site4 OT3 | GGCGCTGCGGCGGGAGGTGG |

| Amino acid sequence | | |
|---|---|---|
| 65 | SpCas9-WT | MAPKKKRKVGIHGVPAADKKYSIGLDIGTNSVGWAVITDEYKVPSKKFKVLGNTDRH SIKKNLIGALLFDSGETAEATRLKRTARRRYTRRKNRICYLQEIFSNEMAKVDDSFFHR LEESFLVEEDKKHERHPIFGNIVDEVAYHEKYPTIYHLRKKLVDSTDKADLRLIYLALA HMIKFRGHFLIEGDLNPDNSDVDKLFIQLVQTYNQLFEENPINASGVDAKAILSARLSK SRRLENLIAQLPGEKKNGLFGNLIALSLGLTPNFKSNFDLAEDAKLQLSKDTYDDDLD NLLAQIGDQYADLFLAAKNLSDAILLSDILRVNTEITKAPLSASMIKRYDEHHQDLTLL KALVRQQLPEKYKEIFFDQSKNGYAGYIDGGASQEEFYKFIKPILEKMDGTEELLVKL NREDLLRKQRTFDNGSIPHQIHLGELHAILRRQEDFYPFLKDNREKIEKILTFRIPYYVG PLARGNSRFAWMTRKSEETITPWNFEEVVDKGASAQSFIERMTNFDKNLPNEKVLPK HSLLYEYFTVYNELTKVKYVTEGMRKPAFLSGEQKKAIVDLLFKTNRKVTVKQLKED YFKKIECFDSVEISGVEDRFNASLGTYHDLLKIIKDKDFLDNEENEDILEDIVLTLTLFE DREMIEERLKTYAHLFDDKVMKQLKRRRYTGWGRLSRKLINGIRDKQSGKTILDFLK SDGFANRNFMQLIHDDSLTFKEDIQKAQVSGQGDSLHEHIANLAGSPAIKKGILQTVK VVDELVKVMGRHKPENIVIEMARENQTTQKGQKNSRERMKRIEEGIKELGSQILKEHP VENTQLQNEKLYLYYLQNGRDMYVDQELDINRLSDYDVDHIVPQSFLKDDSIDNKVL TRSDKNRGKSDNVPSEEVVKKMKNYWRQLLNAKLITQRKFDNLTKAERGGLSELDK AGFIKRQLVETRQITKHVAQILDSRMNTKYDENDKLIREVKVITLKSKLVSDFRKDFQF YKVREINNYHHAHDAYLNAVVGTALIKKYPKLESEFVYGDYKVYDVRKMIAKSEQEI GKATAKYFFYSNIMNFFKTEITLANGEIRKRPLIETNGETGEIVWDKGRDFATVRKVLS MPQVNIVKKTEVQTGGFSKESILPKRNSDKLIARKKDWDPKKYGGFDSPTVAYSVLV VAKVEKGKSKKLKSVKELLGITIMERSSFEKNPIDFLEAKGYKEVKKDLIIKLPKYSLF ELENGRKRMLASAGELQKGNELALPSKYVNFLYLASHYEKLKGSPEDNEQKQLFVEQ HKHYLDEIIEQISEFSKRVILADANLDKVLSAYNKHRDKPIREQAENIIHLFTLTNLGAP AAFKYFDTTIDRKRYTSTKEVLDATLIHQSITGLYETRIDLSQLGGDKRPAATKKAGQ AKKKK |
| 66 | SpCas9-mut5 | MAPKKKRKVGIHGVPAADKKYSIGLDIGTNSVGWAVITDEYKVPSKKFKVLGNTDRH SIKKNLIGALLFDSGETAEATRLKRTARRRYTRRKNRICYLQEIFSNEMAKVDDSFFHR LEESFLVEEDKKHERHPIFGNIVDEVAYHEKYPTIYHLRKKLVDSTDKADLRLIYLALA HMIKFRGHFLIEGDLNPDNSDVDKLFIQLVQTYNQLFEENPINASGVDAKAILSARLSK |
| | | SRRLENLIAQLPGEKKNGLFGNLIALSLGLTPNFKSNFDLAEDAKLQLSKDTYDDDLD NLLAQIGDQYADLFLAAKNLSDAILLSDILRVNTEITKAPLSASMIKRYDEHHQDLTLL KALVRQQLPEKYKEIFFDQSKNGYAGYIDGGASQEEFYKFIKPILEKMDGTEELLVKL NREDLLRKQRTFDNGSIPHQIHLGELHAILRRQEDFYPFLKDNREKIEKILTFRIPYYVG PLARGNSRFAWMTRKSEETITPWNFEEVVDKGASAQSFIERMTNFDKNLPNEKVLPK HSLLYEYFTVYNELTAVKYVTEGMRKPAFLSGEQKKAIVDLLFKTNRKVTVKQLKED YFKKIECFDSVEISGVEDRFNASLGTYHDLLKIIKDKDFLDNEENEDILEDIVLTLTLFE DREMIEERLKTYAHLFDDKVMKQLKRRRYTGWGRLSRKLINGIRDKQSGKTILDFLK SDGFANANFMALIADDSLTFKEDIQKAQVSGQGDSLHEHIANLAGSPAIKKGILQTVK VVDELVKVMGRHKPENIVIEMARENQTTQKGQKNSRERMKRIEEGIKELGSQILKEHP VENTQLQNEKLYLYYLQNGRDMYVDQELDINRLSDYDVDHIVPQSFLKDDSIDNKVL TRSDKNRGKSDNVPSEEVVKKMKNYWRQLLNAKLITQRKFDNLTKAERGGLSELDK AGFIKRQLVETRQITKHVAQILDSRMNTKYDENDKLIREVKVITLKSKLVSDFRKDFQF YKVREINNYHHAHDAYLNAVVGTALIKKYPKLESEFVYGDYKVYDVRKMIAKSEQEI GKATAKYFFYSNIMNFFKTEITLANGEIRKRPLIETNGETGEIVWDKGRDFATVRKVLS MPQVNIVKKTEVQTGGFSKESILPKRNSDKLIARKKDWDPKKYGGFDSPTVAYSVLV VAKVEKGKSKKLKSVKELLGITIMERSSFEKNPIDFLEAKGYKEVKKDLIIKLPKYSLF ELENGRKRMLASAGELQKGNELALPSKYVNFLYLASHYEKLKGSPEDNEQKQLFVEQ HKHYLDEIIEQISEFSKRVILADANLDKVLSAYNKHRDKPIREQAENIIHLFTLTNLGAP AAFKYFDTTIDRKRYTSTKEVLDATLIHQSITGLYETRIDLSQLGGDKRPAATKKAGQ AKKKK |
| 67 | SpCas9-HF1 | MAPKKKRKVGIHGVPAADKKYSIGLDIGTNSVGWAVITDEYKVPSKKFKVLGNTDRH SIKKNLIGALLFDSGETAEATRLKRTARRRYTRRKNRICYLQEIFSNEMAKVDDSFFHR LEESFLVEEDKKHERHPIFGNIVDEVAYHEKYPTIYHLRKKLVDSTDKADLRLIYLALA HMIKFRGHFLIEGDLNPDNSDVDKLFIQLVQTYNQLFEENPINASGVDAKAILSARLSK SRRLENLIAQLPGEKKNGLFGNLIALSLGLTPNFKSNFDLAEDAKLQLSKDTYDDDLD NLLAQIGDQYADLFLAAKNLSDAILLSDILRVNTEITKAPLSASMIKRYDEHHQDLTLL KALVRQQLPEKYKEIFFDQSKNGYAGYIDGGASQEEFYKFIKPILEKMDGTEELLVKL NREDLLRKQRTFDNGSIPHQIHLGELHAILRRQEDFYPFLKDNREKIEKILTFRIPYYVG PLARGNSRFAWMTRKSEETITPWNFEEVVDKGASAQSFIERMTAFDKNLPNEKVLPK HSLLYEYFTVYNELTKVKYVTEGMRKPAFLSGEQKKAIVDLLFKTNRKVTVKQLKED YFKKIECFDSVEISGVEDRFNASLGTYHDLLKIIKDKDFLDNEENEDILEDIVLTLTLFE DREMIEERLKTYAHLFDDKVMKQLKRRRYTGWGALSRKLINGIRDKQSGKTILDFLK SDGFANRNFMALIHDDSLTFKEDIQKAQVSGQGDSLHEHIANLAGSPAIKKGILQTVK VVDELVKVMGRHKPENIVIEMARENQTTQKGQKNSRERMKRIEEGIKELGSQILKEHP VENTQLQNEKLYLYYLQNGRDMYVDQELDINRLSDYDVDHIVPQSFLKDDSIDNKVL TRSDKNRGKSDNVPSEEVVKKMKNYWRQLLNAKLITQRKFDNLTKAERGGLSELDK AGFIKRQLVETRAITKHVAQILDSRMNTKYDENDKLIREVKVITLKSKLVSDFRKDFQF YKVREINNYHHAHDAYLNAVVGTALIKKYPKLESEFVYGDYKVYDVRKMIAKSEQEI GKATAKYFFYSNIMNFFKTEITLANGEIRKRPLIETNGETGEIVWDKGRDFATVRKVLS MPQVNIVKKTEVQTGGFSKESILPKRNSDKLIARKKDWDPKKYGGFDSPTVAYSVLV VAKVEKGKSKKLKSVKELLGITIMERSSFEKNPIDFLEAKGYKEVKKDLIIKLPKYSLF ELENGRKRMLASAGELQKGNELALPSKYVNFLYLASHYEKLKGSPEDNEQKQLFVEQ HKHYLDEIIEQISEFSKRVILADANLDKVLSAYNKHRDKPIREQAENIIHLFTLTNLGAP |
| | | AAFKYFDTTIDRKRYTSTKEVLDATLIHQSITGLYETRIDLSQLGGDKRPAATKKAGQ AKKKK |
| 68 | SpCas9 reference sequence | |
| 69 | SpCas9 double stranded cleavage enzyme-Mut5 | ADKKYSIGLDIGTNSVGWAVITDEYKVPSKKFKVLGNTDRHSIKKNLIGALLFDSGET AEATRLKRTARRRYTRRKNRICYLQEIFSNEMAKVDDSFFHRLEESFLVEEDKKHERH PIFGNIVDEVAYHEKYPTIYHLRKKLVDSTDKADLRLIYLALAHMIKFRGHFLIEGDLN PDNSDVDKLFIQLVQTYNQLFEENPINASGVDAKAILSARLSKSRRLENLIAQLPGEKK NGLFGNLIALSLGLTPNFKSNFDLAEDAKLQLSKDTYDDDLDNLLAQIGDQYADLFLA AKNLSDAILLSDILRVNTEITKAPLSASMIKRYDEHHQDLTLLKALVRQQLPEKYKEIF FDQSKNGYAGYIDGGASQEEFYKFIKPILEKMDGTEELLVKLNREDLLRKQRTFDNGS IPHQIHLGELHAILRRQEDFYPFLKDNREKIEKILTFRIPYYVGPLARGNSRFAWMTRKS EETITPWNFEEVVDKGASAQSFIERMTNFDKNLPNEKVLPKHSLLYEYFTVYNELTAV KYVTEGMRKPAFLSGEQKKAIVDLLFKTNRKVTVKQLKEDYFKKIECFDSVEISGVED RFNASLGTYHDLLKIIKDKDFLDNEENEDILEDIVLTLTLFEDREMIEERLKTYAHLFDD KVMKQLKRRRYTGWGRLSRKLINGIRDKQSGKTILDFLKSDGFANANFMALIADDSL TFKEDIQKAQVSGQGDSLHEHIANLAGSPAIKKGILQTVKVVDELVKVMGRHKPENIV IEMARENQTTQKGQKNSRERMKRIEEGIKELGSQILKEHPVENTQLQNEKLYLYYLQN GRDMYVDQELDINRLSDYDVDHIVPQSFLKDDSIDNKVLTRSDKNRGKSDNVPSEEV VKKMKNYWRQLLNAKLITQRKFDNLTKAERGGLSELDKAGFIKRQLVETRQITKHVA QILDSRMNTKYDENDKLIREVKVITLKSKLVSDFRKDFQFYKVREINNYHHAHDAYL NAVVGTALIKKYPKLESEFVYGDYKVYDVRKMIAKSEQEIGKATAKYFFYSNIMNFF |
| | | KTEITLANGEIRKRPLIETNGETGEIVWDKGRDFATVRKVLSMPQVNIVKKTEVQTGG FSKESILPKRNSDKLIARKKDWDPKKYGGFDSPTVAYSVLVVAKVEKGKSKKLKSVK ELLGITIMERSSFEKNPIDFLEAKGYKEVKKDLIIKLPKYSLFELENGRKRMLASAGELQ KGNELALPSKYVNFLYLASHYEKLKGSPEDNEQKQLFVEQHKHYLDEIIEQISEFSKRV ILADANLDKVLSAYNKHRDKPIREQAENIIHLFTLTNLGAPAAFKYFDTTIDRKRYTST KEVLDATLIHQSITGLYETRIDLSQLGGD |
| 70 | C-termina 1 nuclear localizati on signal peptide of the cleavage enzyme | KRPAATKKAGQAKKKK |
| 71 | N-termin al nuclear localizati on signal peptide of the cleavage enzyme | APKKKRKVGIHGVPAA |
| 72 | ABE8e-WT | MKRTADGSEFESPKKKRKVSEVEFSHEYWMRHALTLAKRARDEREVPVGAVLVLNN RVIGEGWNRAIGLHDPTAHAEIMALRQGGLVMQNYRLIDATLYVTFEPCVMCAGAMI HSRIGRVVFGVRNSKRGAAGSLMNVLNYPGMNHRVEITEGILADECAALLCDFYRMP RQVFNAQKKAQSSINSGGSSGGSSGSETPGTSESATPESSGGSSGGSDKKYSIGLAIGTN SVGWAVITDEYKVPSKKFKVLGNTDRHSIKKNLIGALLFDSGETAEATRLKRTARRRY TRRKNRICYLQEIFSNEMAKVDDSFFHRLEESFLVEEDKKHERHPIFGNIVDEVAYHEK YPTIYHLRKKLVDSTDKADLRLIYLALAHMIKFRGHFLIEGDLNPDNSDVDKLFIQLVQ TYNQLFEENPINASGVDAKAILSARLSKSRRLENLIAQLPGEKKNGLFGNLIALSLGLTP NFKSNFDLAEDAKLQLSKDTYDDDLDNLLAQIGDQYADLFLAAKNLSDAILLSDILRV NTEITKAPLSASMIKRYDEHHQDLTLLKALVRQQLPEKYKEIFFDQSKNGYAGYIDGG ASQEEFYKFIKPILEKMDGTEELLVKLNREDLLRKQRTFDNGSIPHQIHLGELHAILRRQ EDFYPFLKDNREKIEKILTFRIPYYVGPLARGNSRFAWMTRKSEETITPWNFEEVVDKG ASAQSFIERMTNFDKNLPNEKVLPKHSLLYEYFTVYNELTKVKYVTEGMRKPAFLSGE QKKAIVDLLFKTNRKVTVKQLKEDYFKKIECFDSVEISGVEDRFNASLGTYHDLLKIIK DKDFLDNEENEDILEDIVLTLTLFEDREMIEERLKTYAHLFDDKVMKQLKRRRYTGW GRLSRKLINGIRDKQSGKTILDFLKSDGFANRNFMQLIHDDSLTFKEDIQKAQVSGQGD SLHEHIANLAGSPAIKKGILQTVKVVDELVKVMGRHKPENIVIEMARENQTTQKGQK NSRERMKRIEEGIKELGSQILKEHPVENTQLQNEKLYLYYLQNGRDMYVDQELDINRL SDYDVDHIVPQSFLKDDSIDNKVLTRSDKNRGKSDNVPSEEVVKKMKNYWRQLLNA KLITQRKFDNLTKAERGGLSELDKAGFIKRQLVETRQITKHVAQILDSRMNTKYDEND KLIREVKVITLKSKLVSDFRKDFQFYKVREINNYHHAHDAYLNAVVGTALIKKYPKLE SEFVYGDYKVYDVRKMIAKSEQEIGKATAKYFFYSNIMNFFKTEITLANGEIRKRPLIE |
| | | TNGETGEIVWDKGRDFATVRKVLSMPQVNIVKKTEVQTGGFSKESILPKRNSDKLIAR KKDWDPKKYGGFDSPTVAYSVLVVAKVEKGKSKKLKSVKELLGITIMERSSFEKNPID FLEAKGYKEVKKDLIIKLPKYSLFELENGRKRMLASAGELQKGNELALPSKYVNFLYL ASHYEKLKGSPEDNEQKQLFVEQHKHYLDEIIEQISEFSKRVILADANLDKVLSAYNK HRDKPIREQAENIIHLFTLTNLGAPAAFKYFDTTIDRKRYTSTKEVLDATLIHQSITGLY ETRIDLSQLGGDSGGSKRTADGSEFEPKKKRKV |
| 73 | ABE8e-Mut5 | MKRTADGSEFESPKKKRKVSEVEFSHEYWMRHALTLAKRARDEREVPVGAVLVLNN RVIGEGWNRAIGLHDPTAHAEIMALRQGGLVMQNYRLIDATLYVTFEPCVMCAGAMI HSRIGRVVFGVRNSKRGAAGSLMNVLNYPGMNHRVEITEGILADECAALLCDFYRMP RQVFNAQKKAQSSINSGGSSGGSSGSETPGTSESATPESSGGSSGGSDKKYSIGLAIGTN SVGWAVITDEYKVPSKKFKVLGNTDRHSIKKNLIGALLFDSGETAEATRLKRTARRRY TRRKNRICYLQEIFSNEMAKVDDSFFHRLEESFLVEEDKKHERHPIFGNIVDEVAYHEK YPTIYHLRKKLVDSTDKADLRLIYLALAHMIKFRGHFLIEGDLNPDNSDVDKLFIQLVQ TYNQLFEENPINASGVDAKAILSARLSKSRRLENLIAQLPGEKKNGLFGNLIALSLGLTP NFKSNFDLAEDAKLQLSKDTYDDDLDNLLAQIGDQYADLFLAAKNLSDAILLSDILRV NTEITKAPLSASMIKRYDEHHQDLTLLKALVRQQLPEKYKEIFFDQSKNGYAGYIDGG ASQEEFYKFIKPILEKMDGTEELLVKLNREDLLRKQRTFDNGSIPHQIHLGELHAILRRQ EDFYPFLKDNREKIEKILTFRIPYYVGPLARGNSRFAWMTRKSEETITPWNFEEVVDKG ASAQSFIERMTNFDKNLPNEKVLPKHSLLYEYFTVYNELTAVKYVTEGMRKPAFLSGE QKKAIVDLLFKTNRKVTVKQLKEDYFKKIECFDSVEISGVEDRFNASLGTYHDLLKIIK DKDFLDNEENEDILEDIVLTLTLFEDREMIEERLKTYAHLFDDKVMKQLKRRRYTGW GRLSRKLINGIRDKQSGKTILDFLKSDGFANANFMALIADDSLTFKEDIQKAQVSGQG DSLHEHIANLAGSPAIKKGILQTVKVVDELVKVMGRHKPENIVIEMARENQTTQKGQ KNSRERMKRIEEGIKELGSQILKEHPVENTQLQNEKLYLYYLQNGRDMYVDQELDINR LSDYDVDHIVPQSFLKDDSIDNKVLTRSDKNRGKSDNVPSEEVVKKMKNYWRQLLN AKLITQRKFDNLTKAERGGLSELDKAGFIKRQLVETRQITKHVAQILDSRMNTKYDEN DKLIREVKVITLKSKLVSDFRKDFQFYKVREINNYHHAHDAYLNAVVGTALIKKYPKL ESEFVYGDYKVYDVRKMIAKSEQEIGKATAKYFFYSNIMNFFKTEITLANGEIRKRPLI ETNGETGEIVWDKGRDFATVRKVLSMPQVNIVKKTEVQTGGFSKESILPKRNSDKLIA RKKDWDPKKYGGFDSPTVAYSVLVVAKVEKGKSKKLKSVKELLGITIMERSSFEKNPI DFLEAKGYKEVKKDLIIKLPKYSLFELENGRKRMLASAGELQKGNELALPSKYVNFLY LASHYEKLKGSPEDNEQKQLFVEQHKHYLDEIIEQISEFSKRVILADANLDKVLSAYNK HRDKPIREQAENIIHLFTLTNLGAPAAFKYFDTTIDRKRYTSTKEVLDATLIHQSITGLY ETRIDLSQLGGDSGGSKRTADGSEFEPKKKRKV |
| 74 | N-terminal nuclear localization signal peptide of ABE8e | KRTADGSEFESPKKKRKV |
| 75 | C-terminal nuclear localization signal peptide of ABE8e | KRTADGSEFEPKKKRKV |
| 76 | TadA* | SEVEFSHEYWMRHALTLAKRARDEREVPVGAVLVLNNRVIGEGWNRAIGLHDPTAH AEIMALRQGGLVMQNYRLIDATLYVTFEPCVMCAGAMIHSRIGRVVFGVRNSKRGAA GSLMNVLNYPGMNHRVEITEGILADECAALLCDFYRMPRQVFNAQKKAQSSIN |
| 77 | 32-aa Linker | SGGSSGGSSGSETPGTSESATPESSGGSSGGS |
| 78 | nCas9-m ut5 | DKKYSIGLAIGTNSVGWAVITDEYKVPSKKFKVLGNTDRHSIKKNLIGALLFDSGETA EATRLKRTARRRYTRRKNRICYLQEIFSNEMAKVDDSFFHRLEESFLVEEDKKHERHPI FGNIVDEVAYHEKYPTIYHLRKKLVDSTDKADLRLIYLALAHMIKFRGHFLIEGDLNP DNSDVDKLFIQLVQTYNQLFEENPINASGVDAKAILSARLSKSRRLENLIAQLPGEKKN GLFGNLIALSLGLTPNFKSNFDLAEDAKLQLSKDTYDDDLDNLLAQIGDQYADLFLAA KNLSDAILLSDILRVNTEITKAPLSASMIKRYDEHHQDLTLLKALVRQQLPEKYKEIFF DQSKNGYAGYIDGGASQEEFYKFIKPILEKMDGTEELLVKLNREDLLRKQRTFDNGSI PHQIHLGELHAILRRQEDFYPFLKDNREKIEKILTFRIPYYVGPLARGNSRFAWMTRKS EETITPWNFEEVVDKGASAQSFIERMTNFDKNLPNEKVLPKHSLLYEYFTVYNELTAV KYVTEGMRKPAFLSGEQKKAIVDLLFKTNRKVTVKQLKEDYFKKIECFDSVEISGVED RFNASLGTYHDLLKIIKDKDFLDNEENEDILEDIVLTLTLFEDREMIEERLKTYAHLFDD KVMKQLKRRRYTGWGRLSRKLINGIRDKQSGKTILDFLKSDGFANANFMALIADDSL TFKEDIQKAQVSGQGDSLHEHIANLAGSPAIKKGILQTVKVVDELVKVMGRHKPENIV IEMARENQTTQKGQKNSRERMKRIEEGIKELGSQILKEHPVENTQLQNEKLYLYYLQN GRDMYVDQELDINRLSDYDVDHIVPQSFLKDDSIDNKVLTRSDKNRGKSDNVPSEEV VKKMKNYWRQLLNAKLITQRKFDNLTKAERGGLSELDKAGFIKRQLVETRQITKHVA QILDSRMNTKYDENDKLIREVKVITLKSKLVSDFRKDFQFYKVREINNYHHAHDAYL NAVVGTALIKKYPKLESEFVYGDYKVYDVRKMIAKSEQEIGKATAKYFFYSNIMNFF KTEITLANGEIRKRPLIETNGETGEIVWDKGRDFATVRKVLSMPQVNIVKKTEVQTGG FSKESILPKRNSDKLIARKKDWDPKKYGGFDSPTVAYSVLVVAKVEKGKSKKLKSVK ELLGITIMERSSFEKNPIDFLEAKGYKEVKKDLIIKLPKYSLFELENGRKRMLASAGELQ KGNELALPSKYVNFLYLASHYEKLKGSPEDNEQKQLFVEQHKHYLDEIIEQISEFSKRV ILADANLDKVLSAYNKHRDKPIREQAENIIHLFTLTNLGAPAAFKYFDTTIDRKRYTST KEVLDATLIHQSITGLYETRIDLSQLGGD |
| 79 | CRISPRo ff-EE-W T | MNHDQEFDPPKVYPPVPAEKRKPIRVLSLFDGIATGLLVLKDLGIQVDRYIASEVCEDS ITVGMVRHQGKIMYVGDVRSVTQKHIQEWGPFDLVIGGSPCNDLSIVNPARKGLYEG TGRLFFEFYRLLHDARPKEGDDRPFFWLFENVVAMGVSDKRDISRFLESNPVMIDAKE VSAAHRARYFWGNLPGMNRPLASTVNDKLELQECLEHGRIAKFSKVRTITTRSNSIKQ GKDQHFPVFMNEKEDILWCTEMERVFGFPVHYTDVSNMSRLARQRLLGRSWSVPVIR HLFAPLKEYFACVSSGNSNANSRGPSFSSGLVPLSLRGSHMGPMEIYKTVSAWKRQPV RVLSLFRNIDKVLKSLGFLESGSGSGGGTLKYVEDVTNVVRRDVEKWGPFDLVYGST QPLGSSCDRCPGWYMFQFHRILQYALPRQESQRPFFWIFMDNLLLTEDDQETTTRFLQ TEAVTLQDVRGRDYQNAMRVWSNIPGLKSKHAPLTPKEEEYLQAQVRSRSKLDAPK VDLLVKNCLLPLREYFKYFSQNSLPLGGPSSGAPPPSGGSPAGSPTSTEEGTSESATPES GPGTSTEPSEGSAPGSPAGSPTSTEEGTSTEPSEGSAPGTSTEPSEMDKKYSIGLAIGTNS VGWAVITDEYKVPSKKFKVLGNTDRHSIKKNLIGALLFDSGETAEATRLKRTARRRYT RRKNRICYLQEIFSNEMAKVDDSFFHRLEESFLVEEDKKHERHPIFGNIVDEVAYHEKY |
| | | PTIYHLRKKLVDSTDKADLRLIYLALAHMIKFRGHFLIEGDLNPDNSDVDKLFIQLVQT YNQLFEENPINASGVDAKAILSARLSKSRRLENLIAQLPGEKKNGLFGNLIALSLGLTP NFKSNFDLAEDAKLQLSKDTYDDDLDNLLAQIGDQYADLFLAAKNLSDAILLSDILRV NTEITKAPLSASMIKRYDEHHQDLTLLKALVRQQLPEKYKEIFFDQSKNGYAGYIDGG ASQEEFYKFIKPILEKMDGTEELLVKLNREDLLRKQRTFDNGSIPHQIHLGELHAILRRQ EDFYPFLKDNREKIEKILTFRIPYYVGPLARGNSRFAWMTRKSEETITPWNFEEVVDKG ASAQSFIERMTNFDKNLPNEKVLPKHSLLYEYFTVYNELTKVKYVTEGMRKPAFLSGE QKKAIVDLLFKTNRKVTVKQLKEDYFKKIECFDSVEISGVEDRFNASLGTYHDLLKIIK DKDFLDNEENEDILEDIVLTLTLFEDREMIEERLKTYAHLFDDKVMKQLKRRRYTGW GRLSRKLINGIRDKQSGKTILDFLKSDGFANRNFMQLIHDDSLTFKEDIQKAQVSGQGD SLHEHIANLAGSPAIKKGILQTVKVVDELVKVMGRHKPENIVIEMARENQTTQKGQK NSRERMKRIEEGIKELGSQILKEHPVENTQLQNEKLYLYYLQNGRDMYVDQELDINRL SDYDVDAIVPQSFLKDDSIDNKVLTRSDKNRGKSDNVPSEEVVKKMKNYWRQLLNA KLITQRKFDNLTKAERGGLSELDKAGFIKRQLVETRQITKHVAQILDSRMNTKYDEND KLIREVKVITLKSKLVSDFRKDFQFYKVREINNYHHAHDAYLNAVVGTALIKKYPKLE SEFVYGDYKVYDVRKMIAKSEQEIGKATAKYFFYSNIMNFFKTEITLANGEIRKRPLIE TNGETGEIVWDKGRDFATVRKVLSMPQVNIVKKTEVQTGGFSKESILPKRNSDKLIAR KKDWDPKKYGGFDSPTVAYSVLVVAKVEKGKSKKLKSVKELLGITIMERSSFEKNPID FLEAKGYKEVKKDLIIKLPKYSLFELENGRKRMLASAGELQKGNELALPSKYVNFLYL ASHYEKLKGSPEDNEQKQLFVEQHKHYLDEIIEQISEFSKRVILADANLDKVLSAYNK HRDKPIREQAENIIHLFTLTNLGAPAAFKYFDTTIDRKRYTSTKEVLDATLIHQSITGLY ETRIDLSQLGGDPKKKRKVEDPKKKRKVGGGGGMDAKSLTAWSRTLVTFKDVFVDF TREEWKLLDTAQQIVYRNVMLENYKNLVSLGYQLTKPDVILRLEKGEEP |
| 80 | CRISPRo ff-EE-Mu t5 | MNHDQEFDPPKVYPPVPAEKRKPIRVLSLFDGIATGLLVLKDLGIQVDRYIASEVCEDS ITVGMVRHQGKIMYVGDVRSVTQKHIQEWGPFDLVIGGSPCNDLSIVNPARKGLYEG TGRLFFEFYRLLHDARPKEGDDRPFFWLFENVVAMGVSDKRDISRFLESNPVMIDAKE VSAAHRARYFWGNLPGMNRPLASTVNDKLELQECLEHGRIAKFSKVRTITTRSNSIKQ GKDQHFPVFMNEKEDILWCTEMERVFGFPVHYTDVSNMSRLARQRLLGRSWSVPVIR HLFAPLKEYFACVSSGNSNANSRGPSFSSGLVPLSLRGSHMGPMEIYKTVSAWKRQPV RVLSLFRNIDKVLKSLGFLESGSGSGGGTLKYVEDVTNVVRRDVEKWGPFDLVYGST QPLGSSCDRCPGWYMFQFHRILQYALPRQESQRPFFWIFMDNLLLTEDDQETTTRFLQ TEAVTLQDVRGRDYQNAMRVWSNIPGLKSKHAPLTPKEEEYLQAQVRSRSKLDAPK VDLLVKNCLLPLREYFKYFSQNSLPLGGPSSGAPPPSGGSPAGSPTSTEEGTSESATPES GPGTSTEPSEGSAPGSPAGSPTSTEEGTSTEPSEGSAPGTSTEPSEMDKKYSIGLAIGTNS VGWAVITDEYKVPSKKFKVLGNTDRHSIKKNLIGALLFDSGETAEATRLKRTARRRYT RRKNRICYLQEIFSNEMAKVDDSFFHRLEESFLVEEDKKHERHPIFGNIVDEVAYHEKY PTIYHLRKKLVDSTDKADLRLIYLALAHMIKFRGHFLIEGDLNPDNSDVDKLFIQLVQT YNQLFEENPINASGVDAKAILSARLSKSRRLENLIAQLPGEKKNGLFGNLIALSLGLTP NFKSNFDLAEDAKLQLSKDTYDDDLDNLLAQIGDQYADLFLAAKNLSDAILLSDILRV NTEITKAPLSASMIKRYDEHHQDLTLLKALVRQQLPEKYKEIFFDQSKNGYAGYIDGG ASQEEFYKFIKPILEKMDGTEELLVKLNREDLLRKQRTFDNGSIPHQIHLGELHAILRRQ EDFYPFLKDNREKIEKILTFRIPYYVGPLARGNSRFAWMTRKSEETITPWNFEEVVDKG ASAQSFIERMTNFDKNLPNEKVLPKHSLLYEYFTVYNELTAVKYVTEGMRKPAFLSGE QKKAIVDLLFKTNRKVTVKQLKEDYFKKIECFDSVEISGVEDRFNASLGTYHDLLKIIK |
| | | DKDFLDNEENEDILEDIVLTLTLFEDREMIEERLKTYAHLFDDKVMKQLKRRRYTGW GRLSRKLINGIRDKQSGKTILDFLKSDGFANANFMALIADDSLTFKEDIQKAQVSGQG DSLHEHIANLAGSPAIKKGILQTVKVVDELVKVMGRHKPENIVIEMARENQTTQKGQ KNSRERMKRIEEGIKELGSQILKEHPVENTQLQNEKLYLYYLQNGRDMYVDQELDINR LSDYDVDAIVPQSFLKDDSIDNKVLTRSDKNRGKSDNVPSEEVVKKMKNYWRQLLN AKLITQRKFDNLTKAERGGLSELDKAGFIKRQLVETRQITKHVAQILDSRMNTKYDEN DKLIREVKVITLKSKLVSDFRKDFQFYKVREINNYHHAHDAYLNAVVGTALIKKYPKL ESEFVYGDYKVYDVRKMIAKSEQEIGKATAKYFFYSNIMNFFKTEITLANGEIRKRPLI ETNGETGEIVWDKGRDFATVRKVLSMPQVNIVKKTEVQTGGFSKESILPKRNSDKLIA RKKDWDPKKYGGFDSPTVAYSVLVVAKVEKGKSKKLKSVKELLGITIMERSSFEKNPI DFLEAKGYKEVKKDLIIKLPKYSLFELENGRKRMLASAGELQKGNELALPSKYVNFLY LASHYEKLKGSPEDNEQKQLFVEQHKHYLDEIIEQISEFSKRVILADANLDKVLSAYNK HRDKPIREQAENIIHLFTLTNLGAPAAFKYFDTTIDRKRYTSTKEVLDATLIHQSITGLY ETRIDLSQLGGDPKKKRKVEDPKKKRKVGGGGGMDAKSLTAWSRTLVTFKDVFVDF TREEWKLLDTAQQIVYRNVMLENYKNLVSLGYQLTKPDVILRLEKGEEP |
| 81 | dCas-Mut 5 | DKKYSIGLAIGTNSVGWAVITDEYKVPSKKFKVLGNTDRHSIKKNLIGALLFDSGETA EATRLKRTARRRYTRRKNRICYLQEIFSNEMAKVDDSFFHRLEESFLVEEDKKHERHPI FGNIVDEVAYHEKYPTIYHLRKKLVDSTDKADLRLIYLALAHMIKFRGHFLIEGDLNP DNSDVDKLFIQLVQTYNQLFEENPINASGVDAKAILSARLSKSRRLENLIAQLPGEKKN GLFGNLIALSLGLTPNFKSNFDLAEDAKLQLSKDTYDDDLDNLLAQIGDQYADLFLAA KNLSDAILLSDILRVNTEITKAPLSASMIKRYDEHHQDLTLLKALVRQQLPEKYKEIFF DQSKNGYAGYIDGGASQEEFYKFIKPILEKMDGTEELLVKLNREDLLRKQRTFDNGSI PHQIHLGELHAILRRQEDFYPFLKDNREKIEKILTFRIPYYVGPLARGNSRFAWMTRKS EETITPWNFEEVVDKGASAQSFIERMTNFDKNLPNEKVLPKHSLLYEYFTVYNELTAV KYVTEGMRKPAFLSGEQKKAIVDLLFKTNRKVTVKQLKEDYFKKIECFDSVEISGVED RFNASLGTYHDLLKIIKDKDFLDNEENEDILEDIVLTLTLFEDREMIEERLKTYAHLFDD KVMKQLKRRRYTGWGRLSRKLINGIRDKQSGKTILDFLKSDGFANANFMALIADDSL TFKEDIQKAQVSGQGDSLHEHIANLAGSPAIKKGILQTVKVVDELVKVMGRHKPENIV IEMARENQTTQKGQKNSRERMKRIEEGIKELGSQILKEHPVENTQLQNEKLYLYYLQN GRDMYVDQELDINRLSDYDVDAIVPQSFLKDDSIDNKVLTRSDKNRGKSDNVPSEEV VKKMKNYWRQLLNAKLITQRKFDNLTKAERGGLSELDKAGFIKRQLVETRQITKHVA QILDSRMNTKYDENDKLIREVKVITLKSKLVSDFRKDFQFYKVREINNYHHAHDAYL NAVVGTALIKKYPKLESEFVYGDYKVYDVRKMIAKSEQEIGKATAKYFFYSNIMNFF KTEITLANGEIRKRPLIETNGETGEIVWDKGRDFATVRKVLSMPQVNIVKKTEVQTGG FSKESILPKRNSDKLIARKKDWDPKKYGGFDSPTVAYSVLVVAKVEKGKSKKLKSVK ELLGITIMERSSFEKNPIDFLEAKGYKEVKKDLIIKLPKYSLFELENGRKRMLASAGELQ KGNELALPSKYVNFLYLASHYEKLKGSPEDNEQKQLFVEQHKHYLDEIIEQISEFSKRV ILADANLDKVLSAYNKHRDKPIREQAENIIHLFTLTNLGAPAAFKYFDTTIDRKRYTST KEVLDATLIHQSITGLYETRIDLSQLGGD |
| 82 | DNMT3 A+3L | MNHDQEFDPPKVYPPVPAEKRKPIRVLSLFDGIATGLLVLKDLGIQVDRYIASEVCEDS ITVGMVRHQGKIMYVGDVRSVTQKHIQEWGPFDLVIGGSPCNDLSIVNPARKGLYEG TGRLFFEFYRLLHDARPKEGDDRPFFWLFENVVAMGVSDKRDISRFLESNPVMIDAKE VSAAHRARYFWGNLPGMNRPLASTVNDKLELQECLEHGRIAKFSKVRTITTRSNSIKQ GKDQHFPVFMNEKEDILWCTEMERVFGFPVHYTDVSNMSRLARQRLLGRSWSVPVIR |
| | | HLFAPLKEYFACVSSGNSNANSRGPSFSSGLVPLSLRGSHMGPMEIYKTVSAWKRQPV RVLSLFRNIDKVLKSLGFLESGSGSGGGTLKYVEDVTNVVRRDVEKWGPFDLVYGST QPLGSSCDRCPGWYMFQFHRILQYALPRQESQRPFFWIFMDNLLLTEDDQETTTRFLQ TEAVTLQDVRGRDYQNAMRVWSNIPGLKSKHAPLTPKEEEYLQAQVRSRSKLDAPK VDLLVKNCLLPLREYFKYFSQNSLPL |
| 83 | DNMT3 A | MNHDQEFDPPKVYPPVPAEKRKPIRVLSLFDGIATGLLVLKDLGIQVDRYIASEVCEDS ITVGMVRHQGKIMYVGDVRSVTQKHIQEWGPFDLVIGGSPCNDLSIVNPARKGLYEG TGRLFFEFYRLLHDARPKEGDDRPFFWLFENVVAMGVSDKRDISRFLESNPVMIDAKE VSAAHRARYFWGNLPGMNRPLASTVNDKLELQECLEHGRIAKFSKVRTITTRSNSIKQ GKDQHFPVFMNEKEDILWCTEMERVFGFPVHYTDVSNMSRLARQRLLGRSWSVPVIR HLFAPLKEYFACV |
| 84 | DNMT3L | SSGNSNANSRGPSFSSGLVPLSLRGSHMGPMEIYKTVSAWKRQPVRVLSLFRNIDKVL KSLGFLESGSGSGGGTLKYVEDVTNVVRRDVEKWGPFDLVYGSTQPLGSSCDRCPGW YMFQFHRILQYALPRQESQRPFFWIFMDNLLLTEDDQETTTRFLQTEAVTLQDVRGRD YQNAMRVWSNIPGLKSKHAPLTPKEEEYLQAQVRSRSKLDAPKVDLLVKNCLLPLRE YFKYFSQNSLPL |
| 85 | Xten80 linker | GGPSSGAPPPSGGSPAGSPTSTEEGTSESATPESGPGTSTEPSEGSAPGSPAGSPTSTEEG TSTEPSEGSAPGTSTEPSE |
| 86 | SV40 NLS | PKKKRKVEDPKKKRKVGGGGGMDAKSLTAWS |
| 87 | KRAB | RTLVTFKDVFVDFTREEWKLLDTAQQIVYRNVMLENYKNLVSLGYQLTKPDVILRLE KGEEP |
| 88 | Exemplary linker 1 | GGGSGGS |
| 89 | Exemplary linker 2 | SGGGS |
| 90 | Exemplary linker 3 | SGSETPGTSESATPES |
| 91 | Exemplary linker 4 | GGGS |

| | | |
|---|---|---|
| Note: when SEQ ID NOs.1-SEQ ID NO.13 serve as mRNA sequences, all thymidine (T) residues thereof represent uridine (U). When SEQ ID NO.1-SEQ ID NO.13 are used in the examples, all U residues thereof are 1-methylpseudouridine. | | |

When SEQ ID NOs.14-46 serve as gRNA sequences, all thymidine (T) thereof represent uridine (U). When the gRNAs shown in SEQ ID NO.14-23 are used in the example, the sequence of SEQ ID NO.14-23 possess the following modifications: the first to third nucleotides from the 5' end and the last one to three nucleotides are 2'-O-methylated, and the internucleotide linkages between the first to third nucleotides from the 5 'end and between the last one to three nucleotides are phosphorothioate linkages.

The above describes the embodiments of the present application. However, the present application is not limited to the above embodiments. Any modifications, equivalent substitutions, improvements, etc. made within the spirit and principles of the present application shall be included within the protection scope of the present application.

## Claims

1. An isolated modified Cas9 nuclease or a DNA-binding fragment thereof, comprising one or more mutations at amino acid residue positions selected from the group consisting of: K526, N692, Q695, H698, N497, Y450, Q926, K377, E387, D397, R400, D406, A421, L423, R424, Q426, Y430, K442, P449, V452, A456, R457, W464, M465, K468, E470, T474, P475, W476, F478, K484, S487, A488, T496, F498, L502, N504, K506, P509, F518, N522, E523, L540, S541, I548, D550, F553, V561, K562, E573, A589, L598, D605, L607, N609, N612, E617, D618, D628, R629, R635, K637, L651, K652, R654, T657, G658, L666, K673, S675, I679, L680, L683, N690, R691, F693, S701, F704, Q712, G715, Q716, H723, I724, L727, I733, L738, Q739, N803, Q805, Q807, K810, Y812, D829, N831, R832, S834, D835, Q844, S845, K848, R859, K862, R864, K866, K890, T893, Q894, D898, N899, K902, K913, K918, Q920, T924, R925, T928, K929, H930, S960, K961, S964, K968, R976, H982, H983, Y1013, K1031, T1033, SI106, K1107, S1109, Y1237, Y1242, K1244 and K1246, wherein the amino acid residue positions correspond to, or are defined by reference to, the amino acid numbering in the amino acid sequence of Streptococcus pyogenes Cas9 (SpCas9) protein set forth in SEQ ID NO.68.

2. The modified Cas9 nuclease or DNA-binding fragment thereof according to claim 1, comprising one or more mutations selected from the group consisting of: K526X, Q695X, H698X and R691X, wherein X is glycine (G), alanine (A), valine (V), isoleucine (I), leucine (L), aspartic acid (D), glutamic acid (E), asparagine (N), glutamine (Q), serine (S), threonine (T), lysine (K), arginine (R), phenylalanine (F) or tyrosine (Y).

3. The modified Cas9 nuclease or DNA binding fragment thereof according to claim 1 or 2, wherein each X is independently any one selected from the group consisting of: glycine (G), alanine (A), aspartic acid (D) and glutamic acid (E).

4. The modified Cas9 nuclease or DNA binding fragment thereof according to claim 3, comprising any mutation combination selected from the group consisting of K526A+R691A+Q695A+H698A, K526A+R691A+N692A+Q695A+H698A, K526G+R691G+Q695G+H698G, K526D+R691A+Q695A+H698A, K526A+R691D+Q695A+H698A, K526A+R691A+Q695A+H698D, K526E+R691A+ Q695A+H698A, K526 A+R691E+Q695A+HO698A, and K526A+R691A+Q695A+H698E.

5. The modified Cas9 nuclease or DNA binding fragment thereof according to any one of claims 1 to 4, which is a Cas9 double stranded nucleic acid cleavage enzyme, nCas9 (Cas9 nickase), or dCas9 (catalytically dead Cas9).

6. The modified Cas9 nuclease or DNA binding fragment thereof according to any one of claims 1 to 5, further comprising one or more mutations in the RuvC domain and/or the HNH domain, optionally comprising a mutation of aspartic acid to alanine (D10A) at position 10 in the RuvC domain and/or a mutation of histidine to alanine (H840A) at position 840 in the HNH domain.

7. The modified Cas9 nuclease or DNA binding fragment thereof according to any one of claims 1 to 6, wherein the amino acid sequence of the Cas9 nuclease is shown in SEQ ID NO.69, SEQ ID NO.78 or SEQ ID NO.81, or comprises an amino acid sequence having at least 80%, 85%, 90%, 95%, 96%, 97%, 98%, 99% or 100% sequence identity to the amino acid sequence shown in SEQ ID NO.69, SEQ ID NO.78 or SEQ ID NO.81.

8. The Cas9 nuclease or DNA binding fragment thereof according to any one of claims 1 to 7, wherein the DNA binding fragment does not comprise one or more amino acid segments selected from the group consisting of:
amino acid segments at positions 494-501, 179-296, 503-708, 792-897 and 1010-1081, wherein the amino acid positions correspond to the amino acid numbering in the SpCas9 protein amino acid sequence of SEQ ID NO. 68,
optionally, the DNA binding fragment is a DNA binding fragment comprised in SEQ ID NO.69, SEQ ID NO.78 or SEQ ID NO.81, wherein the DNA binding fragment does not comprise one or more amino acid segments selected from the following group in these sequences:
amino acid segments at positions 494-501, 179-296, 503-708, 792-897 and 1010-1081.

9. A fusion protein comprising the Cas9 nuclease or DNA binding fragment thereof according to any one of claims 1 to 8.

10. The fusion protein according to claim 9, further comprising a nuclear localization signal peptide on the N-terminus and/or the C-terminus of the Cas9 nuclease or DNA binding fragment thereof.

11. The fusion protein according to claim 9 or 10, further comprising a cytidine deaminase, an adenine deaminase, an oxidase, a glycosylase, an alkyltransferase, a DNA synthase, an RNA synthase, a uracil glycosylase inhibitor (UGI), a transcription activator, a transcription repressor, a methylase, a demethylase, a Gam protein from bacteriophage Mu, and/or a fluorescent protein, fused to the Cas9 nuclease or DNA binding fragment thereof; optionally, the transcription activator comprises VP64, and optionally, the transcription repressor comprises a KRAB protein.

12. The fusion protein according to claim 11, wherein the fusion protein comprises or consists of, from the N-terminus to the C-terminus:
1) a nuclear localization signal peptide-Cas9 double stranded nucleic acid cleavage enzyme, nCas9 or dCas9 or a DNA binding fragment thereof-a nuclear localization signal peptide;
2) a nuclear localization signal peptide-TadA* enzyme-nCas9-a nuclear localization signal peptide;
3) TadA* enzyme-nCas9;
4)Dnmt3A-Dnmt3L-dCas9-KRAB; or
5) Dnmt3A-Dnmt3L-dCas9-a nuclear localization signal peptide-KRAB;
wherein "-" indicates a linkage via a peptide bond or a linker.

13. The fusion protein according to claim 12, wherein:
each linker independently comprises or is any one or more amino acid sequences selected from the group consisting of: SEQ ID NO.77, SEQ ID NO.85, SEQ ID NO.88, SEQ ID NO.89, SEQ ID NO.90 and SEQ ID NO.91, or an amino acid sequence having at least 80%, 85%, 90%, 95%, 96%, 97%, 98%, or 99% sequence identity thereto;
the amino acid sequence of each nuclear localization signal peptide independently comprises or is any one selected from the group consisting of: SEQ ID NO.70, SEQ ID NO.71, SEQ ID NO.74, and SEQ ID NO.75, optionally, the amino acid sequence shown in SEQ ID NO.71 or 74 is located on the N-terminal side, and/or the amino acid sequence shown in SEQ ID NO.70, 75 or 85 is located on the C-terminal side;
the amino acid sequence of the TadA*enzyme comprises or is the amino acid sequence shown in SEQ ID NO.76;
the amino acid sequence of KRAB comprises or is the amino acid sequence shown in SEQ ID NO.87;
the amino acid sequence of DNMT3A comprises or is the amino acid sequence shown in SEQ ID NO.87; and/or
the amino acid sequence of DNMT3L comprises or is the amino acid sequence shown in SEQ ID NO.87.

14. The fusion protein according to any one of claims 11 to 13, wherein the amino acid sequence of the fusion protein is shown in SEQ ID NO.66, SEQ ID NO.73 or SEQ ID NO.80, or comprises an amino acid sequence having at least 80%, 85%, 90%, 95%, 96%, 97%, 98%, 99% or 100% sequence identity to the amino acid sequence shown in SEQ ID NO.66, SEQ ID NO.73 or SEQ ID NO.80.

15. An engineered nucleic acid molecule, comprising a nucleotide sequence encoding the modified Cas9 nuclease or DNA binding fragment thereof according to any one of claims 1 to 8, or the fusion protein according to any one of claims 9 to 14.

16. The nucleic acid molecule according to claim 15, comprising a 5' untranslated region sequence (5'UTR) and a 3' untranslated region (3'UTR) sequence; wherein the 5'UTR comprises or is a 5'UTR of tobacco etch virus gene, and/or the 3'UTR comprises or is a 3'UTR of human hemoglobin alpha 1 (hHBA1) gene.

17. The nucleic acid molecule according to claim 15 or 16, wherein the nucleotide sequence of the 5'UTR is shown in SEQ ID NO.10 or 11, or comprises a nucleotide sequence having at least 80%, 85%, 90%, 95%, 96%, 97%, 98%, 99% or 100% nucleotide sequence identity to the nucleotide sequence shown in SEQ ID NO.10 or 11, and/or the nucleotide sequence of the 3'UTR is shown in SEQ ID NO.13, or comprises a nucleotide sequence having at least 80%, 85%, 90%, 95%, 96%, 97%, 98%, 99% or 100% sequence identity to the nucleotide sequence shown in SEQ ID NO.13.

18. The nucleic acid molecule according to any one of claims 15 to 17, further comprising a poly (A) tail sequence or a polyadenylation signal sequence, preferably the poly (A) tail sequence comprises a nucleotide sequence shown in SEQ ID NO. 12 or comprises a nucleotide sequence having at least 80%, 85%, 90%, 95%, 96%, 97%, 98%, 99% or 100% sequence identity to SEQ ID NO.12.

19. The nucleic acid molecule according to any one of claims 15 to 18, which is an mRNA and comprises a 5' cap structure, optionally, the 5' cap structure is m7G(5')ppp(5')(2'OMeA)pG.

20. The nucleic acid molecule according to any one of claims 15 to 19, wherein the nucleotide sequence of the nucleic acid molecule is shown in SEQ ID NO. 2, SEQ ID NO.6 or SEQ ID NO.8, or comprises a nucleotide sequence having at least 80%, 85%, 90%, 95%, 96%, 97%, 98%, 99% or 100% sequence identity to the nucleotide sequence shown in SEQ ID NO.2, SEQ ID NO.6 or SEQ ID NO.8.

21. The nucleic acid molecule according to any one of claims 15 to 20, which is an mRNA molecule comprising one or more uridine (U) residues with modified bases, optionally, the U residues with modified bases are 1-methylpseudouridines; optionally, each U in the nucleic acid molecule encoding the Cas9 nuclease or an enzymatically active fragment thereof is 1-methylpseudouridine.

22. A composition, a ribonucleoprotein complex or a protein-lipid complex comprising the modified Cas9 nuclease or DNA binding fragment thereof according to any one of claims 1 to 8, or the fusion protein according to any one of claims 9 to 14, or the nucleic acid molecule according to any one of claims 15 to 21.

23. The composition, ribonucleoprotein complex or protein-lipid complex according to claim 22, further comprises a gRNA targeting a gene of interest, a nucleic acid molecule encoding the gRNA, or a construct comprising the gRNA.

24. The composition, ribonucleoprotein complex or protein-lipid complex according to claim 23, wherein the target gene is any one or more selected from the group consisting of: hepatitis B virus (HBV) gene, PCSK9, EMX1 and VEGFA3.

25. A gene editing method, comprising introducing into a host cell the modified Cas9 nuclease or DNA binding fragment thereof according to any one of claims 1 to 8, or the fusion protein according to any one of claims 9 to 14, or the nucleic acid molecule according to any one of claims 15 to 21, or the composition, ribonucleoprotein complex or protein-lipid complex according to any one of claims 22 to 24.

26. Use of the modified Cas9 nuclease or DNA binding fragment thereof according to any one of claims 1 to 8, or the fusion protein according to any one of claims 9 to 14, or the nucleic acid molecule according to any one of claims 15 to 21, or the composition, ribonucleoprotein complex or protein-lipid complex according to any one of claims 22 to 24 in the preparation of a medicament for treating a disease or disorder in a subject in need thereof.
